# EUROPEAN PATENT SPECIFICATION

(11) **EP 1 222 262 B1**
(45) Date of publication and mention of the grant of the patent: **05.09.2007**
(21) Application number: 00974397.2
(22) Date of filing: 16.10.2000
(51) Int. Cl.: C12N 15/10, A01K 67/027, C12N 15/90

(54) **CONDITIONAL GENE TRAPPING CONSTRUCT FOR THE DISRUPTION OF GENES**
BEDINGTES 'GENE TRAPPING'-KONSTRUKT ZUR UNTERBRECHUNG VON GENEN
CONSTRUCTION DE PIEGEAGE DE GENES CONDITIONNEL POUR LA DISRUPTION GENETIQUE

(30) Priority: 16.10.1999 EP 99120592; 27.10.1999 US 162016 P
(43) Date of publication of application: 17.07.2002
(73) Proprietor: ARTEMIS Pharmaceuticals GmbH, 51063 Köln (DE); FrankGen Biotechnologie AG, 40593 Düsseldorf (DE)
(72) Inventor: KÜHN, Ralf, 50937 Köln (DE); VON MELCHENER, Harald, 61476 Kronberg (DE); ALTSCHMIED, Joachim, 63128 Dietzenbach (DE)
(74) Representative: Helbing, Jörg
(86) International application number: PCT/EP2000/010162
(87) International publication number: WO 2001/029208

(56) References cited:
- WO-A-94/17176
- WO-A-99/50426
- ARAKI K. ET AL.: "Exchangeable gene trap using the Cre/mutated lox system." CELL MOL BIOL (NOISY-LE-GRAND) 1999 JUL;45(5):737-50, XP000877476
- B P ZAMBROWICZ AND G A FRIEDRICH: "Comprehensive mammalian genetics: history and fututre prospects of gene trapping in the mouse" INTERNATIONAL JOURNAL OF DEVELOPMENTAL BIOLOGY, vol. 42, 1998, pages 1025-1036, XP002111634 ISSN: 0214-6282
- ARAKI K. ET AL.: "Targeted integration of DNA using mutant lox sites in embryonic stem cells." NUCLEIC ACIDS RES 1997 FEB 15;25(4):868-72, XP002097340
- THORPE ET AL: "In vitro site-specific integration of bacteriophage DNA catalyzed by a recombinase of the resolvase/invertase family" PROCEEDINGS OF THE NATIONAL ACADEMY OF SCIENCES OF USA, vol. 95, 1998, pages 5505-5510, XP002157128 ISSN: 0027-8424 cited in the application
- KÜHN, R. & SCHWENK, F.: "Advances in gene targeting methods" CURRENT OPINION IN IMMUNOLOGY., vol. 9, 1997, pages 183-188, XP002133099 ISSN: 0952-7915
- NUNES-DUBY SIMONE E ET AL: "Similarities and differences among 105 members of the Int family of site-specific recombinases." NUCLEIC ACIDS RESEARCH, vol. 26, no. 2, 15 January 1998 (1998-01-15), pages 391-406, XP002163305 ISSN: 0305-1048 cited in the application
- LORBACH E ET AL: "SITE-SPECIFIC RECOMBINATION IN HUMAN CELLS CATALYZED BY PHAGE LAMBDA INTEGRASE MUTANTS" JOURNAL OF MOLECULAR BIOLOGY,GB,LONDON, vol. 296, 10 March 2000 (2000-03-10), pages 1175-1181, XP000924818 ISSN: 0022-2836 cited in the application

### SEQUENCE LISTING

<110> Artemis Pharmaceuticals GmbH
   FrankGen Biotechnologie AG
<120> Conditional Gene Trapping Construct for the Disruption of Genes
<130> 002256wo/JH/ml
<140>
   <141>
<160> 33
<170> PatentIn Ver. 2.1
<210> 1
   <211> 120
   <212> DNA
   <213> Adenovirus
<400> 1
   gtgacctgca cgtctagggc gcagtagtcc agggtttcct tgatgatgtc atacttatcc 60
   tgtccctttt ttttccacag ctcgcggttg aggacaaact cttcgcggtc tttccagtac 120
<210> 2
   <211> 34
   <212> DNA
   <213> Artificial Sequence
<220>
   <223> Description of Artificial Sequence: mutant loxP site - lox66
<400> 2
   ataacttcgt atagcataca ttatacgaac ggta 34
<210> 3
   <211> 34
   <212> DNA
   <213> Artificial Sequence
<220>
   <223> Description of Artificial Sequence:mutant loxP site - lox71
<400> 3
   taccgttcgt atagcataca ttatacgaag ttat 34
<210> 4
   <211> 34
   <212> DNA
   <213> Artificial Sequence
<220>
   <223> Description of Artificial Sequence:double mutant loxP site - lox66/71
<400> 4
   taccgttcgt,atagcataca ttatacgaac ggta 34
<210> 5
   <211> 34
   <212> DNA
   <213> Bacteriophage P1
<400> 5
   ataacttcgt atagcataca ttatacgaag ttat 34
<210> 6
   <211> 277
   <212> DNA
   <213> Artificial Sequence
<220>
   <223> Description of Artificial Sequence:poly.A signal sequence
<400> 6
   tctagagctc gctgatcagc ctcgactgtg ccttctagtt gccagccatc tgttgtttgc 60
   ccctcccccg tgccttcctt gaccctggaa ggtgccactc ccactgtcct ttcctaataa 120
   aatgaggaaa ttgcatcgca ttgtctgagt aggtgtcatt ctattctggg gggtggggtg 180
   gggcaggaca gcaaggggga ggattgggaa gacaatagca ggcatgctgg ggatgcggtg 240
   ggctctatgg cttctgaggc ggaaagaacc agctggg 277
<210> 7
   <211> 48
   <212> DNA
   <213> Artificial Sequence
<220>
   <223> Description of Artificial Sequence:FRT site
<400> 7
   gaagttccta tacttctaga agaataggaa cttccgaata ggaacttc 48
<210> 8
   <211> 585
   <212> DNA
   <213> ECM virus
<400> 8
   ctctccctcc ccccccccta acgttactgg ccgaagccgc ttggaataag gccggtgtgc 60
   gtttgtctat atgtgatttt ccaccatatt gccgtctttt ggcaatgtga gggcccggaa 120
   acctggccct gtcttcttga cgagcattcc taggggtctt tcccctctcg ccaaaggaat 180
   gcaaggtctg ttgaatgtcg tgaaggaagc agttcctctg gaagcttctt gaagacaaac 240
   aacgtctgta gcgacccttt gcaggcagcg gaacccccca cctggcgaca ggtgcctctg 300
   cggccaaaag ccacgtgtat aagatacacc tgcaaaggcg gcacaacccc agtgccacgt 360
   tgtgagttgg atagttgtgg aaagagtcaa atggctctcc tcaagcgtat tcaacaaggg 420
   gctgaaggat gcccagaagg taccccattg tatgggatct gatctggggc ctcggtgcac 480
   atgctttaca tgtgtttagt cgaggttaaa aaaacgtcta ggccccccga accacgggga 540
   cgtggttttc ctttgaaaaa cacgatgata agcttgccac aaccc 585
<210> 9
   <211> 600
   <212> DNA
   <213> Artificial Sequence
<220>
   <223> Description of Artificial Sequence:Puromycin resistance gene
<400> 9
   atgaccgagt acaagcccac ggtgcgcctc gccacccgcg acgacgtccc ccgggccgta 60
   cgcaccctcg ccgccgcgtt cgccgactac cccgccacgc gccacaccgt cgacccggac 120
   cgccacatcg agcgggtcac cgagctgcaa gaactcttcc tcacgcgcgt cgggctcgac 180
   atcggcaagg tgtgggtcgc ggacgacggc gccgcggtgg cggtctggac cacgccggag 240
   agcgtcgaag cgggggcggt gttcgccgag atcggcccgc gcatggccga gttgagcggt 300
   tcccggctgg ccgcgcagca acagatggaa ggcctcctgg cgccgcaccg gcccaaggag 360
   cccgcgtggt tcctggccac cgtcggcgtc tcgcccgacc accagggcaa gggtctgggc 420
   agcgccgtcg tgctccccgg agtggaggcg gccgagcgcg ccggggtgcc cgccttcctg 480
   gagacctccg cgccccgcaa cctccccttc tacgagcggc tcggcttcac cgtcaccgcc 540
   gacgtcgagt gcccgaagga ccgcgcgacc tggtgcatga cccgcaagcc cggtgcctga 600
<210> 10
   <211> 3652
   <212> DNA
   <213> Escherichia coli
<400> 10
   atgagcgaaa aatacatcgt cacctgggac atgttgcaga tccatgcacg taaactcgca 60
   agccgactga tgccttctga acaatggaaa ggcattattg ccgtaagccg tggcggtctg 120
   gtaccggtgg gtgaagacca gaaacagcac ctcgaactga gccgcgatat tgcccagcgt 180
   ttcaacgcgc tgtatggcga gatcgatccc gtcgttttac aacgtcgtga ctgggaaaac 240
   cctggcgtta cccaacttaa tcgccttgca gcacatcccc ctttcgccag ctggcgtaat 300
   agcgaagagg cccgcaccga tcgcccttcc caacagttgc gcagcctgaa tggcgaatgg 360
   cgctttgcct ggtttccggc accagaagcg gtgccggaaa gctggctgga gtgcgatctt 420
   cctgaggccg atactgtcgt cgtcccctca aactggcaga tgcacggtta cgatgcgccc 480
   atctacacca acgtaaccta tcccattacg gtcaatccgc cgtttgttcc cacggagaat 540
   ccgacgggtt gttactcgct cacatttaat gttgatgaaa gctggctaca ggaaggccag 600
   acgcgaatta tttttgatgg cgttaactcg gcgtttcatc tgtggtgcaa cgggcgctgg 660
   gtcggttacg gccaggacag tcgtttgccg tctgaatttg acctgagcgc atttttacgc 720
   gccggagaaa accgcctcgc ggtgatggtg ctgcgttgga gtgacggcag ttatctggaa 780
   gatcaggata tgtggcggat gagcggcatt ttccgtgacg tctcgttgct gcataaaccg 840
   actacacaaa tcagcgattt ccatgttgcc actcgcttta atgatgattt cagccgcgct 900
   gtactggagg ctgaagttca gatgtgcggc gagttgcgtg actacctacg ggtaacagtt 960
   tctttatggc agggtgaaac gcaggtcgcc agcggcaccg cgcctttcgg cggtgaaatt 1020
   atcgatgagc gtggtggtta tgccgatcgc gtcacactac gtctgaacgt cgaaaacccg 1080
   aaactgtgga gcgccgaaat cccgaatctc tatcgtgcgg tggttgaact gcacaccgcc 1140
   gacggcacgc tgattgaagc agaagcctgc gatgtcggtt tccgcgaggt gcggattgaa 1200
   aatggtctgc tgctgctgaa cggcaagccg ttgctgattc gaggcgttaa ccgtcacgag 1260
   catcatcctc tgcatggtca ggtcatggat gagcagacga tggtgcagga tatcctgctg 1320
   atgaagcaga acaactttaa cgccgtgcgc tgttcgcatt atccgaacca tccgctgtgg 1380
   tacacgctgt gcgaccgcta cggcctgtat gtggtggatg aagccaatat tgaaacccac 1440
   ggcatggtgc caatgaatcg tctgaccgat gatccgcgct ggctaccggc gatgagcgaa 1500
   cgcgtaacgc gaatggtgca gcgcgatcgt aatcacccga gtgtgatcat ctggtcgctg 1560
   gggaatgaat caggccacgg cgctaatcac gacgcgctgt atcgctggat caaatctgtc 1620
   gatccttccc gcccggtgca gtatgaaggc ggcggagccg acaccacggc caccgatatt 1680
   atttgcccga tgtacgcgcg cgtggatgaa gaccagccct tcccggctgt gccgaaatgg 1740
   tccatcaaaa aatggctttc gctacctgga gagacgcgcc cgctgatcct ttgcgaatac 1800
   gcccacgcga tgggtaacag tcttggcggt ttcgctaaat actggcaggc gtttcgtcag 1860
   tatccccgtt tacagggcgg cttcgtctgg gactgggtgg atcagtcgct gattaaatat 1920
   gatgaaaacg gcaacccgtg gtcggcttac ggcggtgatt ttggcgatac gccgaacgat 1980
   cgccagttct gtatgaacgg tctggtcttt gccgaccgca cgccgcatcc agcgctgacg 2040
   gaagcaaaac accagcagca gtttttccag ttccgtttat ccgggcaaac catcgaagtg 2100
   accagcgaat acctgttccg tcatagcgat aacgagctcc tgcactggat ggtggcgctg 2160
   gatggtaagc cgctggcaag cggtgaagtg cctctggatg tcgctccaca aggtaaacag 2220
   ttgattgaac tgcctgaact accgcagccg gagagcgccg ggcaactctg gctcacagta 2280
   cgcgtagtgc aaccgaacgc gaccgcatgg tcagaagccg ggcacatcag cgcctggcag 2340
   cagtggcgtc tggcggaaaa cctcagtgtg acgctccccg ccgcgtccca cgccatcccg 2400
   catctgacca ccagcgaaat ggatttttgc atcgagctgg gtaataagcg ttggcaattt 2460
   aaccgccagt caggctttct ttcacagatg tggattggcg ataaaaaaca actgctgacg 2520
   ccgctgcgcg atcagttcac ccgtgcaccg ctggataacg acattggcgt aagtgaagcg 2580
   acccgcattg accctaacgc ctgggtcgaa cgctggaagg cggcgggcca ttaccaggcc 2640
   gaagcagcgt tgttgcagtg cacggcagat acacttgctg atgcggtgct gattacgacc 2700
   gctcacgcgt ggcagcatca ggggaaaacc ttatttatca gccggaaaac ctaccggatt 2760
   gatggtagtg gtcaaatggc gattaccgtt gatgttgaag tggcgagcga tacaccgcat 2820
   ccggcgcgga ttggcctgaa ctgccagctg gcgcaggtag cagagcgggt aaactggctc 2880
   ggattagggc cgcaagaaaa ctatcccgac cgccttactg ccgcctgttt tgaccgctgg 2940
   gatctgccat tgtcagacat gtataccccg tacgtcttcc cgagcgaaaa cggtctgcgc 3000
   tgcgggacgc gcgaattgaa ttatggccca caccagtggc gcggcgactt ccagttcaac 3060
   atcagccgct acagtcaaca gcaactgatg gaaaccagcc atcgccatct gctgcacgcg 3120
   gaagaaggca catggctgaa tatcgacggt ttccatatgg ggattggtgg cgacgactcc 3180
   tggagcccgt cagtatcggc ggaattccag ctgagcgccg gtcgctacca ttaccagttg 3240
   gtctggtgtc aaaaataata ataaccgggc aggccatgtc tgcccgtatt tcgcgtaagg 3300
   aaatccatta tgtactattt aaaaaacaca aacttttgga tgttcggttt attctttttc 3360
   ttttactttt ttatcatggg agcctacttc ccgtttttcc cgatttggct acatgacatc 3420
   aaccatatca gcaaaagtga tacgggtatt atttttgccg ctatttctct gttctcgcta 3480
   ttattccaac cgctgtttgg tctgctttct gacaaactcg gaacttgttt attgcagctt 3540
   ataatggtta caaataaagc aatagcatca caaatttcac aaataaagca tttttttcac 3600
   tgcattctag ttgtggtttg tccaaactca tcaatgtatc ttatcatgtc tg 3652
<210> 11
   <211> 78
   <212> DNA
   <213> Artificial Sequence
<220>
   <223> Description of Artificial Sequence: primer lox3
<400> 11
   ctagataact tcgtatagca tacattatac gaacggtact cgagataact tcgtataatg 60
   tatgctatac gaacggta 78
<210> 12
   <211> 79
   <212> DNA
   <213> Artificial Sequence
<220>
   <223> Description of Artificial Sequence: primer lox4
<400> 12
   ctagataccg ttcgtatagc atacattata cgaagttatc tcgagtaccg ttcgtataat 60
   gtatgctata cgaagttat                                               79
<210> 13
   <211> 7175
   <212> DNA
   <213> Artificial Sequence
<220>
   <223> Description of Artificial Sequence: vector pRK57SA-beta
<400> 13
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt acccgggggc 420
   gcgccggatc cttaattaag tctagataac ttcgtatagc atacattata cgaacggtac 480
   tcgatcgagg ctagaactag tggatccccc gggctgcaga tctgtagggc gcagtagtcc 540
   agggtttcct tgatgatgtc atacttatcc tgtccctttt ttttccacag ctcgcggttg 600
   aggacaaact cttcgcggtc tttccagtgg ggatcgacgg tatcgataag cttgatgatc 660
   tgtgacatgg cggatcccgt cgttttacaa cgtcgtgact gggaaaaccc tggcgttacc 720
   caacttaatc gccttgcagc acatccccct ttcgccagct ggcgtaatag cgaagaggcc 780
   cgcaccgatc gcccttccca acagttgcgc agcctgaatg gcgaatggcg ctttgcctgg 840
   tttccggcac cagaagcggt gccggaaagc tggctggagt gcgatcttcc tgaggccgat 900
   actgtcgtcg tcccctcaaa ctggcagatg cacggttacg atgcgcccat ctacaccaac 960
   gtaacctatc ccattacggt caatccgccg tttgttccca cggagaatcc gacgggttgt 1020
   tactcgctca catttaatgt tgatgaaagc tggctacagg aaggccagac gcgaattatt 1080
   tttgatggcg ttaactcggc gtttcatctg tggtgcaacg ggcgctgggt cggttacggc 1140
   caggacagtc gtttgccgtc tgaatttgac ctgagcgcat ttttacgcgc cggagaaaac 1200
   cgcctcgcgg tgatggtgct gcgttggagt gacggcagtt atctggaaga tcaggatatg 1260
   tggcggatga gcggcatttt ccgtgacgtc tcgttgctgc ataaaccgac tacacaaatc 1320
   agcgatttcc atgttgccac tcgctttaat gatgatttca gccgcgctgt actggaggct 1380
   gaagttcaga tgtgcggcga gttgcgtgac tacctacggg taacagtttc tttatggcag 1440
   ggtgaaacgc aggtcgccag cggcaccgcg cctttcggcg gtgaaattat cgatgagcgt 1500
   ggtggttatg ccgatcgcgt cacactacgt ctgaacgtcg aaaacccgaa actgtggagc 1560
   gccgaaatcc cgaatctcta tcgtgcggtg gttgaactgc acaccgccga cggcacgctg 1620
   attgaagcag aagcctgcga tgtcggtttc cgcgaggtgc ggattgaaaa tggtctgctg 1680
   ctgctgaacg gcaagccgtt gctgattcga ggcgttaacc gtcacgagca tcatcctctg 1740
   catggtcagg tcatggatga gcagacgatg gtgcaggata tcctgctgat gaagcagaac 1800
   aactttaacg ccgtgcgctg ttcgcattat ccgaaccatc cgctgtggta cacgctgtgc 1860
   gaccgctacg gcctgtatgt ggtggatgaa gccaatattg aaacccacgg catggtgcca 1920
   atgaatcgtc tgaccgatga tccgcgctgg ctaccggcga tgagcgaacg cgtaacgcga 1980
   atggtgcagc gcgatcgtaa tcacccgagt gtgatcatct ggtcgctggg gaatgaatca 2040
   ggccacggcg ctaatcacga cgcgctgtat cgctggatca aatctgtcga tccttcccgc 2100
   ccggtgcagt atgaaggcgg cggagccgac accacggcca ccgatattat ttgcccgatg 2160
   tacgcgcgcg tggatgaaga ccagcccttc ccggctgtgc cgaaatggtc catcaaaaaa 2220
   tggctttcgc tacctggaga gacgcgcccg ctgatccttt gcgaatacgc ccacgcgatg 2280
   ggtaacagtc ttggcggttt cgctaaatac tggcaggcgt ttcgtcagta tccccgttta 2340
   cagggcggct tcgtctggga ctgggtggat cagtcgctga ttaaatatga tgaaaacggc 2400
   aacccgtggt cggcttacgg cggtgatttt ggcgatacgc cgaacgatcg ccagttctgt 2460
   atgaacggtc tggtctttgc cgaccgcacg ccgcatccag cgctgacgga agcaaaacac 2520
   cagcagcagt ttttccagtt ccgtttatcc gggcaaacca tcgaagtgac cagcgaatac 2580
   ctgttccgtc atagcgataa cgagctcctg cactggatgg tggcgctgga tggtaagccg 2640
   ctggcaagcg gtgaagtgcc tctggatgtc gctccacaag gtaaacagtt gattgaactg 2700
   cctgaactac cgcagccgga gagcgccggg caactctggc tcacagtacg cgtagtgcaa 2760
   ccgaacgcga ccgcatggtc agaagccggg cacatcagcg cctggcagca gtggcgtctg 2820
   gcggaaaacc tcagtgtgac gctccccgcc gcgtcccacg ccatcccgca tctgaccacc 2880
   agcgaaatgg atttttgcat cgagctgggt aataagcgtt ggcaatttaa ccgccagtca 2940
   ggctttcttt cacagatgtg gattggcgat aaaaaacaac tgctgacgcc gctgcgcgat 3000
   cagttcaccc gtgcaccgct ggataacgac attggcgtaa gtgaagcgac ccgcattgac 3060
   cctaacgcct gggtcgaacg ctggaaggcg gcgggccatt accaggccga agcagcgttg 3120
   ttgcagtgca cggcagatac acttgctgat gcggtgctga ttacgaccgc tcacgcgtgg 3180
   cagcatcagg ggaaaacctt atttatcagc cggaaaacct accggattga tggtagtggt 3240
   caaatggcga ttaccgttga tgttgaagtg gcgagcgata caccgcatcc ggcgcggatt 3300
   ggcctgaact gccagctggc gcaggtagca gagcgggtaa actggctcgg attagggccg 3360
   caagaaaact atcccgaccg ccttactgcc gcctgttttg accgctggga tctgccattg 3420
   tcagacatgt ataccccgta cgtcttcccg agcgaaaacg gtctgcgctg cgggacgcgc 3480
   gaattgaatt atggcccaca ccagtggcgc ggcgacttcc agttcaacat cagccgctac 3540
   agtcaacagc aactgatgga aaccagccat cgccatctgc tgcacgcgga agaaggcaca 3600
   tggctgaata tcgacggttt ccatatgggg attggtggcg acgactcctg gagcccgtca 3660
   gtatcggcgg aattccagct gagcgccggt cgctaccatt accagttggt ctggtgtcag 3720
   gggatccccc gggctgcagc caatatggga tcggccattg aacaagatgg attgcacgca 3780
   ggttctccgg ccgcttgggt ggagaggcta ttcggctatg actgggcaca acagacaatc 3840
   ggctgctctg atgccgccgt gttccggctg tcagcgcagg ggcgcccggt tctttttgtc 3900
   aagaccgacc tgtccggtgc cctgaatgaa ctgcaggacg aggcagcgcg gctatcgtgg 3960
   ctggccacga cgggcgttcc ttgcgcagct gtgctcgacg ttgtcactga agcgggaagg 4020
   gactggctgc tattgggcga agtgccgggg caggatctcc tgtcatctca ccttgctcct 4080
   gccgagaaag tatccatcat ggctgatgca atgcggcggc tgcatacgct tgatccggct 4140
   acctgcccat tcgaccacca agcgaaacat cgcatcgagc gagcacgtac tcggatggaa 4200
   gccggtcttg tcgatcagga tgatctggac gaagagcatc aggggctcgc gccagccgaa 4260
   ctgttcgcca ggctcaaggc gcgcatgccc gacggcgagg atctcgtcgt gacccatggc 4320
   gatgcctgct tgccgaatat catggtggaa aatggccgct tttctggatt catcgactgt 4380
   ggccggctgg gtgtggcgga ccgctatcag gacatagcgt tggctacccg tgatattgct 4440
   gaagagcttg gcggcgaatg ggctgaccgc ttcctcgtgc tttacggtat cgccgctccc 4500
   gattcgcagc gcatcgcctt ctatcgcctt cttgacgagt tcttctgagg ggatcaattc 4560
   tctagagctc gctgatcagc ctcgactgtg ccttctagtt gccagccatc tgttgtttgc 4620
   ccctcccccg tgccttcctt gaccctggaa ggtgccactc ccactgtcct ttcctaataa 4680
   aatgaggaaa ttgcatcgca ttgtctgagt aggtgtcatt ctattctggg gggtggggtg 4740
   gggcaggaca gcaaggggga ggattgggaa gacaatagca ggcatgctgg ggatgcggtg 4800
   ggctctatgg cttctgagac ggaaagaacc agctggggct cgatcctcta gagtcgacct 4860
   cgatcgagat aacttcgtat aatgtatgct atacgaacgg tatctagagt cgactgttta 4920
   aacctgcagg catgcaagct tggcgtaatc atggtcatag ctgtttcctg tgtgaaattg 4980
   ttatccgctc acaattccac acaacatacg agccggaagc ataaagtgta aagcctgggg 5040
   tgcctaatga gtgagctaac tcacattaat tgcgttgcgc tcactgcccg ctttccagtc 5100
   gggaaacctg tcgtgccagc tgcattaatg aatcggccaa cgcgcgggga gaggcggttt 5160
   gcgtattggg cgctcttccg cttcctcgct cactgactcg ctgcgctcgg tcgttcggct 5220
   gcggcgagcg gtatcagctc actcaaaggc ggtaatacgg ttatccacag aatcagggga 5280
   taacgcagga aagaacatgt gagcaaaagg ccagcaaaag gccaggaacc gtaaaaaggc 5340
   cgcgttgctg gcgtttttcc ataggctccg cccccctgac gagcatcaca aaaatcgacg 5400
   ctcaagtcag aggtggcgaa acccgacagg actataaaga taccaggcgt ttccccctgg 5460
   aagctccctc gtgcgctctc ctgttccgac cctgccgctt accggatacc tgtccgcctt 5520
   tctcccttcg ggaagcgtgg cgctttctca tagctcacgc tgtaggtatc tcagttcggt 5580
   gtaggtcgtt cgctccaagc tgggctgtgt gcacgaaccc cccgttcagc ccgaccgctg 5640
   cgccttatcc ggtaactatc gtcttgagtc caacccggta agacacgact tatcgccact 5700
   ggcagcagcc actggtaaca ggattagcag agcgaggtat gtaggcggtg ctacagagtt 5760
   cttgaagtgg tggcctaact acggctacac tagaaggaca gtatttggta tctgcgctct 5820
   gctgaagcca gttaccttcg gaaaaagagt tggtagctct tgatccggca aacaaaccac 5880
   cgctggtagc ggtggttttt ttgtttgcaa gcagcagatt acgcgcagaa aaaaaggatc 5940
   tcaagaagat cctttgatct tttctacggg gtctgacgct cagtggaacg aaaactcacg 6000
   ttaagggatt ttggtcatga gattatcaaa aaggatcttc acctagatcc ttttaaatta 6060
   aaaatgaagt tttaaatcaa tctaaagtat atatgagtaa acttggtctg acagttacca 6120
   atgcttaatc agtgaggcac ctatctcagc gatctgtcta tttcgttcat ccatagttgc 6180
   ctgactcccc gtcgtgtaga taactacgat acgggagggc ttaccatctg gccccagtgc 6240
   tgcaatgata ccgcgagacc cacgctcacc ggctccagat ttatcagcaa taaaccagcc 6300
   agccggaagg gccgagcgca gaagtggtcc tgcaacttta tccgcctcca tccagtctat 6360
   taattgttgc cgggaagcta gagtaagtag ttcgccagtt aatagtttgc gcaacgttgt 6420
   tgccattgct acaggcatcg tggtgtcacg ctcgtcgttt ggtatggctt cattcagctc 6480
   cggttcccaa cgatcaaggc gagttacatg atcccccatg ttgtgcaaaa aagcggttag 6540
   ctccttcggt cctccgatcg ttgtcagaag taagttggcc gcagtgttat cactcatggt 6600
   tatggcagca ctgcataatt ctcttactgt catgccatcc gtaagatgct tttctgtgac 6660
   tggtgagtac tcaaccaagt cattctgaga atagtgtatg cggcgaccga gttgctcttg 6720
   cccggcgtca atacgggata ataccgcgcc acatagcaga actttaaaag tgctcatcat 6780
   tggaaaacgt tcttcggggc gaaaactctc aaggatctta ccgctgttga gatccagttc 6840
   gatgtaaccc actcgtgcac ccaactgatc ttcagcatct tttactttca ccagcgtttc 6900
   tgggtgagca aaaacaggaa ggcaaaatgc cgcaaaaaag ggaataaggg cgacacggaa 6960
   atgttgaata ctcatactct tcctttttca atattattga agcatttatc agggttattg 7020
   tctcatgagc ggatacatat ttgaatgtat ttagaaaaat aaacaaatag gggttccgcg 7080
   cacatttccc cgaaaagtgc cacctgacgt ctaagaaacc attattatca tgacattaac 7140
   ctataaaaat aggcgtatca cgaggccctt tcgtc                            7175
<210> 14
   <211> 5365
   <212> DNA
   <213> Artificial Sequence
<220>
   <223> Description of Artificial Sequence: vector pBABE-pgkCre
<400> 14
   ctgcagcctg aatatgggcc aaacaggata tctgtggtaa gcagttcctg ccccggctca 60
   gggccaagaa cagatggaac agctgaatat gggccaaaca ggatatctgt ggtaagcagt 120
   tcctgccccg gctcagggcc aagaacagat ggtccccaga tgcggtccag ccctcagcag 180
   tttctagaga accatcagat gtttccaggg tgccccaagg acctgaaatg accctgtgcc 240
   ttatttgaac taaccaatca gttcgcttct cgcttctgtt cgcgcgcttc tgctccccga 300
   gctcaataaa agagcccaca acccctcact cggggcgcca gtcctccgat tgactgagtc 360
   gcccgggtac ccgtgtatcc aataaaccct cttgcagttg catccgactt gtggtctcgc 420
   tgttccttgg gagggtctcc tctgagtgat tgactacccg tcagcggggg tctttcattt 480
   gggggctcgt ccgggatcgg gagacccctg cccagggacc accgacccac caccgggagg 540
   caagctggcc agcaacttat ctgtgtctgt ccgattgtct agtgtctatg actgatttta 600
   tgcgcctgcg tcggtactag ttagctaact agctctgtat ctggcggacc cgtggtggaa 660
   ctgacgagtt cggaacaccc ggccgcaacc ctgggagacg tcccagggac ttcgggggcc 720
   gtttttgtgg cccgacctga gtccaaaaat cccgatcgtt ttggactctt tggtgcaccc 780
   cccttagagg agggatatgt ggttctggta ggagacgaga acctaaaaca gttcccgcct 840
   ccgtctgaat ttttgctttc ggtttgggac cgaagccgcg ccgcgcgtct tgtctgctgc 900
   agcatcgttc tgtgttgtct ctgtctgact gtgtttctgt atttgtctga gaattagggc 960
   cagactgtta ccactccctt aagtttgacc ttaggtcact ggaaagatgt cgagcggatc 1020
   gctcacaacc agtcggtaga tgtcaagaag agacgttggg ttaccttctg ctctgcagaa 1080
   tggccaacct ttaacgtcgg atggccgcga gacggcacct ttaaccgaga cctcatcacc 1140
   caggttaaga tcaaggtctt ttcacctggc ccgcatggac acccagacca ggtcccctac 1200
   atcgtgacct gggaagcctt ggcttttgac ccccctccct gggtcaagcc ctttgtacac 1260
   cctaagcctc cgcctcctct tcctccatcc gccccgtctc tcccccttga acctcctcgt 1320
   tcgaccccgc ctcgatcctc cctttatcca gccctcactc cttctctagg cgccggccgg 1380
   atcccagtgt ggtggtacgg ccgctctaga ctcgaggaat tctacgggta ggggaggcgc 1440
   ttttcccaag gcagtctgga gcatgcgctt tagcagcccc gctggcactt ggcgctacac 1500
   aagtggcctc tggcctcgca cacattccac atccaccggt agcgccaacc ggctccgttc 1560
   tttggtggcc ccttcgcgcc acttctactc ctcccctagt caggaagttt cccccagcaa 1620
   gctcgcgtcg tgcaggacgt gacaaatgga agtagcacgt ctcactagtc tcgtgcagat 1680
   ggacagcacc gctgagcaat ggaagcgggt aggcctttgg ggcagcggcc aatagcagct 1740
   ttgttccttc gctttctggg ctcagaggct gggaaggggt gggtccgggg gcgggctcag 1800
   gggcgggctc aggggcgggc gggcgcccga aggtcctccc gaggcccggc attctgcacg 1860
   cttcaaaagc gcacgtctgc cgcgctgttc tcctcttcct catctccggg cctttcgacc 1920
   tgcagcgacc cgcttaacag cgtcaacagc gtgccgcaga tcagttctag catgcccaag 1980
   aagaagagga aggtgtccaa tttactgacc gtacaccaaa atttgcctgc attaccggtc 2040
   gatgcaacga gtgatgaggt tcgcaagaac ctgatggaca tgttcaggga tcgccaggcg 2100
   ttttctgagc atacctggaa aatgcttctg tccgtttgcc ggtcgtgggc ggcatggtgc 2160
   aagttgaata accggaaatg gtttcccgca gaacctgaag atgttcgcga ttatcttcta 2220
   tatcttcagg cgcgcggtct ggcagtaaaa actatccagc aacatttggg ccagctaaac 2280
   atgcttcatc gtcggtccgg gctgccacga ccaagtgaca gcaatgctgt ttcactggtt 2340
   atgcggcgga tccgaaaaga aaacgttgat gccggtgaac gtgcaaaaca ggctctagcg 2400
   ttcgaacgca ctgatttcga ccaggttcgt tcactcatgg aaaatagcga tcgctgccag 2460
   gatatacgta atctggcatt tctggggatt gcttataaca ccctgttacg tatagccgaa 2520
   attgccagga tcagggttaa agatatctca cgtactgacg gtgggagaat gttaatccat 2580
   attggcagaa cgaaaacgct ggttagcacc gcaggtgtag agaaggcact tagcctgggg 2640
   gtaactaaac tggtcgagcg atggatttcc gtctctggtg tagctgatga tccgaataac 2700
   tacctgtttt gccgggtcag aaaaaatggt gttgccgcgc catctgccac cagccagcta 2760
   tcaactcgcg ccctggaagg gatttttgaa gcaactcatc gattgattta cggcgctaag 2820
   gatgactctg gtcagagata cctggcctgg tctggacaca gtgcccgtgt cggagccgcg 2880
   cgagatatgg cccgcgctgg agtttcaata ccggagatca tgcaagctgg tggctggacc 2940
   aatgtaaata ttgtcatgaa ctatatccgt aacctggata gtgaaacagg ggcaatggtg 3000
   cgcctgctgg aagatggcga ttaggctaga gcggccgtag gaattcgcca gcacagtggt 3060
   cgaccagctg tgcgcatagt ggcttgaatc gataaaataa aagattttat ttagtctcca 3120
   gaaaaagggg ggaatgaaag accccacctg taggtttggc aagctagcac aacccctcac 3180
   tcggggcgcc agtcctccga ttgactgagt cgcccgggta cccgtgtatc caataaaccc 3240
   tcttgcagtt gcatccgact tgtggtctcg ctgttccttg ggagggtctc ctctgagtga 3300
   ttgactaccc gtcagcgggg gtctttcaca tgcagcatgt atcaaaatta atttggtttt 3360
   ttttcttaag tatttacatt aaatggccat agttgcatta atgaatcggc caacgcgcgg 3420
   ggagaggcgg tttgcgtatt gggcgctctt ccgcttcctc gctcactgac tcgctgcgct 3480
   cggtcgttcg gctgcggcga gcggtatcag ctcactcaaa ggcggtaata cggttatcca 3540
   cagaatcagg ggataacgca ggaaagaaca tgtgagcaaa aggccagcaa aaggccagga 3600
   accgtaaaaa ggccgcgttg ctggcgtttt tccataggct ccgcccccct gacgagcatc 3660
   acaaaaatcg acgctcaagt cagaggtggc gaaacccgac aggactataa agataccagg 3720
   cgtttccccc tggaagctcc ctcgtgcgct ctcctgttcc gaccctgccg cttaccggat 3780
   acctgtccgc ctttctccct tcgggaagcg tggcgctttc tcatagctca cgctgtaggt 3840
   atctcagttc ggtgtaggtc gttcgctcca agctgggctg tgtgcacgaa ccccccgttc 3900
   agcccgaccg ctgcgcctta tccggtaact atcgtcttga gtccaacccg gtaagacacg 3960
   acttatcgcc actggcagca gccactggta acaggattag cagagcgagg tatgtaggcg 4020
   gtgctacaga gttcttgaag tggtggccta actacggcta cactagaagg acagtatttg 4080
   gtatctgcgc tctgctgaag ccagttacct tcggaaaaag agttggtagc tcttgatccg 4140
   gcaaacaaac caccgctggt agcggtggtt tttttgtttg caagcagcag attacgcgca 4200
   gaaaaaaagg atctcaagaa gatcctttga tcttttctac ggggtctgac gctcagtgga 4260
   acgaaaactc acgttaaggg attttggtca tgagattatc aaaaaggatc ttcacctaga 4320
   tccttttgcg gccgcaaatc aatctaaagt atatatgagt aaacttggtc tgacagttac 4380
   caatgcttaa tcagtgaggc acctatctca gcgatctgtc tatttcgttc atccatagtt 4440
   gcctgactcc ccgtcgtgta gataactacg atacgggagg gcttaccatc tggccccagt 4500
   gctgcaatga taccgcgaga cccacgctca ccggctccag atttatcagc aataaaccag 4560
   ccagccggaa gggccgagcg cagaagtggt cctgcaactt tatccgcctc catccagtct 4620
   attaattgtt gccgggaagc tagagtaagt agttcgccag ttaatagttt gcgcaacgtt 4680
   gttgccattg ctgcaggcat cgtggtgtca cgctcgtcgt ttggtatggc ttcattcagc 4740
   tccggttccc aacgatcaag gcgagttaca tgatccccca tgttgtgcaa aaaagcggtt 4800
   agctccttcg gtcctccgat cgttgtcaga agtaagttgg ccgcagtgtt atcactcatg 4860
   gttatggcag cactgcataa ttctcttact gtcatgccat ccgtaagatg cttttctgtg 4920
   actggtgagt actcaaccaa gtcattctga gaatagtgta tgcggcgacc gagttgctct 4980
   tgcccggcgt caatacggga taataccgcg ccacatagca gaactttaaa agtgctcatc 5040
   attggaaaac gttcttcggg gcgaaaactc tcaaggatct taccgctgtt gagatccagt 5100
   tcgatgtaac ccactcgtgc acccaactga tcttcagcat cttttacttt caccagcgtt 5160
   tctgggtgag caaaaacagg aaggcaaaat gccgcaaaaa agggaataag ggcgacacgg 5220
   aaatgttgaa tactcatact cttccttttt caatattatt gaagcattta tcagggttat 5280
   tgtctcatga gcggatacat atttgaatgt atttagaaaa ataaacaaat aggggttccg 5340
   cgcacatttc cccgaaaagt gccac 5365
<210> 15
   <211> 3728
   <212> DNA
   <213> Artificial Sequence
<220>
   <223> Description of Artificial Sequence: vector pBABE-Srf
<400> 15
   ctgcagcctg aatatgggcc aaacaggata tctgtggtaa gcagttcctg ccccggctca 60
   gggccaagaa cagatggaac agctgaatat gggccaaaca ggatatctgt ggtaagcagt 120
   tcctgccccg gctcagggcc aagaacagat ggtccccaga tgcggtccag ccctcagcag 180
   tttctagaga accatcagat gtttccaggg tgccccaagg acctgaaatg accctgtgcc 240
   ttatttgaac taaccaatca gttcgcttct cgcttctgtt cgcgcgcttc tgctccccga 300
   gctcaataaa agagcccaca acccctcact cggggcgcca gtcctccgat tgactgagtc 360
   gcccgggtac ccgtgtatcc aataaaccct cttgcagttg catccgactt gtggtctcgc 420
   tgttccttgg gagggtctcc tctgagtgat tgactacccg tcagcggggg tctttcattt 480
   gggggctcgt ccgggatcgg gagacccctg cccagggacc accgacccac caccgggagg 540
   caagctggcc agcaacttat ctgtgtctgt ccgattgtct agtgtctatg actgatttta 600
   tgcgcctgcg tcggtactag ttagctaact agctctgtat ctggcggacc cgtggtggaa 660
   ctgacgagtt cggaacaccc ggccgcaacc ctgggagacg tcccagggac ttcgggggcc 720
   gtttttgtgg cccgacctga gtccaaaaat cccgatcgtt ttggactctt tggtgcaccc 780
   cccttagagg agggatatgt ggttctggta ggagacgaga acctaaaaca gttcccgcct 840
   ccgtctgaat ttttgctttc ggtttgggac cgaagccgcg ccgcgcgtct tgtctgctgc 900
   agcatcgttc tgtgttgtct ctgtctgact gtgtttctgt atttgtctga gaattagggc 960
   cagactgtta ccactccctt aagtttgacc ttaggtcact ggaaagatgt cgagcggatc 1020
   gctcacaacc agtcggtaga tgtcaagaag agacgttggg ttaccttctg ctctgcagaa 1080
   tggccaacct ttaacgtcgg atggccgcga gacggcacct ttaaccgaga cctcatcacc 1140
   caggttaaga tcaaggtctt ttcacctggc ccgcatggac acccagacca ggtcccctac 1200
   atcgtgacct gggaagcctt ggcttttgac ccccctccct gggtcaagcc ctttgtacac 1260
   cctaagcctc cgcctcctct tcctccatcc gccccgtctc tcccccttga acctcctcgt 1320
   tcgaccccgc ctcgatcctc cctttatcca gccctcactc cttctctagg cgccggccgg 1380
   atcccagtgt ggtggtacgt aggaattctc gagggcccgg gctcgaccag ctgtgcgcat 1440
   agtggcttga atcgataaaa taaaagattt tatttagtct ccagaaaaag gggggaatga 1500
   aagaccccac ctgtaggttt ggcaagctag cacaacccct cactcggggc gccagtcctc 1560
   cgattgactg agtcgcccgg gtacccgtgt atccaataaa ccctcttgca gttgcatccg 1620
   acttgtggtc tcgctgttcc ttgggagggt ctcctctgag tgattgacta cccgtcagcg 1680
   ggggtctttc acatgcagca tgtatcaaaa ttaatttggt tttttttctt aagtatttac 1740
   attaaatggc catagttgca ttaatgaatc ggccaacgcg cggggagagg cggtttgcgt 1800
   attgggcgct cttccgcttc ctcgctcact gactcgctgc gctcggtcgt tcggctgcgg 1860
   cgagcggtat cagctcactc aaaggcggta atacggttat ccacagaatc aggggataac 1920
   gcaggaaaga acatgtgagc aaaaggccag caaaaggcca ggaaccgtaa aaaggccgcg 1980
   ttgctggcgt ttttccatag gctccgcccc cctgacgagc atcacaaaaa tcgacgctca 2040
   agtcagaggt ggcgaaaccc gacaggacta taaagatacc aggcgtttcc ccctggaagc 2100
   tccctcgtgc gctctcctgt tccgaccctg ccgcttaccg gatacctgtc cgcctttctc 2160
   ccttcgggaa gcgtggcgct ttctcatagc tcacgctgta ggtatctcag ttcggtgtag 2220
   gtcgttcgct ccaagctggg ctgtgtgcac gaaccccccg ttcagcccga ccgctgcgcc 2280
   ttatccggta actatcgtct tgagtccaac ccggtaagac acgacttatc gccactggca 2340
   gcagccactg gtaacaggat tagcagagcg aggtatgtag gcggtgctac agagttcttg 2400
   aagtggtggc ctaactacgg ctacactaga aggacagtat ttggtatctg cgctctgctg 2460
   aagccagtta ccttcggaaa aagagttggt agctcttgat ccggcaaaca aaccaccgct 2520
   ggtagcggtg gtttttttgt ttgcaagcag cagattacgc gcagaaaaaa aggatctcaa 2580
   gaagatcctt tgatcttttc tacggggtct gacgctcagt ggaacgaaaa ctcacgttaa 2640
   gggattttgg tcatgagatt atcaaaaagg atcttcacct agatcctttt gcggccgcaa 2700
   atcaatctaa agtatatatg agtaaacttg gtctgacagt taccaatgct taatcagtga 2760
   ggcacctatc tcagcgatct gtctatttcg ttcatccata gttgcctgac tccccgtcgt 2820
   gtagataact acgatacggg agggcttacc atctggcccc agtgctgcaa tgataccgcg 2880
   agacccacgc tcaccggctc cagatttatc agcaataaac cagccagccg gaagggccga 2940
   gcgcagaagt ggtcctgcaa ctttatccgc ctccatccag tctattaatt gttgccggga 3000
   agctagagta agtagttcgc cagttaatag tttgcgcaac gttgttgcca ttgctgcagg 3060
   catcgtggtg tcacgctcgt cgtttggtat ggcttcattc agctccggtt cccaacgatc 3120
   aaggcgagtt acatgatccc ccatgttgtg caaaaaagcg gttagctcct tcggtcctcc 3180
   gatcgttgtc agaagtaagt tggccgcagt gttatcactc atggttatgg cagcactgca 3240
   taattctctt actgtcatgc catccgtaag atgcttttct gtgactggtg agtactcaac 3300
   caagtcattc tgagaatagt gtatgcggcg accgagttgc tcttgcccgg cgtcaatacg 3360
   ggataatacc gcgccacata gcagaacttt aaaagtgctc atcattggaa aacgttcttc 3420
   ggggcgaaaa ctctcaagga tcttaccgct gttgagatcc agttcgatgt aacccactcg 3480
   tgcacccaac tgatcttcag catcttttac tttcaccagc gtttctgggt gagcaaaaac 3540
   aggaaggcaa aatgccgcaa aaaagggaat aagggcgaca cggaaatgtt gaatactcat 3600
   actcttcctt tttcaatatt attgaagcat ttatcagggt tattgtctca tgagcggata 3660
   catatttgaa tgtatttaga aaaataaaca aataggggtt ccgcgcacat ttccccgaaa 3720
   agtgccac                                                          3728
<210> 16
   <211> 7573
   <212> DNA
   <213> Artificial Sequence
<220>
   <223> Description of Artificial Sequence: vector pRK73
<400> 16
   cgtcatcacc gaaacgcgcg aggcagctgt ggaatgtgtg tcagttaggg tgtggaaagt 60
   ccccaggctc cccagcaggc agaagtatgc aaagcatgca tctcaattag tcagcaacca 120
   ggctccccag caggcagaag tgtgcaaagc atgcatctca attagtcagc aaccatagtc 180
   ccgcccctaa ctccgcccat cccgccccta actccgccca gttccgccca ttctccgccc 240
   catggctgac taattttttt tatttatgca gaggccgagg ccgcctcggc ctaggaacag 300
   tcgacgacac tgcagagacc tacttcacta acaaccggta cagttcgtgg accagatggg 360
   tgaggtggag tacgcgcccg gggagcccaa gggcacgccc tggcacccgc accgcggctt 420
   cgagaccgtc acgaataact tcgtatagca tacattatac gaagttataa gcttgcatgc 480
   ctgcaggtcg gccgccacga ccggccggcc ggtgccgcca ccatcccctg acccacgccc 540
   ctgacccctc acaaggagac gaccttccat gaccgagtac aagcccacgg tgcgcctcgc 600
   cacccgcgac gacgtccccc gggccgtacg caccctcgcc gccgcgttcg ccgactaccc 660
   cgccacgcgc cacaccgtcg acccggaccg ccacatcgag cgggtcaccg agctgcaaga 720
   actcttcctc acgcgcgtcg ggctcgacat cggcaaggtg tgggtcgcgg acgacggcgc 780
   cgcggtggcg gtctggacca cgccggagag cgtcgaagcg ggggcggtgt tcgccgagat 840
   cggcccgcgc atggccgagt tgagcggttc ccggctggcc gcgcagcaac agatggaagg 900
   cctcctggcg ccgcaccggc ccaaggagcc cgcgtggttc ctggccaccg tcggcgtctc 960
   gcccgaccac cagggcaagg gtctgggcag cgccgtcgtg ctccccggag tggaggcggc 1020
   cgagcgcgcc ggggtgcccg ccttcctgga gacctccgcg ccccgcaacc tccccttcta 1080
   cgagcggctc ggcttcaccg tcaccgccga cgtcgagtgc ccgaaggacc gcgcgacctg 1140
   gtgcatgacc cgcaagcccg gtgcctgacg cccgccccac gacccgcagc gcccgaccga 1200
   aaggagcgca cgaccccatg gctccgaccg aagccgaccc gggcggcccc gccgaccccg 1260
   cacccgcccc cgaggcccac cgactctaga ggatcataat cagccatacc acatttgtag 1320
   aggttttact tgctttaaaa aacctcccac acctccccct gaacctgaaa cataaaatga 1380
   atgcaattgt tgttgttaac ttgtttattg cagcttataa tggttacaaa taaagcaata 1440
   gcatcacaaa tttcacaaat aaagcatttt tttcactgca ttctagttgt ggtttgtcca 1500
   aactcatcaa tgtatcttat catgtctgga tccgaccatg gtggcccggt ataacttcgt 1560
   ataatgtatg ctatacgaag ttatggatcc agaagcggtt ttcgggagta gtgccccaac 1620
   tggggtaacc tttgagttct ctcagttggg ggcgtagggt cgccgacatg acacggatcc 1680
   agacatgata agatacattg atgagtttgg acaaaccaca actagaatgc agtgaaaaaa 1740
   atgctttatt tgtgaaattt gtgatgctat tgctttattt gtaaccatta taagctgcaa 1800
   taaacaagtt cgagtagctt ggcactggcc gtcgttttac aacgtcgtga ctgggaaaac 1860
   cctggcgtta cccaacttaa tcgccttgca gcacatcccc ctttcgccag ctggcgtaat 1920
   agcgaagagg cccgcaccga tcgcccttcc caacagttgc gcagcctgaa tggcgaatgg 1980
   cgctttgcct ggtttccggc accagaagcg gtgccggaaa gctggctgga gtgcgatctt 2040
   cctgaggccg atactgtcgt cgtcccctca aactggcaga tgcacggtta cgatgcgccc 2100
   atctacacca acgtaaccta tcccattacg gtcaatccgc cgtttgttcc cacggagaat 2160
   ccgacgggtt gttactcgct cacatttaat gttgatgaaa gctggctaca ggaaggccag 2220
   acgcgaatta tttttgatgg cgttaactcg gcgtttcatc tgtggtgcaa cgggcgctgg 2280
   gtcggttacg gccaggacag tcgtttgccg tctgaatttg acctgagcgc atttttacgc 2340
   gccggagaaa accgcctcgc ggtgatggtg ctgcgttgga gtgacggcag ttatctggaa 2400
   gatcaggata tgtggcggat gagcggcatt ttccgtgacg tctcgttgct gcataaaccg 2460
   actacacaaa tcagcgattt ccatgttgcc actcgcttta atgatgattt cagccgcgct 2520
   gtactggagg ctgaagttca gatgtgcggc gagttgcgtg actacctacg ggtaacagtt 2580
   tctttatggc agggtgaaac gcaggtcgcc agcggcaccg cgcctttcgg cggtgaaatt 2640
   atcgatgagc gtggtggtta tgccgatcgc gtcacactac gtctgaacgt cgaaaacccg 2700
   aaactgtgga gcgccgaaat cccgaatctc tatcgtgcgg tggttgaact gcacaccgcc 2760
   gacggcacgc tgattgaagc agaagcctgc gatgtcggtt tccgcgaggt gcggattgaa 2820
   aatggtctgc tgctgctgaa cggcaagccg ttgctgattc gaggcgttaa ccgtcacgag 2880
   catcatcctc tgcatggtca ggtcatggat gagcagacga tggtgcagga tatcctgctg 2940
   atgaagcaga acaactttaa cgccgtgcgc tgttcgcatt atccgaacca tccgctgtgg 3000
   tacacgctgt gcgaccgcta cggcctgtat gtggtggatg aagccaatat tgaaacccac 3060
   ggcatggtgc caatgaatcg tctgaccgat gatccgcgct ggctaccggc gatgagcgaa 3120
   cgcgtaacgc gaatggtgca gcgcgatcgt aatcacccga gtgtgatcat ctggtcgctg 3180
   gggaatgaat caggccacgg cgctaatcac gacgcgctgt atcgctggat caaatctgtc 3240
   gatccttccc gcccggtgca gtatgaaggc ggcggagccg acaccacggc caccgatatt 3300
   atttgcccga tgtacgcgcg cgtggatgaa gaccagccct tcccggctgt gccgaaatgg 3360
   tccatcaaaa aatggctttc gctacctgga gagacgcgcc cgctgatcct ttgcgaatac 3420
   gcccacgcga tgggtaacag tcttggcggt ttcgctaaat actggcaggc gtttcgtcag 3480
   tatccccgtt tacagggcgg cttcgtctgg gactgggtgg atcagtcgct gattaaatat 3540
   gatgaaaacg gcaacccgtg gtcggcttac ggcggtgatt ttggcgatac gccgaacgat 3600
   cgccagttct gtatgaacgg tctggtcttt gccgaccgca cgccgcatcc agcgctgacg 3660
   gaagcaaaac accagcagca gtttttccag ttccgtttat ccgggcaaac catcgaagtg 3720
   accagcgaat acctgttccg tcatagcgat aacgagctcc tgcactggat ggtggcgctg 3780
   gatggtaagc cgctggcaag cggtgaagtg cctctggatg tcgctccaca aggtaaacag 3840
   ttgattgaac tgcctgaact accgcagccg gagagcgccg ggcaactctg gctcacagta 3900
   cgcgtagtgc aaccgaacgc gaccgcatgg tcagaagccg ggcacatcag cgcctggcag 3960
   cagtggcgtc tggcggaaaa cctcagtgtg acgctccccg ccgcgtccca cgccatcccg 4020
   catctgacca ccagcgaaat ggatttttgc atcgagctgg gtaataagcg ttggcaattt 4080
   aaccgccagt caggctttct ttcacagatg tggattggcg ataaaaaaca actgctgacg 4140
   ccgctgcgcg atcagttcac ccgtgcaccg ctggataacg acattggcgt aagtgaagcg 4200
   acccgcattg accctaacgc ctgggtcgaa cgctggaagg cggcgggcca ttaccaggcc 4260
   gaagcagcgt tgttgcagtg cacggcagat acacttgctg atgcggtgct gattacgacc 4320
   gctcacgcgt ggcagcatca ggggaaaacc ttatttatca gccggaaaac ctaccggatt 4380
   gatggtagtg gtcaaatggc gattaccgtt gatgttgaag tggcgagcga tacaccgcat 4440
   ccggcgcgga ttggcctgaa ctgccagctg gcgcaggtag cagagcgggt aaactggctc 4500
   ggattagggc cgcaagaaaa ctatcccgac cgccttactg ccgcctgttt tgaccgctgg 4560
   gatctgccat tgtcagacat gtataccccg tacgtcttcc cgagcgaaaa cggtctgcgc 4620
   tgcgggacgc gcgaattgaa ttatggccca caccagtggc gcggcgactt ccagttcaac 4680
   atcagccgct acagtcaaca gcaactgatg gaaaccagcc atcgccatct gctgcacgcg 4740
   gaagaaggca catggctgaa tatcgacggt ttccatatgg ggattggtgg cgacgactcc 4800
   tggagcccgt cagtatcggc ggaattccag ctgagcgccg gtcgctacca ttaccagttg 4860
   gtctggtgtc aaaaataata ataaccgggc aggggggatc tttgtgaagg aaccttactt 4920
   ctgtggtgtg acataattgg acaaactacc tacagagatt taaagctcta aggtaaatat 4980
   aaaattttta agtgtataat gtgttaaact actgattcta attgtttgtg tattttagat 5040
   tccaacctat ggaactgatg aatgggagca gtggtggaat gccagatcca gacatgataa 5100
   gatacattga tgagtttgga caaaccacaa ctagaatgca gtgaaaaaaa tgctttattt 5160
   gtgaaatttg tgatgctatt gctttatttg taaccattat aagctgcaat aaacaagtta 5220
   acaacaacaa ttgcattcat tttatgtttc aggttcaggg ggaggtgtgg gaggtttttt 5280
   aaagcaagta aaacctctac aaatgtggta tggctgatta tgatctgcgg ccgcagggcc 5340
   tcgtgatacg cctattttta taggttaatg tcatgataat aatggtttct tagacgtcag 5400
   gtggcacttt tcggggaaat gtgcgcggaa cccctatttg tttatttttc taaatacatt 5460
   caaatatgta tccgctcatg agacaataac cctgataaat gcttcaataa tattgaaaaa 5520
   ggaagagtat gagtattcaa catttccgtg tcgcccttat tccctttttt gcggcatttt 5580
   gccttcctgt ttttgctcac ccagaaacgc tggtgaaagt aaaagatgct gaagatcagt 5640
   tgggtgcacg agtgggttac atcgaactgg atctcaacag cggtaagatc cttgagagtt 5700
   ttcgccccga agaacgtttt ccaatgatga gcacttttaa agttctgcta tgtggcgcgg 5760
   tattatcccg tattgacgcc gggcaagagc aactcggtcg ccgcatacac tattctcaga 5820
   atgacttggt tgagtactca ccagtcacag aaaagcatct tacggatggc atgacagtaa 5880
   gagaattatg cagtgctgcc ataaccatga gtgataacac tgcggccaac ttacttctga 5940
   caacgatcgg aggaccgaag gagctaaccg cttttttgca caacatgggg gatcatgtaa 6000
   ctcgccttga tcgttgggaa ccggagctga atgaagccat accaaacgac gagcgtgaca 6060
   ccacgatgcc tgtagcaatg gcaacaacgt tgcgcaaact attaactggc gaactactta 6120
   ctctagcttc ccggcaacaa ttaatagact ggatggaggc ggataaagtt gcaggaccac 6180
   ttctgcgctc ggcccttccg gctggctggt ttattgctga taaatctgga gccggtgagc 6240
   gtgggtctcg cggtatcatt gcagcactgg ggccagatgg taagccctcc cgtatcgtag 6300
   ttatctacac gacggggagt caggcaacta tggatgaacg aaatagacag atcgctgaga 6360
   taggtgcctc actgattaag cattggtaac tgtcagacca agtttactca tatatacttt 6420
   agattgattt aaaacttcat ttttaattta aaaggatcta ggtgaagatc ctttttgata 6480
   atctcatgac caaaatccct taacgtgagt tttcgttcca ctgagcgtca gaccccgtag 6540
   aaaagatcaa aggatcttct tgagatcctt tttttctgcg cgtaatctgc tgcttgcaaa 6600
   caaaaaaacc accgctacca gcggtggttt gtttgccgga tcaagagcta ccaactcttt 6660
   ttccgaaggt aactggcttc agcagagcgc agataccaaa tactgtcctt ctagtgtagc 6720
   cgtagttagg ccaccacttc aagaactctg tagcaccgcc tacatacctc gctctgctaa 6780
   tcctgttacc agtggctgct gccagtggcg ataagtcgtg tcttaccggg ttggactcaa 6840
   gacgatagtt accggataag gcgcagcggt cgggctgaac ggggggttcg tgcacacagc 6900
   ccagcttgga gcgaacgacc tacaccgaac tgagatacct acagcgtgag ctatgagaaa 6960
   gcgccacgct tcccgaaggg agaaaggcgg acaggtatcc ggtaagcggc agggtcggaa 7020
   caggagagcg cacgagggag cttccagggg gaaacgcctg gtatctttat agtcctgtcg 7080
   ggtttcgcca cctctgactt gagcgtcgat ttttgtgatg ctcgtcaggg gggcggagcc 7140
   tatggaaaaa cgccagcaac gcggcctttt tacggttcct ggccttttgc tggccttttg 7200
   ctcacatgtt ctttcctgcg ttatcccctg attctgtgga taaccgtatt accgcctttg 7260
   agtgagctga taccgctcgc cgcagccgaa cgaccgagcg cagcgagtca gtgagcgagg 7320
   aagcggaaga gcgcccaata cgcaaaccgc ctctccccgc gcgttggccg attcattaat 7380
   gcagctggca cgacaggttt cccgactgga aagcgggcag tgagcgcaac gcaattaatg 7440
   tgagttagct cactcattag gcaccccagg ctttacactt tatgcttccg gctcgtatgt 7500
   tgtgtggaat tgtgagcgga taacaatttc acacaggaaa cagctatgac catgattacg 7560
   ccaagctggc gcg                                                     7573
<210> 17
   <211> 86
   <212> DNA
   <213> Artificial Sequence
<220>
   <223> Description of Artificial Sequence: primer C31-4
<400> 17
   cgtgacggtc tcgaagccgc ggtgcgggtg ccagggcgtg cccttgggct ccccgggcgc 60
   gtactccacc tcacccatct ggtcca 86
<210> 18
   <211> 86
   <212> DNA
   <213> Artificial Sequence
<220>
   <223> Description of Artificial Sequence: primer C31-5
<400> 18
   cgtggaccag atgggtgagg tggagtacgc gcccggggag cccaagggca cgccctggcc 60
   cacgcaccgc ggcttcgaga ccgtca 86
<210> 19
   <211> 90
   <212> DNA
   <213> Artificial Sequence
<220>
   <223> Description of Artificial Sequence: primer C31-6-2 -
<400> 19
   gatccagaag cggttttcgg gagtagtgcc ccaactgggg taacctttga gttctctcag 60
   ttgggggcgt agggtcgccg acatgacacg 90
<210> 20
   <211> 90
   <212> DNA
   <213> Artificial Sequence
<220>
   <223> Description of Artificial Sequence: primer C31-7-2
<400> 20
   gatccgtgtc atgtcggcga ccctacgccc ccaactgaga gaactcaaag gttaccccag 60
   ttggggcact actcccgaaa accgcttctg 90
<210> 21
   <211> 5711
   <212> DNA
   <213> Artificial Sequence
<220>
   <223> Description of Artificial Sequence: vector pRK65
<400> 21
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcccgatatg acacaagggg ttgtgaccgg ggtggacacg tacgcgggtg 1020
   cttacgaccg tcagtcgcgc gagcgcgaga attcgagcgc agcaagccca gcgacacagc 1080
   gtagcgccaa cgaagacaag gcggccgacc ttcagcgcga agtcgagcgc gacgggggcc 1140
   ggttcaggtt cgtcgggcat ttcagcgaag cgccgggcac gtcggcgttc gggacggcgg 1200
   agcgcccgga gttcgaacgc atcctgaacg aatgccgcgc cgggcggctc aacatgatca 1260
   ttgtctatga cgtgtcgcgc ttctcgcgcc tgaaggtcat ggacgcgatt ccgattgtct 1320
   cggaattgct cgccctgggc gtgacgattg tttccactca ggaaggcgtc ttccggcagg 1380
   gaaacgtcat ggacctgatt cacctgatta tgcggctcga cgcgtcgcac aaagaatctt 1440
   cgctgaagtc ggcgaagatt ctcgacacga agaaccttca gcgcgaattg ggcgggtacg 1500
   tcggcgggaa ggcgccttac ggcttcgagc ttgtttcgga gacgaaggag atcacgcgca 1560
   acggccgaat ggtcaatgtc gtcatcaaca agcttgcgca ctcgaccact ccccttaccg 1620
   gacccttcga gttcgagccc gacgtaatcc ggtggtggtg gcgtgagatc aagacgcaca 1680
   aacaccttcc cttcaagccg ggcagtcaag ccgccattca cccgggcagc atcacggggc 1740
   tttgtaagcg catggacgct gacgccgtgc cgacccgggg cgagacgatt gggaagaaga 1800
   ccgcttcaag cgcctgggac ccggcaaccg ttatgcgaat ccttcgggac ccgcgtattg 1860
   cgggcttcgc cgctgaggtg atctacaaga agaagccgga cggcacgccg accacgaaga 1920
   ttgagggtta ccgcattcag cgcgacccga tcacgctccg gccggtcgag cttgattgcg 1980
   gaccgatcat cgagcccgct gagtggtatg agcttcaggc gtggttggac ggcagggggc 2040
   gcggcaaggg gctttcccgg gggcaagcca ttctgtccgc catggacaag ctgtactgcg 2100
   agtgtggcgc cgtcatgact tcgaagcgcg gggaagaatc gatcaaggac tcttaccgct 2160
   gccgtcgccg gaaggtggtc gacccgtccg cacctgggca gcacgaaggc acgtgcaacg 2220
   tcagcatggc ggcactcgac aagttcgttg cggaacgcat cttcaacaag atcaggcacg 2280
   ccgaaggcga cgaagagacg ttggcgcttc tgtgggaagc cgcccgacgc ttcggcaagc 2340
   tcactgaggc gcctgagaag agcggcgaac gggcgaacct tgttgcggag cgcgccgacg 2400
   ccctgaacgc ccttgaagag ctgtacgaag accgcgcggc aggcgcgtac gacggacccg 2460
   ttggcaggaa gcacttccgg aagcaacagg cagcgctgac gctccggcag caaggggcgg 2520
   aagagcggct tgccgaactt gaagccgccg aagccccgaa gcttcccctt gaccaatggt 2580
   tccccgaaga cgccgacgct gacccgaccg gccctaagtc gtggtggggg cgcgcgtcag 2640
   tagacgacaa gcgcgtgttc gtcgggctct tcgtagacaa gatcgttgtc acgaagtcga 2700
   ctacgggcag gggcagggga acgcccatcg agaagcgcgc ttcgatcacg tgggcgaagc 2760
   cgccgaccga cgacgacgaa gacgacgccc aggacggcac ggaagacgta gcggcgtagg 2820
   cggcgcccgg gctcgagatc caggcgcgga tcaataaaag atcattattt tcaatagatc 2880
   tgtgtgttgg ttttttgtgt gccttggggg agggggaggc cagaatgagg cgcggccaag 2940
   ggggaggggg aggccagaat gaccttgggg gagggggagg ccagaatgac cttgggggag 3000
   ggggaggcca gaatgaggcg cgcccccggg taccgagctc gaattcactg gccgtcgttt 3060
   tacaacgtcg tgactgggaa aaccctggcg ttacccaact taatcgcctt gcagcacatc 3120
   cccctttcgc cagctggcgt aatagcgaag aggcccgcac cgatcgccct tcccaacagt 3180
   tgcgcagcct gaatggcgaa tggcgcctga tgcggtattt tctccttacg catctgtgcg 3240
   gtatttcaca ccgcatatgg tgcactctca gtacaatctg ctctgatgcc gcatagttaa 3300
   gccagccccg acacccgcca acacccgctg acgcgccctg acgggcttgt ctgctcccgg 3360
   catccgctta cagacaagct gtgaccgtct ccgggagctg catgtgtcag aggttttcac 3420
   cgtcatcacc gaaacgcgcg agacgaaagg gcctcgtgat acgcctattt ttataggtta 3480
   atgtcatgat aataatggtt tcttagacgt caggtggcac ttttcgggga aatgtgcgcg 3540
   gaacccctat ttgtttattt ttctaaatac attcaaatat gtatccgctc atgagacaat 3600
   aaccctgata aatgcttcaa taatattgaa aaaggaagag tatgagtatt caacatttcc 3660
   gtgtcgccct tattcccttt tttgcggcat tttgccttcc tgtttttgct cacccagaaa 3720
   cgctggtgaa agtaaaagat gctgaagatc agttgggtgc acgagtgggt tacatcgaac 3780
   tggatctcaa cagcggtaag atccttgaga gttttcgccc cgaagaacgt tttccaatga 3840
   tgagcacttt taaagttctg ctatgtggcg cggtattatc ccgtattgac gccgggcaag 3900
   agcaactcgg tcgccgcata cactattctc agaatgactt ggttgagtac tcaccagtca 3960
   cagaaaagca tcttacggat ggcatgacag taagagaatt atgcagtgct gccataacca 4020
   tgagtgataa cactgcggcc aacttacttc tgacaacgat cggaggaccg aaggagctaa 4080
   ccgctttttt gcacaacatg ggggatcatg taactcgcct tgatcgttgg gaaccggagc 4140
   tgaatgaagc cataccaaac gacgagcgtg acaccacgat gcctgtagca atggcaacaa 4200
   cgttgcgcaa actattaact ggcgaactac ttactctagc ttcccggcaa caattaatag 4260
   actggatgga ggcggataaa gttgcaggac cacttctgcg ctcggccctt ccggctggct 4320
   ggtttattgc tgataaatct ggagccggtg agcgtgggtc tcgcggtatc attgcagcac 4380
   tggggccaga tggtaagccc tcccgtatcg tagttatcta cacgacgggg agtcaggcaa 4440
   ctatggatga acgaaataga cagatcgctg agataggtgc ctcactgatt aagcattggt 4500
   aactgtcaga ccaagtttac tcatatatac tttagattga tttaaaactt catttttaat 4560
   ttaaaaggat ctaggtgaag atcctttttg ataatctcat gaccaaaatc ccttaacgtg 4620
   agttttcgtt ccactgagcg tcagaccccg tagaaaagat caaaggatct tcttgagatc 4680
   ctttttttct gcgcgtaatc tgctgcttgc aaacaaaaaa accaccgcta ccagcggtgg 4740
   tttgtttgcc ggatcaagag ctaccaactc tttttccgaa ggtaactggc ttcagcagag 4800
   cgcagatacc aaatactgtc cttctagtgt agccgtagtt aggccaccac ttcaagaact 4860
   ctgtagcacc gcctacatac ctcgctctgc taatcctgtt accagtggct gctgccagtg 4920
   gcgataagtc gtgtcttacc gggttggact caagacgata gttaccggat aaggcgcagc 4980
   ggtcgggctg aacggggggt tcgtgcacac agcccagctt ggagcgaacg acctacaccg 5040
   aactgagata cctacagcgt gagctatgag aaagcgccac gcttcccgaa gggagaaagg 5100
   cggacaggta tccggtaagc ggcagggtcg gaacaggaga gcgcacgagg gagcttccag 5160
   ggggaaacgc ctggtatctt tatagtcctg tcgggtttcg ccacctctga cttgagcgtc 5220
   gatttttgtg atgctcgtca ggggggcgga gcctatggaa aaacgccagc aacgcggcct 5280
   ttttacggtt cctggccttt tgctggcctt ttgctcacat gttctttcct gcgttatccc 5340
   ctgattctgt ggataaccgt attaccgcct ttgagtgagc tgataccgct cgccgcagcc 5400
   gaacgaccga gcgcagcgag tcagtgagcg aggaagcgga agagcgccca atacgcaaac 5460
   cgcctctccc cgcgcgttgg ccgattcatt aatgcagctg gcacgacagg tttcccgact 5520
   ggaaagcggg cagtgagcgc aacgcaatta atgtgagtta gctcactcat taggcacccc 5580
   aggctttaca ctttatgctt ccggctcgta tgttgtgtgg aattgtgagc ggataacaat 5640
   ttcacacagg aaacagctat gaccatgatt acgccaagct agcccgggct agcttgcatg 5700
   cctgcaggtt t                                                      5711
<210> 22
   <211> 45
   <212> DNA
   <213> Artificial Sequence
<220>
   <223> Description of Artificial Sequence: primer PC31-1
<400> 22
   ataagaatgc ggccgcccga tatgacacaa ggggttgtga ccggg 45
<210> 23
   <211> 40
   <212> DNA
   <213> Artificial Sequence
<220>
   <223> Description of Artificial Sequence: primer PC31-3
<400> 23
   ataagaatgc ggccgcatcc gccgctacgt cttccgtgcc 40
<210> 24
   <211> 24
   <212> DNA
   <213> Artificial Sequence
<220>
   <223> Description of Artificial Sequence: primer PC31-8
<400> 24
   cccgttggca ggaagcactt ccgg 24
<210> 25
   <211> 55
   <212> DNA
   <213> Artificial Sequence
<220>
   <223> Description of Artificial Sequence: primer PC31-9
<400> 25
   ggatcctcga gccgcgggcg gccgcctacg ccgctacgtc ttccgtgccg tcctg 55
<210> 26
   <211> 25
   <212> DNA
   <213> Artificial Sequence
<220>
   <223> Description of Artificial Sequence: primer P64-1
<400> 26
   tcagcaacca ggctccccag caggc 25
<210> 27
   <211> 27
   <212> DNA
   <213> Artificial Sequence
<220>
   <223> Description of Artificial Sequence: primer P64-3
<400> 27
   tagaggatca taaatcagcc ataccac 27
<210> 28
   <211> 770
   <212> DNA.
   <213> Artificial Sequence
<220>
   <223> Description of Artificial Sequence: DNA sequence derived from vector pRK80b
<400> 28
   ggtaccgagc tcggatccac tagtaacggc cgccagtgtg ctggaattcg gctttcagca 60
   accaggctcc ccagcaggca gaagtatgca aagcatgcat ctcaattagt cagcaaccat 120
   agncccgccc ctaactccgc ccatcccgcc cctaactccg cccagttccg cccattctcc 180
   gccccatggc tgactaattt tttttattta tgcagaggcc gaggccgcct cggcctagga 240
   acagtcgacg acactgcaga gacctacttc actaacaacc ggtacagttc gtggaccaga 300
   tgggtgaggt ggagtacgcg cccggggagc ccaaaggtta ccccagttgg ggcactactc 360
   ccgaaaaccg cttctggatc cataacttcg tatagcatac attatacgaa gttataccgg 420
   gccaccatgg tcggatccag acatgataag atacattgat gagtttggac aaaccacaac 480
   tagaatgcag tgaaaaaaat gctttatttg tgaaatttgt gatgctattg ctttatttgt 540
   aaccattata agctgcaata aacaagttaa caacaacaat tgcattcatt ttatgtttca 600
   ggttcagggg gaggtgtggg aggtttttta aagcaagtaa aacctctaca aatgtggtat 660
   ggctgattta tgatcctcta agccgaattc tgcagatatc catcacactg gcggccgctc 720
   gagcatgcat ctagagggcc caattcgccc tatagtgagt cgtattacaa 770
<210> 29
   <211> 667
   <212> DNA
   <213> Artificial Sequence
<220>
   <223> Description of Artificial Sequence: DNA sequence derived from vector pRK80c
<400> 29
   ggtaccgagc tcggatccnc tagtaacggc cgccagtgtg ctggaattcg gcttgcccag 60
   ttccgcccat tctccgcccc atggctgact aattttttta tttatgcaga ggccgaggcc 120
   gcctcggcct aggaacagtc gacgacactg cagagaccta cttcactaac aaccggtaca 180
   gttcgtggac cagatgggtg aggtggagta cgcgcccggg gagcccaaag gttaccccag 240
   ttggggcact actcccgaaa accgcttctg gatccataac ttcgtatagc atacattata 300
   cgaagttata ccgggccacc atggtcggat ccagacatga taagatacat tgatgagttt 360
   ggacaaacca caactagaat gcagtgaaaa aaatgcttta tttgtgaaat ttgtgatgct 420
   attgctttat ttgtaaccat tataagctgc aataaacaag ttaacaacaa caattgcatt 480
   cattttatgt ttcaggttca gggggaggtg tgggaggttt tttaaagcaa gtaaaacctc 540
   tacaaatgtg gtatggctga tttatgatcc tctaaagccg aattctgcag atatccatca 600
   cactggcggc cgctcgagca tgcatctaga gggcccaatt cgccctatag tgagtcgtat 660
   tacaatt 667
<210> 30
   <211> 7573
   <212> DNA
   <213> Artificial Sequence
<220>
   <223> Description of Artificial Sequence: vector pRK73-inv
<400> 30
   cgtcatcacc gaaacgcgcg aggcagctgt ggaatgtgtg tcagttaggg tgtggaaagt 60
   ccccaggctc cccagcaggc agaagtatgc aaagcatgca tctcaattag tcagcaacca 120
   ggctccccag caggcagaag tgtgcaaagc atgcatctca attagtcagc aaccatagtc 180
   ccgcccctaa ctccgcccat cccgccccta actccgccca gttccgccca ttctccgccc 240
   catggctgac taattttttt tatttatgca gaggccgagg ccgcctcggc ctaggaacag 300
   tcgacgacac tgcagagacc tacttcacta acaaccggta cagttcgtgg accagatggg 360
   tgaggtggag tacgcgcccg gggagcccaa caaaggttac cccagttggg gcactactcc 420
   cgaaaaccgc ttctggatcc ataacttcgt atagcataca ttatacgaag ttataccggg 480
   ccaccatggt cggatccaga catgataaga tacattgatg agtttggaca aaccacaact 540
   agaatgcagt gaaaaaaatg ctttatttgt gaaatttgtg atgctattgc tttatttgta 600
   accattataa gctgcaataa acaagttaac aacaacaatt gcattcattt tatgtttcag 660
   gttcaggggg aggtgtggga ggttttttaa agcaagtaaa acctctacaa atgtggtatg 720
   gctgattatg atcctctaga gtcggtgggc ctcgggggcg ggtgcggggt cggcggggcc 780
   gcccgggtcg gcttcggtcg gagccatggg gtcgtgcgct cctttcggtc gggcgctgcg 840
   ggtcgtgggg cgggcgtcag gcaccgggct tgcgggtcat gcaccaggtc gcgcggtcct 900
   tcgggcactc gacgtcggcg gtgacggtga agccgagccg ctcgtagaag gggaggttgc 960
   ggggcgcgga ggtctccagg aaggcgggca ccccggcgcg ctcggccgcc tccactccgg 1020
   ggagcacgac ggcgctgccc agacccttgc cctggtggtc gggcgagacg ccgacggtgg 1080
   ccaggaacca cgcgggctcc ttgggccggt gcggcgccag gaggccttcc atctgttgct 1140
   gcgcggccag ccgggaaccg ctcaactcgg ccatgcgcgg gccgatctcg gcgaacaccg 1200
   cccccgcttc gacgctctcc ggcgtggtcc agaccgccac cgcggcgccg tcgtccgcga 1260
   cccacacctt gccgatgtcg agcccgacgc gcgtgaggaa gagttcttgc agctcggtga 1320
   cccgctcgat gtggcggtcc gggtcgacgg tgtggcgcgt ggcggggtag tcggcgaacg 1380
   cggcggcgag ggtgcgtacg gcccggggga cgtcgtcgcg ggtggcgagg cgcaccgtgg 1440
   gcttgtactc ggtcatggaa ggtcgtctcc ttgtgagggg tcaggggcgt gggtcagggg 1500
   atggtggcgg caccggccgg ccggtcgtgg cggccgacct gcaggcatgc aagcttataa 1560
   cttcgtataa tgtatgctat acgaagttat tcgtgacggt ctcgaagccg cggtgcgggt 1620
   gccagggcgt gcccagttct ctcagttggg ggcgtagggt cgccgacatg acacggatcc 1680
   agacatgata agatacattg atgagtttgg acaaaccaca actagaatgc agtgaaaaaa 1740
   atgctttatt tgtgaaattt gtgatgctat tgctttattt gtaaccatta taagctgcaa 1800
   taaacaagtt cgagtagctt ggcactggcc gtcgttttac aacgtcgtga ctgggaaaac 1860
   cctggcgtta cccaacttaa tcgccttgca gcacatcccc ctttcgccag ctggcgtaat 1920
   agcgaagagg cccgcaccga tcgcccttcc caacagttgc gcagcctgaa tggcgaatgg 1980
   cgctttgcct ggtttccggc accagaagcg gtgccggaaa gctggctgga gtgcgatctt 2040
   cctgaggccg atactgtcgt cgtcccctca aactggcaga tgcacggtta cgatgcgccc 2100
   atctacacca acgtaaccta tcccattacg gtcaatccgc cgtttgttcc cacggagaat 2160
   ccgacgggtt gttactcgct cacatttaat gttgatgaaa gctggctaca ggaaggccag 2220
   acgcgaatta tttttgatgg cgttaactcg gcgtttcatc tgtggtgcaa cgggcgctgg 2280
   gtcggttacg gccaggacag tcgtttgccg tctgaatttg acctgagcgc atttttacgc 2340
   gccggagaaa accgcctcgc ggtgatggtg ctgcgttgga gtgacggcag ttatctggaa 2400
   gatcaggata tgtggcggat gagcggcatt ttccgtgacg tctcgttgct gcataaaccg 2460
   actacacaaa tcagcgattt ccatgttgcc actcgcttta atgatgattt cagccgcgct 2520
   gtactggagg ctgaagttca gatgtgcggc gagttgcgtg actacctacg ggtaacagtt 2580
   tctttatggc agggtgaaac gcaggtcgcc agcggcaccg cgcctttcgg cggtgaaatt 2640
   atcgatgagc gtggtggtta tgccgatcgc gtcacactac gtctgaacgt cgaaaacccg 2700
   aaactgtgga gcgccgaaat cccgaatctc tatcgtgcgg tggttgaact gcacaccgcc 2760
   gacggcacgc tgattgaagc agaagcctgc gatgtcggtt tccgcgaggt gcggattgaa 2820
   aatggtctgc tgctgctgaa cggcaagccg ttgctgattc gaggcgttaa ccgtcacgag 2880
   catcatcctc tgcatggtca ggtcatggat gagcagacga tggtgcagga tatcctgctg 2940
   atgaagcaga acaactttaa cgccgtgcgc tgttcgcatt atccgaacca tccgctgtgg 3000
   tacacgctgt gcgaccgcta cggcctgtat gtggtggatg aagccaatat tgaaacccac 3060
   ggcatggtgc caatgaatcg tctgaccgat gatccgcgct ggctaccggc gatgagcgaa 3120
   cgcgtaacgc gaatggtgca gcgcgatcgt aatcacccga gtgtgatcat ctggtcgctg 3180
   gggaatgaat caggccacgg cgctaatcac gacgcgctgt atcgctggat caaatctgtc 3240
   gatccttccc gcccggtgca gtatgaaggc ggcggagccg acaccacggc caccgatatt 3300
   atttgcccga tgtacgcgcg cgtggatgaa gaccagccct tcccggctgt gccgaaatgg 3360
   tccatcaaaa aatggctttc gctacctgga gagacgcgcc cgctgatcct ttgcgaatac 3420
   gcccacgcga tgggtaacag tcttggcggt ttcgctaaat actggcaggc gtttcgtcag 3480
   tatccccgtt tacagggcgg cttcgtctgg gactgggtgg atcagtcgct gattaaatat 3540
   gatgaaaacg gcaacccgtg gtcggcttac ggcggtgatt ttggcgatac gccgaacgat 3600
   cgccagttct gtatgaacgg tctggtcttt gccgaccgca cgccgcatcc agcgctgacg 3660
   gaagcaaaac accagcagca gtttttccag ttccgtttat ccgggcaaac catcgaagtg 3720
   accagcgaat acctgttccg tcatagcgat aacgagctcc tgcactggat ggtggcgctg 3780
   gatggtaagc cgctggcaag cggtgaagtg cctctggatg tcgctccaca aggtaaacag 3840
   ttgattgaac tgcctgaact accgcagccg gagagcgccg ggcaactctg gctcacagta 3900
   cgcgtagtgc aaccgaacgc gaccgcatgg tcagaagccg ggcacatcag cgcctggcag 3960
   cagtggcgtc tggcggaaaa cctcagtgtg acgctccccg ccgcgtccca cgccatcccg 4020
   catctgacca ccagcgaaat ggatttttgc atcgagctgg gtaataagcg ttggcaattt 4080
   aaccgccagt caggctttct ttcacagatg tggattggcg ataaaaaaca actgctgacg 4140
   ccgctgcgcg atcagttcac ccgtgcaccg ctggataacg acattggcgt aagtgaagcg 4200
   acccgcattg accctaacgc ctgggtcgaa cgctggaagg cggcgggcca ttaccaggcc 4260
   gaagcagcgt tgttgcagtg cacggcagat acacttgctg atgcggtgct gattacgacc 4320
   gctcacgcgt ggcagcatca ggggaaaacc ttatttatca gccggaaaac ctaccggatt 4380
   gatggtagtg gtcaaatggc gattaccgtt gatgttgaag tggcgagcga tacaccgcat 4440
   ccggcgcgga ttggcctgaa ctgccagctg gcgcaggtag cagagcgggt aaactggctc 4500
   ggattagggc cgcaagaaaa ctatcccgac cgccttactg ccgcctgttt tgaccgctgg 4560
   gatctgccat tgtcagacat gtataccccg tacgtcttcc cgagcgaaaa cggtctgcgc 4620
   tgcgggacgc gcgaattgaa ttatggccca caccagtggc gcggcgactt ccagttcaac 4680
   atcagccgct acagtcaaca gcaactgatg gaaaccagcc atcgccatct gctgcacgcg 4740
   gaagaaggca catggctgaa tatcgacggt ttccatatgg ggattggtgg cgacgactcc 4800
   tggagcccgt cagtatcggc ggaattccag ctgagcgccg gtcgctacca ttaccagttg 4860
   gtctggtgtc aaaaataata ataaccgggc aggggggatc tttgtgaagg aaccttactt 4920
   ctgtggtgtg acataattgg acaaactacc tacagagatt taaagctcta aggtaaatat 4980
   aaaattttta agtgtataat gtgttaaact actgattcta attgtttgtg tattttagat 5040
   tccaacctat ggaactgatg aatgggagca gtggtggaat gccagatcca gacatgataa 5100
   gatacattga tgagtttgga caaaccacaa ctagaatgca gtgaaaaaaa tgctttattt 5160
   gtgaaatttg tgatgctatt gctttatttg taaccattat aagctgcaat aaacaagtta 5220
   acaacaacaa ttgcattcat tttatgtttc aggttcaggg ggaggtgtgg gaggtttttt 5280
   aaagcaagta aaacctctac aaatgtggta tggctgatta tgatctgcgg ccgcagggcc 5340
   tcgtgatacg cctattttta taggttaatg tcatgataat aatggtttct tagacgtcag 5400
   gtggcacttt tcggggaaat gtgcgcggaa cccctatttg tttatttttc taaatacatt 5460
   caaatatgta tccgctcatg agacaataac cctgataaat gcttcaataa tattgaaaaa 5520
   ggaagagtat gagtattcaa catttccgtg tcgcccttat tccctttttt gcggcatttt 5580
   gccttcctgt ttttgctcac ccagaaacgc tggtgaaagt aaaagatgct gaagatcagt 5640
   tgggtgcacg agtgggttac atcgaactgg atctcaacag cggtaagatc cttgagagtt 5700
   ttcgccccga agaacgtttt ccaatgatga gcacttttaa agttctgcta tgtggcgcgg 5760
   tattatcccg tattgacgcc gggcaagagc aactcggtcg ccgcatacac tattctcaga 5820
   atgacttggt tgagtactca ccagtcacag aaaagcatct tacggatggc atgacagtaa 5880
   gagaattatg cagtgctgcc ataaccatga gtgataacac tgcggccaac ttacttctga 5940
   caacgatcgg aggaccgaag gagctaaccg cttttttgca caacatgggg gatcatgtaa 6000
   ctcgccttga tcgttgggaa ccggagctga atgaagccat accaaacgac gagcgtgaca 6060
   ccacgatgcc tgtagcaatg gcaacaacgt tgcgcaaact attaactggc gaactactta 6120
   ctctagcttc ccggcaacaa ttaatagact ggatggaggc ggataaagtt gcaggaccac 6180
   ttctgcgctc ggcccttccg gctggctggt ttattgctga taaatctgga gccggtgagc 6240
   gtgggtctcg cggtatcatt gcagcactgg ggccagatgg taagccctcc cgtatcgtag 6300
   ttatctacac gacggggagt caggcaacta tggatgaacg aaatagacag atcgctgaga 6360
   taggtgcctc actgattaag cattggtaac tgtcagacca agtttactca tatatacttt 6420
   agattgattt aaaacttcat ttttaattta aaaggatcta ggtgaagatc ctttttgata 6480
   atctcatgac caaaatccct taacgtgagt tttcgttcca ctgagcgtca gaccccgtag 6540
   aaaagatcaa aggatcttct tgagatcctt tttttctgcg cgtaatctgc tgcttgcaaa 6600
   caaaaaaacc accgctacca gcggtggttt gtttgccgga tcaagagcta ccaactcttt 6660
   ttccgaaggt aactggcttc agcagagcgc agataccaaa tactgtcctt ctagtgtagc 6720
   cgtagttagg ccaccacttc aagaactctg tagcaccgcc tacatacctc gctctgctaa 6780
   tcctgttacc agtggctgct gccagtggcg ataagtcgtg tcttaccggg ttggactcaa 6840
   gacgatagtt accggataag gcgcagcggt cgggctgaac ggggggttcg tgcacacagc 6900
   ccagcttgga gcgaacgacc tacaccgaac tgagatacct acagcgtgag ctatgagaaa 6960
   gcgccacgct tcccgaaggg agaaaggcgg acaggtatcc ggtaagcggc agggtcggaa 7020
   caggagagcg cacgagggag cttccagggg gaaacgcctg gtatctttat agtcctgtcg 7080
   ggtttcgcca cctctgactt gagcgtcgat ttttgtgatg ctcgtcaggg gggcggagcc 7140
   tatggaaaaa cgccagcaac gcggcctttt tacggttcct ggccttttgc tggccttttg 7200
   ctcacatgtt ctttcctgcg ttatcccctg attctgtgga taaccgtatt accgcctttg 7260
   agtgagctga taccgctcgc cgcagccgaa cgaccgagcg cagcgagtca gtgagcgagg 7320
   aagcggaaga gcgcccaata cgcaaaccgc ctctccccgc gcgttggccg attcattaat 7380
   gcagctggca cgacaggttt cccgactgga aagcgggcag tgagcgcaac gcaattaatg 7440
   tgagttagct cactcattag gcaccccagg ctttacactt tatgcttccg gctcgtatgt 7500
   tgtgtggaat tgtgagcgga taacaatttc acacaggaaa cagctatgac catgattacg 7560
   ccaagctggc gcg                                                     7573
<210> 31
   <211> 8153
   <212> DNA
   <213> Artificial Sequence
<220>
   <223> Description of Artificial Sequence: vector pRK74
<400> 31
   cgcgccggat ccttaaaaca gtccgatgta cgggccagat atacgcgttg acattgatta 60
   ttgactagtt attaatagta atcaattacg gggtcattag ttcatagccc atatatggag 120
   ttccgcgtta cataacttac ggtaaatggc ccgcctggct gaccgcccaa cgacccccgc 180
   ccattgacgt caataatgac gtatgttccc atagtaacgc caatagggac tttccattga 240
   cgtcaatggg tggactattt acggtaaact gcccacttgg cagtacatca agtgtatcat 300
   atgccaagta cgccccctat tgacgtcaat gacggtaaat ggcccgcctg gcattatgcc 360
   cagtacatga ccttatggga ctttcctact tggcagtaca tctacgtatt agtcatcgct 420
   attaccatgg tgatgcggtt ttggcagtac atcaatgggc gtggatagcg gtttgactca 480
   cggggatttc caagtctcca ccccattgac gtcaatggga gtttgttttg gcaccaaaat 540
   caacgggact ttccaaaatg tcgtaacaac tccgccccat tgacgcaaat gggcggtagg 600
   cgtgtacggt gggaggtcta tataagcaga gctctctggc taactagaga acccactgct 660
   tactggctta tcgaaattaa tacgactcac tatagggaga cccaagctga ctctagactt 720
   aattaagcgt tggggtgagt actccctctc aaaagcgggc atgacttctg cgctaagatt 780
   gtcagtttcc aaaaacgagg aggatttgat attcacctgg cccgcggtga tgcctttgag 840
   ggtggccgcg tccatctggt cagaaaagac aatctttttg ttgtcaagct taagtctaga 900
   taacttcgta tagcatacat tatacgaacg gtactcgagg ccgcagatca taatcagcca 960
   taccacattt gtagaggttt tacttgcttt aaaaaacctc ccacacctcc ccctgaacct 1020
   gaaacataaa atgaatgcaa ttgttgttgt taacttgttt attgcagctt ataatggtta 1080
   caaataaagc aatagcatca caaatttcac aaataaagca tttttttcac tgcattctag 1140
   ttgtggtttg tccaaactca tcaatgtatc ttatcatgtc tggatctggc attccaccac 1200
   tgctcccatt catcagttcc ataggttgga atctaaaata cacaaacaat tagaatcagt 1260
   agtttaacac attatacact taaaaatttt atatttacct tagagcttta aatctctgta 1320
   ggtagtttgt ccaattatgt cacaccacag aagtaaggtt ccttcacaaa gatcccccct 1380
   gcccggttat tattattttt gacaccagac caactggtaa tggtagcgac cggcgctcag 1440
   ctggaattcc gccgatactg acgggctcca ggagtcgtcg ccaccaatcc ccatatggaa 1500
   accgtcgata ttcagccatg tgccttcttc cgcgtgcagc agatggcgat ggctggtttc 1560
   catcagttgc tgttgactgt agcggctgat gttgaactgg aagtcgccgc gccactggtg 1620
   tgggccataa ttcaattcgc gcgtcccgca gcgcagaccg ttttcgctcg ggaagacgta 1680
   cggggtatac atgtctgaca atggcagatc ccagcggtca aaacaggcgg cagtaaggcg 1740
   gtcgggatag ttttcttgcg gccctaatcc gagccagttt acccgctctg ctacctgcgc 1800
   cagctggcag ttcaggccaa tccgcgccgg atgcggtgta tcgctcgcca cttcaacatc 1860
   aacggtaatc gccatttgac cactaccatc aatccggtag gttttccggc tgataaataa 1920
   ggttttcccc tgatgctgcc acgcgtgagc ggtcgtaatc agcaccgcat cagcaagtgt 1980
   atctgccgtg cactgcaaca acgctgcttc ggcctggtaa tggcccgccg ccttccagcg 2040
   ttcgacccag gcgttagggt caatgcgggt cgcttcactt acgccaatgt cgttatccag 2100
   cggtgcacgg gtgaactgat cgcgcagcgg cgtcagcagt tgttttttat cgccaatcca 2160
   catctgtgaa agaaagcctg actggcggtt aaattgccaa cgcttattac ccagctcgat 2220
   gcaaaaatcc atttcgctgg tggtcagatg cgggatggcg tgggacgcgg cggggagcgt 2280
   cacactgagg ttttccgcca gacgccactg ctgccaggcg ctgatgtgcc cggcttctga 2340
   ccatgcggtc gcgttcggtt gcactacgcg tactgtgagc cagagttgcc cggcgctctc 2400
   cggctgcggt agttcaggca gttcaatcaa ctgtttacct tgtggagcga catccagagg 2460
   cacttcaccg cttgccagcg gcttaccatc cagcgccacc atccagtgca ggagctcgtt 2520
   atcgctatga cggaacaggt attcgctggt cacttcgatg gtttgcccgg ataaacggaa 2580
   ctggaaaaac tgctgctggt gttttgcttc cgtcagcgct ggatgcggcg tgcggtcggc 2640
   aaagaccaga ccgttcatac agaactggcg atcgttcggc gtatcgccaa aatcaccgcc 2700
   gtaagccgac cacgggttgc cgttttcatc atatttaatc agcgactgat ccacccagtc 2760
   ccagacgaag ccgccctgta aacggggata ctgacgaaac gcctgccagt atttagcgaa 2820
   accgccaaga ctgttaccca tcgcgtgggc gtattcgcaa aggatcagcg ggcgcgtctc 2880
   tccaggtagc gaaagccatt ttttgatgga ccatttcggc acagccggga agggctggtc 2940
   ttcatccacg cgcgcgtaca tcgggcaaat aatatcggtg gccgtggtgt cggctccgcc 3000
   gccttcatac tgcaccgggc gggaaggatc gacagatttg atccagcgat acagcgcgtc 3060
   gtgattagcg ccgtggcctg attcattccc cagcgaccag atgatcacac tcgggtgatt 3120
   acgatcgcgc tgcaccattc gcgttacgcg ttcgctcatc gccggtagcc agcgcggatc 3180
   atcggtcaga cgattcattg gcaccatgcc gtgggtttca atattggctt catccaccac 3240
   atacaggccg tagcggtcgc acagcgtgta ccacagcgga tggttcggat aatgcgaaca 3300
   gcgcacggcg ttaaagttgt tctgcttcat cagcaggata tcctgcacca tcgtctgctc 3360
   atccatgacc tgaccatgca gaggatgatg ctcgtgacgg ttaacgcctc gaatcagcaa 3420
   cggcttgccg ttcagcagca gcagaccatt ttcaatccgc acctcgcgga aaccgacatc 3480
   gcaggcttct gcttcaatca gcgtgccgtc ggcggtgtgc agttcaacca ccgcacgata 3540
   gagattcggg atttcggcgc tccacagttt cgggttttcg acgttcagac gtagtgtgac 3600
   gcgatcggca taaccaccac gctcatcgat aatttcaccg ccgaaaggcg cggtgccgct 3660
   ggcgacctgc gtttcaccct gccataaaga aactgttacc cgtaggtagt cacgcaactc 3720
   gccgcacatc tgaacttcag cctccagtac agcgcggctg aaatcatcat taaagcgagt 3780
   ggcaacatgg aaatcgctga tttgtgtagt cggtttatgc agcaacgaga cgtcacggaa 3840
   aatgccgctc atccgccaca tatcctgatc ttccagataa ctgccgtcac tccaacgcag 3900
   caccatcacc gcgaggcggt tttctccggc gcgtaaaaat gcgctcaggt caaattcaga 3960
   cggcaaacga ctgtcctggc cgtaaccgac ccagcgcccg ttgcaccaca gatgaaacgc 4020
   cgagttaacg ccatcaaaaa taattcgcgt ctggccttcc tgtagccagc tttcatcaac 4080
   attaaatgtg agcgagtaac aacccgtcgg attctccgtg ggaacaaacg gcggattgac 4140
   cgtaatggga taggttacgt tggtgtagat gggcgcatcg taaccgtgca tctgccagtt 4200
   tgaggggacg acgacagtat cggcctcagg aagatcgcac tccagccagc tttccggcac 4260
   cgcttctggt gccggaaacc aggcaaagcg ccattcgcca ttcaggctgc gcaactgttg 4320
   ggaagggcga tcggtgcggg cctcttcgct attacgccag ctggcgaaag ggggatgtgc 4380
   tgcaaggcga ttaagttggg taacgccagg gttttcccag tcacgacgtt gtaaaacgac 4440
   gggatcgatc tcgccataca gcgcgttgaa acgctgggca atatcgcggc tcagttcgag 4500
   gtgctgtttc tggtcttcac ccaccggcat ggtattatca tcgtgttttt caaaggaaaa 4560
   ccacgtcccc gtggttcggg gggcctagac gtttttttaa cctcgactaa acacatgtaa 4620
   agcatgtgca ccgaggcccc agatcagatc ccatacaatg gggtaccttc tgggcatcct 4680
   tcagcccctt gttgaatacg cttgaggaga gccatttgac tctttccaca actatccaac 4740
   tcacaacgtg gcactggggt tgtgccgcct ttgcaggtgt atcttataca cgtggctttt 4800
   ggccgcagag gcacctgtcg ccaggtgggg ggttccgctg cctgcaaagg gtcgctacag 4860
   acgttgtttg tcttcaagaa gcttccagag gaactgcttc cttcacgaca ttcaacagac 4920
   cttgcattcc tttggcgaga ggggaaagac ccctaggaat gctcgtcaag aagacagggc 4980
   caggtttccg ggccctcaca ttgccaaaag acggcaatat ggtggaaaat cacatataga 5040
   caaacgcaca ccggccttat tccaagcggc ttcggccagt aacgttaggg gggggggagg 5100
   gagaggggcg gaattcgata tcaagcttat cgataccgta gtactggaaa gaccgcgaag 5160
   agtttgtcct caaccgcgag ctgtggaaaa aaaagggaca ggataagtat gacatcatca 5220
   aggaaaccct ggactactgc gccctagacg tgcaggtcac ctcgatcgag ataacttcgt 5280
   ataatgtatg ctatacgaac ggtatctaga gtcgactgtt ttcgagttaa cacgtgctag 5340
   cgcttaagct tgaagttcct attccgaagt tcctattctc tagaaagtat aggaacttcg 5400
   gcgcgccgtc gacgtttaaa catgcatgaa gttcctattc cgaagttcct attctctaga 5460
   aagtatagga acttcataaa acctgcaggc atgcaagctt ggcgtaatca tggtcatagc 5520
   tgtttcctgt gtgaaattgt tatccgctca caattccaca caacatacga gccggaagca 5580
   taaagtgtaa agcctggggt gcctaatgag tgagctaact cacattaatt gcgttgcgct 5640
   cactgcccgc tttccagtcg ggaaacctgt cgtgccagct gcattaatga atcggccaac 5700
   gcgcggggag aggcggtttg cgtattgggc gctcttccgc ttcctcgctc actgactcgc 5760
   tgcgctcggt cgttcggctg cggcgagcgg tatcagctca ctcaaaggcg gtaatacggt 5820
   tatccacaga atcaggggat aacgcaggaa agaacatgtg agcaaaaggc cagcaaaagg 5880
   ccaggaaccg taaaaaggcc gcgttgctgg cgtttttcca taggctccgc ccccctgacg 5940
   agcatcacaa aaatcgacgc tcaagtcaga ggtggcgaaa cccgacagga ctataaagat 6000
   accaggcgtt tccccctgga agctccctcg tgcgctctcc tgttccgacc ctgccgctta 6060
   ccggatacct gtccgccttt ctcccttcgg gaagcgtggc gctttctcat agctcacgct 6120
   gtaggtatct cagttcggtg taggtcgttc gctccaagct gggctgtgtg cacgaacccc 6180
   ccgttcagcc cgaccgctgc gccttatccg gtaactatcg tcttgagtcc aacccggtaa 6240
   gacacgactt atcgccactg gcagcagcca ctggtaacag gattagcaga gcgaggtatg 6300
   taggcggtgc tacagagttc ttgaagtggt ggcctaacta cggctacact agaaggacag 6360
   tatttggtat ctgcgctctg ctgaagccag ttaccttcgg aaaaagagtt ggtagctctt 6420
   gatccggcaa acaaaccacc gctggtagcg gtggtttttt tgtttgcaag cagcagatta 6480
   cgcgcagaaa aaaaggatct caagaagatc ctttgatctt ttctacgggg tctgacgctc 6540
   agtggaacga aaactcacgt taagggattt tggtcatgag attatcaaaa aggatcttca 6600
   cctagatcct tttaaattaa aaatgaagtt ttaaatcaat ctaaagtata tatgagtaaa 6660
   cttggtctga cagttaccaa tgcttaatca gtgaggcacc tatctcagcg atctgtctat 6720
   ttcgttcatc catagttgcc tgactccccg tcgtgtagat aactacgata cgggagggct 6780
   taccatctgg ccccagtgct gcaatgatac cgcgagaccc acgctcaccg gctccagatt 6840
   tatcagcaat aaaccagcca gccggaaggg ccgagcgcag aagtggtcct gcaactttat 6900
   ccgcctccat ccagtctatt aattgttgcc gggaagctag agtaagtagt tcgccagtta 6960
   atagtttgcg caacgttgtt gccattgcta caggcatcgt ggtgtcacgc tcgtcgtttg 7020
   gtatggcttc attcagctcc ggttcccaac gatcaaggcg agttacatga tcccccatgt 7080
   tgtgcaaaaa agcggttagc tccttcggtc ctccgatcgt tgtcagaagt aagttggccg 7140
   cagtgttatc actcatggtt atggcagcac tgcataattc tcttactgtc atgccatccg 7200
   taagatgctt ttctgtgact ggtgagtact caaccaagtc attctgagaa tagtgtatgc 7260
   ggcgaccgag ttgctcttgc ccggcgtcaa tacgggataa taccgcgcca catagcagaa 7320
   ctttaaaagt gctcatcatt ggaaaacgtt cttcggggcg aaaactctca aggatcttac 7380
   cgctgttgag atccagttcg atgtaaccca ctcgtgcacc caactgatct tcagcatctt 7440
   ttactttcac cagcgtttct gggtgagcaa aaacaggaag gcaaaatgcc gcaaaaaagg 7500
   gaataagggc gacacggaaa tgttgaatac tcatactctt cctttttcaa tattattgaa 7560
   gcatttatca gggttattgt ctcatgagcg gatacatatt tgaatgtatt tagaaaaata 7620
   aacaaatagg ggttccgcgc acatttcccc gaaaagtgcc acctgacgtc taagaaacca 7680
   ttattatcat gacattaacc tataaaaata ggcgtatcac gaggcccttt cgtctcgcgc 7740
   gtttcggtga tgacggtgaa aacctctgac acatgcagct cccggagacg gtcacagctt 7800
   gtctgtaagc ggatgccggg agcagacaag cccgtcaggg cgcgtcagcg ggtgttggcg 7860
   ggtgtcgggg ctggcttaac tatgcggcat cagagcagat tgtactgaga gtgcaccata 7920
   tgcggtgtga aataccgcac agatgcgtaa ggagaaaata ccgcatcagg cgccattcgc 7980
   cattcaggct gcgcaactgt tgggaagggc gatcggtgcg ggcctcttcg ctattacgcc 8040
   agctggcgaa agggggatgt gctgcaaggc gattaagttg ggtaacgcca gggttttccc 8100
   agtcacgacg ttgtaaaacg acggccagtg aattcgagct cggtacccgg ggg 8153
<210> 32
   <211> 8062
   <212> DNA
   <213> Artificial Sequence
<220>
   <223> Description of Artificial Sequence: vector pRK76
<400> 32
   cgcgccggat ccttaattaa gtctagataa cttcgtatag catacatata cgaagttatc 60
   tcgatcgagg tgacctgcac gtctagggcg cagtagtcca gggtttcctt gatgatgtca 120
   tacttatcct gtcccttttt tttccacagc tcgcggttga ggacaaactc ttcgcggtct 180
   ttccagtact acggtatcga taagcttgat atcgaattcc gcccctctcc ctcccccccc 240
   cctaacgtta ctggccgaag ccgcttggaa taaggccggt gtgcgtttgt ctatatgtga 300
   ttttccacca tattgccgtc ttttggcaat gtgagggccc ggaaacctgg ccctgtcttc 360
   ttgacgagca ttcctagggg tctttcccct ctcgccaaag gaatgcaagg tctgttgaat 420
   gtcgtgaagg aagcagttcc tctggaagct tcttgaagac aaacaacgtc tgtagcgacc 480
   ctttgcaggc agcggaaccc cccacctggc gacaggtgcc tctgcggcca aaagccacgt 540
   gtataagata cacctgcaaa ggcggcacaa ccccagtgcc acgttgtgag ttggatagtt 600
   gtggaaagag tcaaatggct ctcctcaagc gtattcaaca aggggctgaa ggatgcccag 660
   aaggtacccc attgtatggg atctgatctg gggcctcggt gcacatgctt tacatgtgtt 720
   tagtcgaggt taaaaaaacg tctaggcccc ccgaaccacg gggacgtggt tttcctttga 780
   aaaacacgat gataatacca tgccggtggg tgaagaccag aaacagcacc tcgaactgag 840
   ccgcgatatt gcccagcgtt tcaacgcgct gtatggcgag atcgatcccg tcgttttaca 900
   acgtcgtgac tgggaaaacc ctggcgttac ccaacttaat cgccttgcag cacatccccc 960
   tttcgccagc tggcgtaata gcgaagaggc ccgcaccgat cgcccttccc aacagttgcg 1020
   cagcctgaat ggcgaatggc gctttgcctg gtttccggca ccagaagcgg tgccggaaag 1080
   ctggctggag tgcgatcttc ctgaggccga tactgtcgtc gtcccctcaa actggcagat 1140
   gcacggttac gatgcgccca tctacaccaa cgtaacctat cccattacgg tcaatccgcc 1200
   gtttgttccc acggagaatc cgacgggttg ttactcgctc acatttaatg ttgatgaaag 1260
   ctggctacag gaaggccaga cgcgaattat ttttgatggc gttaactcgg cgtttcatct 1320
   gtggtgcaac gggcgctggg tcggttacgg ccaggacagt cgtttgccgt ctgaatttga 1380
   cctgagcgca tttttacgcg ccggagaaaa ccgcctcgcg gtgatggtgc tgcgttggag 1440
   tgacggcagt tatctggaag atcaggatat gtggcggatg agcggcattt tccgtgacgt 1500
   ctcgttgctg cataaaccga ctacacaaat cagcgatttc catgttgcca ctcgctttaa 1560
   tgatgatttc agccgcgctg tactggaggc tgaagttcag atgtgcggcg agttgcgtga 1620
   ctacctacgg gtaacagttt ctttatggca gggtgaaacg caggtcgcca gcggcaccgc 1680
   gcctttcggc ggtgaaatta tcgatgagcg tggtggttat gccgatcgcg tcacactacg 1740
   tctgaacgtc gaaaacccga aactgtggag cgccgaaatc ccgaatctct atcgtgcggt 1800
   ggttgaactg cacaccgccg acggcacgct gattgaagca gaagcctgcg atgtcggttt 1860
   ccgcgaggtg cggattgaaa atggtctgct gctgctgaac ggcaagccgt tgctgattcg 1920
   aggcgttaac cgtcacgagc atcatcctct gcatggtcag gtcatggatg agcagacgat 1980
   ggtgcaggat atcctgctga tgaagcagaa caactttaac gccgtgcgct gttcgcatta 2040
   tccgaaccat ccgctgtggt acacgctgtg cgaccgctac ggcctgtatg tggtggatga 2100
   agccaatatt gaaacccacg gcatggtgcc aatgaatcgt ctgaccgatg atccgcgctg 2160
   gctaccggcg atgagcgaac gcgtaacgcg aatggtgcag cgcgatcgta atcacccgag 2220
   tgtgatcatc tggtcgctgg ggaatgaatc aggccacggc gctaatcacg acgcgctgta 2280
   tcgctggatc aaatctgtcg atccttcccg cccggtgcag tatgaaggcg gcggagccga 2340
   caccacggcc accgatatta tttgcccgat gtacgcgcgc gtggatgaag accagccctt 2400
   cccggctgtg ccgaaatggt ccatcaaaaa atggctttcg ctacctggag agacgcgccc 2460
   gctgatcctt tgcgaatacg cccacgcgat gggtaacagt cttggcggtt tcgctaaata 2520
   ctggcaggcg tttcgtcagt atccccgttt acagggcggc ttcgtctggg actgggtgga 2580
   tcagtcgctg attaaatatg atgaaaacgg caacccgtgg tcggcttacg gcggtgattt 2640
   tggcgatacg ccgaacgatc gccagttctg tatgaacggt ctggtctttg ccgaccgcac 2700
   gccgcatcca gcgctgacgg aagcaaaaca ccagcagcag tttttccagt tccgtttatc 2760
   cgggcaaacc atcgaagtga ccagcgaata cctgttccgt catagcgata acgagctcct 2820
   gcactggatg gtggcgctgg atggtaagcc gctggcaagc ggtgaagtgc ctctggatgt 2880
   cgctccacaa ggtaaacagt tgattgaact gcctgaacta ccgcagccgg agagcgccgg 2940
   gcaactctgg ctcacagtac gcgtagtgca accgaacgcg accgcatggt cagaagccgg 3000
   gcacatcagc gcctggcagc agtggcgtct ggcggaaaac ctcagtgtga cgctccccgc 3060
   cgcgtcccac gccatcccgc atctgaccac cagcgaaatg gatttttgca tcgagctggg 3120
   taataagcgt tggcaattta accgccagtc aggctttctt tcacagatgt ggattggcga 3180
   taaaaaacaa ctgctgacgc cgctgcgcga tcagttcacc cgtgcaccgc tggataacga 3240
   cattggcgta agtgaagcga cccgcattga ccctaacgcc tgggtcgaac gctggaaggc 3300
   ggcgggccat taccaggccg aagcagcgtt gttgcagtgc acggcagata cacttgctga 3360
   tgcggtgctg attacgaccg ctcacgcgtg gcagcatcag gggaaaacct tatttatcag 3420
   ccggaaaacc taccggattg atggtagtgg tcaaatggcg attaccgttg atgttgaagt 3480
   ggcgagcgat acaccgcatc cggcgcggat tggcctgaac tgccagctgg cgcaggtagc 3540
   agagcgggta aactggctcg gattagggcc gcaagaaaac tatcccgacc gccttactgc 3600
   cgcctgtttt gaccgctggg atctgccatt gtcagacatg tataccccgt acgtcttccc 3660
   gagcgaaaac ggtctgcgct gcgggacgcg cgaattgaat tatggcccac accagtggcg 3720
   cggcgacttc cagttcaaca tcagccgcta cagtcaacag caactgatgg aaaccagcca 3780
   tcgccatctg ctgcacgcgg aagaaggcac atggctgaat atcgacggtt tccatatggg 3840
   gattggtggc gacgactcct ggagcccgtc agtatcggcg gaattccagc tgagcgccgg 3900
   tcgctaccat taccagttgg tctggtgtca aaaataataa taaccgggca ggggggatct 3960
   ttgtgaagga accttacttc tgtggtgtga cataattgga caaactacct acagagattt 4020
   aaagctctaa ggtaaatata aaatttttaa gtgtataatg tgttaaacta ctgattctaa 4080
   ttgtttgtgt attttagatt ccaacctatg gaactgatga atgggagcag tggtggaatg 4140
   ccagatccag acatgataag atacattgat gagtttggac aaaccacaac tagaatgcag 4200
   tgaaaaaaat gctttatttg tgaaatttgt gatgctattg ctttatttgt aaccattata 4260
   agctgcaata aacaagttaa caacaacaat tgcattcatt ttatgtttca ggttcagggg 4320
   gaggtgtggg aggtttttta aagcaagtaa aacctctaca aatgtggtat ggctgattat 4380
   gatctgcggc ctcgagtacc gttcgtataa atgtatgcta tacgaacggt atctagagtc 4440
   gaagcttgac aacaaaaaga ttgtcttttc tgaccagatg gacgcggcca ccctcaaagg 4500
   catcaccgcg ggccaggtga atatcaaatc ctcctcgttt ttggaaactg acaatcttag 4560
   cgcagaagtc atgcccgctt ttgagaggga gtactcaccc caacgcttaa ttaagtctag 4620
   agtcagcttg ggtctcccta tagtgagtcg tattaatttc gataagccag taagcagtgg 4680
   gttctctagt tagccagaga gctctgctta tatagacctc ccaccgtaca cgcctaccgc 4740
   ccatttgcgt caatggggcg gagttgttac gacattttgg aaagtcccgt tgattttggt 4800
   gccaaaacaa actcccattg acgtcaatgg ggtggagact tggaaatccc cgtgagtcaa 4860
   accgctatcc acgcccattg atgtactgcc aaaaccgcat caccatggta atagcgatga 4920
   ctaatacgta gatgtactgc caagtaggaa agtcccataa ggtcatgtac tgggcataat 4980
   gccaggcggg ccatttaccg tcattgacgt caatgggggg cgtacttggc atatgataca 5040
   cttgatgtac tgccaagtgg gcagtttacc gtaaatagtc cacccattga cgtcaatgga 5100
   aagtccctat tggcgttact atgggaacat acgtcattat tgacgtcaat gggcgggggt 5160
   cgttgggcgg tcagccaggc gggccattta ccgtaagtta tgtaacgcgg aactccatat 5220
   atgggctatg aactaatgac cccgtaattg attactatta ataactagtc aataatcaat 5280
   gtcaacgcgt atatctggcc cgtacatcgg actgttttcg acgtttaaac atgcatgaag 5340
   ttcctattcc gaagttccta ttctctagaa agtataggaa cttcataaaa cctgcaggca 5400
   tgcaagcttg gcgtaatcat ggtcatagct gtttcctgtg tgaaattgtt atccgctcac 5460
   aattccacac aacatacgag ccggaagcat aaagtgtaaa gcctggggtg cctaatgagt 5520
   gagctaactc acattaattg cgttgcgctc actgcccgct ttccagtcgg gaaacctgtc 5580
   gtgccagctg cattaatgaa tcggccaacg cgcggggaga ggcggtttgc gtattgggcg 5640
   ctcttccgct tcctcgctca ctgactcgct gcgctcggtc gttcggctgc ggcgagcggt 5700
   atcagctcac tcaaaggcgg taatacggtt atccacagaa tcaggggata acgcaggaaa 5760
   gaacatgtga gcaaaaggcc agcaaaaggc caggaaccgt aaaaaggccg cgttgctggc 5820
   gtttttccat aggctccgcc cccctgacga gcatcacaaa aatcgacgct caagtcagag 5880
   gtggcgaaac ccgacaggac tataaagata ccaggcgttt ccccctggaa gctccctcgt 5940
   gcgctctcct gttccgaccc tgccgcttac cggatacctg tccgcctttc tcccttcggg 6000
   aagcgtggcg ctttctcata gctcacgctg taggtatctc agttcggtgt aggtcgttcg 6060
   ctccaagctg ggctgtgtgc acgaaccccc cgttcagccc gaccgctgcg ccttatccgg 6120
   taactatcgt cttgagtcca acccggtaag acacgactta tcgccactgg cagcagccac 6180
   tggtaacagg attagcagag cgaggtatgt aggcggtgct acagagttct tgaagtggtg 6240
   gcctaactac ggctacacta gaaggacagt atttggtatc tgcgctctgc tgaagccagt 6300
   taccttcgga aaaagagttg gtagctcttg atccggcaaa caaaccaccg ctggtagcgg 6360
   tggttttttt gtttgcaagc agcagattac gcgcagaaaa aaaggatctc aagaagatcc 6420
   tttgatcttt tctacggggt ctgacgctca gtggaacgaa aactcacgtt aagggatttt 6480
   ggtcatgaga ttatcaaaaa ggatcttcac ctagatcctt ttaaattaaa aatgaagttt 6540
   taaatcaatc taaagtatat atgagtaaac ttggtctgac agttaccaat gcttaatcag 6600
   tgaggcacct atctcagcga tctgtctatt tcgttcatcc atagttgcct gactccccgt 6660
   cgtgtagata actacgatac gggagggctt accatctggc cccagtgctg caatgatacc 6720
   gcgagaccca cgctcaccgg ctccagattt atcagcaata aaccagccag ccggaagggc 6780
   cgagcgcaga agtggtcctg caactttatc cgcctccatc cagtctatta attgttgccg 6840
   ggaagctaga gtaagtagtt cgccagttaa tagtttgcgc aacgttgttg ccattgctac 6900
   aggcatcgtg gtgtcacgct cgtcgtttgg tatggcttca ttcagctccg gttcccaacg 6960
   atcaaggcga gttacatgat cccccatgtt gtgcaaaaaa gcggttagct ccttcggtcc 7020
   tccgatcgtt gtcagaagta agttggccgc agtgttatca ctcatggtta tggcagcact 7080
   gcataattct cttactgtca tgccatccgt aagatgcttt tctgtgactg gtgagtactc 7140
   aaccaagtca ttctgagaat agtgtatgcg gcgaccgagt tgctcttgcc cggcgtcaat 7200
   acgggataat accgcgccac atagcagaac tttaaaagtg ctcatcattg gaaaacgttc 7260
   ttcggggcga aaactctcaa ggatcttacc gctgttgaga tccagttcga tgtaacccac 7320
   tcgtgcaccc aactgatctt cagcatcttt tactttcacc agcgtttctg ggtgagcaaa 7380
   aacaggaagg caaaatgccg caaaaaaggg aataagggcg acacggaaat gttgaatact 7440
   catactcttc ctttttcaat attattgaag catttatcag ggttattgtc tcatgagcgg 7500
   atacatattt gaatgtattt agaaaaataa acaaataggg gttccgcgca catttccccg 7560
   aaaagtgcca cctgacgtct aagaaaccat tattatcatg acattaacct ataaaaatag 7620
   gcgtatcacg aggccctttc gtctcgcgcg tttcggtgat gacggtgaaa acctctgaca 7680
   catgcagctc ccggagacgg tcacagcttg tctgtaagcg gatgccggga gcagacaagc 7740
   ccgtcagggc gcgtcagcgg gtgttggcgg gtgtcggggc tggcttaact atgcggcatc 7800
   agagcagatt gtactgagag tgcaccatat gcggtgtgaa ataccgcaca gatgcgtaag 7860
   gagaaaatac cgcatcaggc gccattcgcc attcaggctg cgcaactgtt gggaagggcg 7920
   atcggtgcgg gcctcttcgc tattacgcca gctggcgaaa gggggatgtg ctgcaaggcg 7980
   attaagttgg gtaacgccag ggttttccca gtcacgacgt tgtaaaacga cggccagtga 8040
   attcgagctc ggtacccggg gg                                          8062
<210> 33
   <211> 3858
   <212> DNA
   <213> Artificial Sequence
<220>
   <223> Description of Artificial Sequence: vector pRK50
<400> 33
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcgtcgacct cgagatccag gcgcggatca ataaaagatc attattttca 1020
   atagatctgt gtgttggttt tttgtgtgcc ttgggggagg gggaggccag aatgaggcgc 1080
   ggccaagggg gagggggagg ccagaatgac cttgggggag gggaggccca gaatgacctt 1140
   gggggagggg gaggccagaa tgaggcgcgc ccccgggtac cgagctcgaa ttcactggcc 1200
   gtcgttttac aacgtcgtga ctgggaaaac cctggcgtta cccaacttaa tcgccttgca 1260
   gcacatcccc ctttcgccag ctggcgtaat agcgaagagg cccgcaccga tcgcccttcc 1320
   caacagttgc gcagcctgaa tggcgaatgg cgcctgatgc ggtattttct ccttacgcat 1380
   ctgtgcggta tttcacaccg catatggtgc actctcagta caatctgctc tgatgccgca 1440
   tagttaagcc agccccgaca cccgccaaca cccgctgacg cgccctgacg ggcttgtctg 1500
   ctcccggcat ccgcttacag acaagctgtg accgtctccg ggagctgcat gtgtcagagg 1560
   ttttcaccgt catcaccgaa acgcgcgaga cgaaagggcc tcgtgatacg cctattttta 1620
   taggttaatg tcatgataat aatggtttct tagacgtcag gtggcacttt tcggggaaat 1680
   gtgcgcggaa cccctatttg tttatttttc taaatacatt caaatatgta tccgctcatg 1740
   agacaataac cctgataaat gcttcaataa tattgaaaaa ggaagagtat gagtattcaa 1800
   catttccgtg tcgcccttat tccctttttt gcggcatttt gccttcctgt ttttgctcac 1860
   ccagaaacgc tggtgaaagt aaaagatgct gaagatcagt tgggtgcacg agtgggttac 1920
   atcgaactgg atctcaacag cggtaagatc cttgagagtt ttcgccccga agaacgtttt 1980
   ccaatgatga gcacttttaa agttctgcta tgtggcgcgg tattatcccg tattgacgcc 2040
   gggcaagagc aactcggtcg ccgcatacac tattctcaga atgacttggt tgagtactca 2100
   ccagtcacag aaaagcatct tacggatggc atgacagtaa gagaattatg cagtgctgcc 2160
   ataaccatga gtgataacac tgcggccaac ttacttctga caacgatcgg aggaccgaag 2220
   gagctaaccg cttttttgca caacatgggg gatcatgtaa ctcgccttga tcgttgggaa 2280
   ccggagctga atgaagccat accaaacgac gagcgtgaca ccacgatgcc tgtagcaatg 2340
   gcaacaacgt tgcgcaaact attaactggc gaactactta ctctagcttc ccggcaacaa 2400
   ttaatagact ggatggaggc ggataaagtt gcaggaccac ttctgcgctc ggcccttccg 2460
   gctggctggt ttattgctga taaatctgga gccggtgagc gtgggtctcg cggtatcatt 2520
   gcagcactgg ggccagatgg taagccctcc cgtatcgtag ttatctacac gacggggagt 2580
   caggcaacta tggatgaacg aaatagacag atcgctgaga taggtgcctc actgattaag 2640
   cattggtaac tgtcagacca agtttactca tatatacttt agattgattt aaaacttcat 2700
   ttttaattta aaaggatcta ggtgaagatc ctttttgata atctcatgac caaaatccct 2760
   taacgtgagt tttcgttcca ctgagcgtca gaccccgtag aaaagatcaa aggatcttct 2820
   tgagatcctt tttttctgcg cgtaatctgc tgcttgcaaa caaaaaaacc accgctacca 2880
   gcggtggttt gtttgccgga tcaagagcta ccaactcttt ttccgaaggt aactggcttc 2940
   agcagagcgc agataccaaa tactgtcctt ctagtgtagc cgtagttagg ccaccacttc 3000
   aagaactctg tagcaccgcc tacatacctc gctctgctaa tcctgttacc agtggctgct 3060
   gccagtggcg ataagtcgtg tcttaccggg ttggactcaa gacgatagtt accggataag 3120
   gcgcagcggt cgggctgaac ggggggttcg tgcacacagc ccagcttgga gcgaacgacc 3180
   tacaccgaac tgagatacct acagcgtgag ctatgagaaa gcgccacgct tcccgaaggg 3240
   agaaaggcgg acaggtatcc ggtaagcggc agggtcggaa caggagagcg cacgagggag 3300
   cttccagggg gaaacgcctg gtatctttat agtcctgtcg ggtttcgcca cctctgactt 3360
   gagcgtcgat ttttgtgatg ctcgtcaggg gggcggagcc tatggaaaaa cgccagcaac 3420
   gcggcctttt tacggttcct ggccttttgc tggccttttg ctcacatgtt ctttcctgcg 3480
   ttatcccctg attctgtgga taaccgtatt accgcctttg agtgagctga taccgctcgc 3540
   cgcagccgaa cgaccgagcg cagcgagtca gtgagcgagg aagcggaaga gcgcccaata 3600
   cgcaaaccgc ctctccccgc gcgttggccg attcattaat gcagctggca cgacaggttt 3660
   cccgactgga aagcgggcag tgagcgcaac gcaattaatg tgagttagct cactcattag 3720
   gcaccccagg ctttacactt tatgcttccg gctcgtatgt tgtgtggaat tgtgagcgga 3780
   taacaatttc acacaggaaa cagctatgac catgattacg ccaagctagc ccgggctagc 3840
   ttgcatgcct gcaggttt                                              3858

## Description

The present invention relates to a gene trapping construct which causes conditional mutations in genes, and the use of this gene trapping construct to mutationally inactivate all cellular genes. In addition the invention relates to a cell, preferably a mammalian cell which contains the above mentioned construct. Said cell is useful for identification and/or isolation of genes and for the creation of transgenic organisms to study gene function at various developmental stages, including the adult.

### Background of the Invention

For some years targeted mutagenesis in totipotent mouse embryonic stem (ES) cells has been used to inactivate genes for which cloned sequences were available (Capecchi, The new mouse genetics: altering the genome by gene targeting, Trends in Genetics, 5, 70 - 76 (1989)). Since ES cells can pass mutations induced in vitro to transgenic offspring in vivo it is possible to analyze the consequences of gene disruption in the context of the entire organism. Thus, numerous mouse strains with functionally inactivated genes ("knock out mice") have been created by this technology and utilized to study the biological function of a variety of genes. However, targeted mutagenesis requires detailed knowledge of gene structure and organization as well as its physical isolation in a cloning vector. Moreover, the targeting vector containing the gene of interest needs to be transduced into ES cells to isolate mutant ES clones in which a normal allele is replaced by a mutant allele by homologous recombination (Capecchi 1989). Overall, the generation of mutant mouse strains by this procedure is time consuming, labor intensive, expensive and inefficient because it can handle only one gene at the time (Koller and Smithies, Altering genes in animals by gene targeting. Ann. Rev. Immunol. 10, 705 - 730 (1992)). More importantly, mouse mutants created by this procedure (also known as "conventional, complete or classical mutants"), contain the inactivated gene in all cells and tissues throughout life.

A refined method of targeted mutagenesis, referred to as conditional mutagenesis, employs a site specific recombination system (e.g. Cre/loxP or Flp/frt - Sauer and Henderson, Site-specific recombination in mammalian cells by the Cre recombinase of bacteriophage P1, Proc. Natl. Acad. Sci. USA 85, 5166-5170 (1988); Senecoff et al., DNA recognition by the FLP recombinase of the yeast 2m plasmid: a mutational analysis of the FLP binding site , J. Mol. Biol.,201, 405 - 421 (1988)) which enables a temporally and/or spatially restricted inactivation of target genes (Rajewsky et al., Conditional gene targeting, J. Clin. Invest., 98, 600 - 603 (1996)). The creation of conditional mouse mutants requires the generation of two mouse strains, i.e. the recombinase recognition strain and the recombinase expressing strain. The recombinase recognition strain is generated by homologous recombination in ES cells as described above except that the targeted exon(s) is (are) flanked by two recombinase recognition sequences (hereinafter "RRS"), e.g. loxP or frt. Since the RRS reside in introns they do not interfere with gene expression. The recombinase expressing strain contains a recombinase transgene (e.g. Cre, Flp) whose expression is either restricted to certain cells and tissues or is inducible by external agents. Crossing of the recombinase recognition strain with the recombinase expressing strain deletes the RRS-flanked exons from the doubly transgenic offspring in a prespecified temporally and/or spatially restricted manner. Thus, the method allows the temporal analysis of gene function in particular cells and tissues of otherwise widely expressed genes. Moreover, it enables the analysis of gene function in the adult organism by circumventing embryonic lethality which is frequently the consequence of gene inactivation. For pharmaceutical research, aiming to validate the utility of genes and their products as targets for drug development, inducible mutations provide an excellent genetic tool. However, as is the case for conventional mutants, the generation of conditional mutants is a time consuming and labor intensive procedure which must be performed individually for every gene.

To address some of the problems encountered with targeted mutagenesis, several types of vectors referred to as "gene traps" have been developed that insert a promoterless reporter gene into mostly random chromosomal sites, including transcriptionally active regions (reviewed in Hill and Wurst, Mutagenic strategies to dissect mammalian development Curr. Topics. Dev. Biol. 28, 181-206 (1993); Gossler and Zachgo, Gene and enhancer trap screens in ES cell chimaeras, in: Gene targeting - a practical approach (A.L. Joyner, Ed.), Oxford University Press, Oxford (1993)). Integrations into the exons (exon traps) or introns (intron traps) of a cellular gene disrupt the gene and generally lead to its functional inactivation. Moreover, the gene trap provides a molecular tag to clone any gene linked to a specific function. Thus, unlike homologous recombination, gene trapping is not restricted to genes for which cloned sequences are available and can be used to disrupt any gene that resides in the genome. In most cases, gene traps are transduced as retroviruses, although electroporated DNA is also used. Retroviruses have the advantage that they integrate by a precise mechanism into the genome and cause no damage to the neighbouring DNA. Moreover, unlike electroporated DNA, retroviruses almost never integrate in tandem. Since gene trap integrations are distributed randomly throughout the genome, it is possible to create mutations in a large number of genes within a a limited number of experiments (Zambrowicz et al., Disruption and sequence identification of 2,000 genes in mouse embryonic stem cells, Nature 392, 608-611 (1998)). In principle, this enables the mutational analysis of all genes within a genome by simply generating an integration library in which every single gene is disrupted by a gene trap. In most cases murine embryonic stem (ES) cells are used as targets for gene trap integrations since mutant clones can be easily obtained in vitro and passaged to transgenic offspring in vivo (Gossler et al., Mouse embryonic stem cells and reporter constructs to detect developmentally regulated genes, Science, 244, 463 - 465 (1989); Friedrich and Soriano, Promoter traps in embryonic stem cells: a genetic screen to identify and mutate developmentally genes in mice, Genes & Development, 5, 1513 - 1523 (1991); Skarnes et al., A gene trap approach in mouse embryonic stem cells: the lacZ reporter is activated by splicing, reflects endogenous gene expression, and is mutagenic in mice, Genes Dev. 6, 903-918 (1992); von Melchner et al., Selective disruption of genes expressed in totipotent embryonal stem cells, Genes & Dev 6, 919-927 (1992)). Mouse mutants obtained with this technology often develop a "loss of function phenotype" which sometimes will disclose the biological significance of a particular gene (Evans et al., Gene trapping and functional genomics, Trends in Genetics, 13, 370 - 374 (1997)). However, all the above gene traps induce an irreversible inactivation of their target. Consequently, a mouse mutant generated from an ES cell clone, is not different from a knock-out mouse obtained by conventional gene targetting.

Recently WO 99/50426 described conditional mutagenesis by utilizing a gene trap cassette capable of producing mutations (genome rearrangements) that can be switched on and off temporarily. These gene trap cassettes comprise suitably arranged frt or loxP recombinase sites, which - when exposed to Flp or Cre, respectively - are removing or "flipping" (i.e., inverting) the gene trap cassette and therewith cause the genome rearrangements. However, conventional site specific recombinases, as Flp and Cre utilized in the above documents, normally recombine reversibly between identical recognition sites (like two loxP or FRT sites). In a conditional gene trap setting where the recombinase effects a "flipping" of the gene trap cassette as described in the above documents this would mean that equal amounts of sense and antisense products would be generated whereby one half of the target alleles carrying the gene trap would be inactivated whereas the other half would still have a functional configuration. Since most genes of the mammalian genome are recessive, inactivation of one allele is normally insufficient to produce an observable phenotype. Therefore, it is important to shift the equilibrium of the recombinase reaction towards the gene trap inversion that causes gene inactivation.

With regard to the above mentioned Cre/loxP recombination system, it is known that the recombination between mutant loxP recognition sites (e.g., single mutant recognition sites lox66 and lox71; Albert et al., The Plant Journal, 7, 649 - 659 (1995)) generates a double mutant- and a wildtype- loxP site (see SEQ ID NOs:4 and 5), each on one side of the inverted DNA. Since the latter combination of loxP sites is less efficiently recognised by the Cre-recombinase, the inversion is mostly unidirectional. The above lox66/lox71 system is utilized by Araki et al., Cell. Mol. Biol. 45(5), 737-750 (July 1999) in a two step gene trap system. In a first step a gene trap vector (having a functional gene flanked by lox sites in the same direction) is integrated into an endogenous gene. In a second step the functional gene is replaced with a second functional gene by Cre-mediated recombination/integration.

In addition to the above Cre/mutant loxP system it was assumed in the references below that recombinases which normally recognize nonidentical RRSs, such as the phage derived integrases of the integrase (Nunes-Duby et al., Nucleic Acid Res. 26, 391 - 406 (1998)) or resolvase (Thorpe et al., Proc. Natl. Acad. Sci. USA 95, 5505 - 5510 (1998)) family of recombinases, may be used for unidirectional inversion.

Among the phage derived integrases, the phage λ-encoded integrase (λ-Int) is the most extensively characterised system (for reviews, see: Craig, Annu. Rev. Genet., 22, 77-105 (1988); Landy, Annu. Rev. Biochem, 58, 913-949 (1989); Landy, Curr. Op. Genet. Dev., 3, 699-707 (1993); Sadowsky, FASEB J., 7, 9-17 (1993); Hallet and Sherratt, FEMS Microb. Rev., 21, 157-178 (1997)). Integrative recombination occurs between an attP attachement site present in the phage genome and an attB attachement site in the bacterial genome. If both sites are placed on the same DNA molecule in the same orientation as used for integration, the intervening DNA segement is deleted from the parental molecule. In contrast to e.g. the loxP/Cre system, attP and attB sites are nonidentical but share some sequence identity in the λ-Int binding core region. A varying number of recognition sequences flanking the core region serves as binding sites for the accessory proteins IHF, Fis or Xis. Upon integrative recombination between an attP and attB site two new sites, termed attR and attL are generated. Since these sites are only recombined again back to an attP/attB pair for excisive recombination if the Xis protein is present, the λ-Int system is unidirectional in its absence.
That the λ-Int system requires the IHF accessory protein also for integrative recombination complicates its biotechnological application in mammalian cells; however, the use of IHF independent mutant λ-Int forms has been recently described in human cells (Lorbach et al., J. Mol. Biol. 296, 1175-1181 (2000)). In contrast to λ-Int, the Integrase of the phage ΦC31 (hereinafter C31-Int) has been characterised to recombine a pair of the respective attP/attB sites without the need for accessory proteins (Thorpe and Smith, Proc. Natl. Acad. Sci. USA 95, 5505 - 5510 (1998)). C31-Int AttP/attB recombination leads to an integrative reaction if the two sites are placed on different molecules or to a deletion of the intervening DNA if the sites are present on the same DNA molecule in the same orientation as used for integration. Since the product of the recombination, a pair of attL/attR sites, is not recombined any more by C31-Int alone (Thorpe and Smith, Proc. Natl. Acad. Sci. USA 95, 5505 - 5510 (1998)), the reaction is unidirectional. C31-Int has been used in human cells for the integration and deletion of DNA molecules in human cells (Groth et al., Proc. Natl. Acad. Sci. 97, 5995-6000 (2000)).

### Summary of the Invention

The object to be solved by the invention of the present application is the provision of a gene trap system that does not show the above drawbacks and allow selective, unidirectional genome rearrangements. It was found that the above object can be solved by a gene trap construct using specific recombinase/recombinase recognition site systems (e.g., systems comprising mutant recombinase recognition sites) which allow an unidirectional inversion, preferably such systems recombine (i.e., invert) only once.

The present invention combines conditional gene targeting with gene trap mutagenesis. A conditional gene trap vector typically integrates into an intron without interfering with gene function. However, activation of the gene trap by a site specific recombinase induces gene disruption and loss of its function. Unlike conditional gene targeting, the strategy is not limited to known genes and can be used to disrupt all genes in a temporally and/or spatially restricted manner.

The present invention thus provides:
(1) a gene trapping construct capable of causing conditional mutations in genes, which comprises a gene disruption cassette comprising a polyadenylation site, said gene disruption cassette being flanked by two recombinase recognition sequences (RRSs) selected from mutant loxP sites, mutant frt sites and AttP/AttB sites, which are present in opposite orientation and which are specific to the site specific recombinases Cre, Flp, F C31-Int and λ-Int, said site-specific recombinases
   (i) being capable of unidirectional inversion of a double stranded gene disruption cassette being flanked by said two RRSs present in opposite orientation, and
   (ii) producing the inverted double stranded gene disruption cassette in an amount of greater 75%, relative to the starting double stranded disruption cassette;
(2) a preferred embodiment of the gene trapping construct defined in (1) above, which comprises
   (a) a gene disruption cassette being flanked by two RRSs which are specific to a first site specific recombinase capable of unidirectional inversion of a double stranded gene disruption cassette, and
   (b) a selection cassette, being positioned in opposite orientation relative to the gene disruption cassette and being flanked by two RRSs of a second site specific recombinase in the same orientation;
(3) a cell comprising the gene trapping construct as defined in (1) or (2) above; and
(4) a transgenic mouse containing in an intron of a gene trapping construct as defined in (1)-(2) above in an antisense direction relative to the gene.

### Short Description of Figures

Fig. 1: Conditional gene trap vector to modify genes expressed in the target cells.
Fig. 2: Conditional retroviral gene trap vector to modify expressed genes.
Fig. 3: Conditional gene trap vector to modifiy genes independently of their expression level in the target cells.
Fig. 4: Conditional retroviral gene trap vector to modify genes independently of their expression.
Fig. 5 shows the conditional gene trap vector pRK57SA-β.
Fig. 6 shows a Southern blot analysis of genomic DNA from the gene trap ES clone GT2.
Fig. 7 shows the test vector for C31-Int mediated inversion, pRK73, and the product of inversion, pRK73-Inv.
Fig. 8 shows the structure of the C31-Int expression vector pRK65.
Fig. 9 shows the PCR products generated with the primers P64-1 and P64-3 and a sequence comparison of the PCR products.
Fig. 10 shows the recombination substrate vectors used for Cre mediated inversion.

### Detailed Description of the Invention

The gene trapping constructs (1) and (2) are hereinafter described in more detail:

The present invention provides a site specific recombination system which enables an unidirectional inversion of sequences flanked by RRS sites. For example, in the Cre/loxP recombination system, it is known that the recombination between mutant loxP recognition sites (e.g., single mutant recognition sites lox66 and lox71; Albert et al., The Plant Journal, 7, 649 - 659 (1995); see SEQ ID NOs:2 and 3) generates a double mutant- and a wildtype-loxP site (see SEQ ID NOs:4 and 5), each on one side of the inverted DNA. Since this combination of loxP sites is less efficiently recognised by the Cre-recombinase, the inversion is "unidirectional".

A recombination system is "unidirectional" in accordance with the present invention if a reaction of a site specific recombinase corresponding to the RRSs within the construct produces the inverted double stranded DNA segment in an amount of greater 75%, preferably greater 90%, relative to the starting double stranded DNA segment.

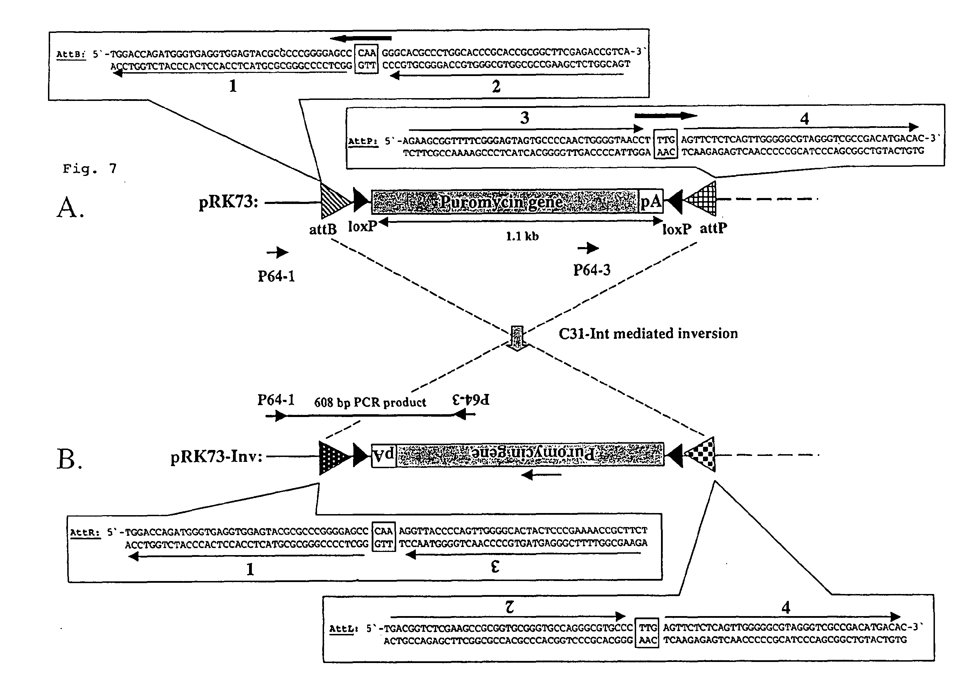

Inversion of a functional DNA segment is achieved through flanking RRSs sites being oriented in opposite orientation. Suitable RRSs in accordance with the present invention include constructs wherein the two RRSs are selected from mutant loxP sites, mutant frt sites and AttP/AttB sites, and the site specific recombinase is Cre, Flp, ΦPC31-Int or λ-Int, respectively. Preferred RRSs are constructs wherein the two RRSs are single mutant loxP sites, preferably a lox66 and a lox71 site (SEQ ID NOs:2 and 3) or an AttP and an AttB site of Φ C31 as shown in Fig. 7, and the site specific recombinase is Cre or Φ C31, respectively.

In a preferred embodiment of the invention the gene disruption cassette of the construct (1) further comprises one or more of the following functional elements: splice acceptor, splice donor, internal ribosomal entry site (IRES), a gene coding for a reporter protein, a resistance gene and a gene coding for a further site specific recombinase. Suitable splice acceptors include, but are not limited to, the adenovirus type 2 splice acceptor of exon 2 shown in SEQ ID NO:1; suitable donors include, but are not limited to, the adenovirus exon 1 splice donor; suitable IRES include, but are not limited to, that of the ECM virus shown in SEQ ID NO:8.

Suitable polyadenylation sequences for the construct (1) are the polyadenylation site sequence of the bovine growth hormone (bpA) or that shown in SEQ ID NO:6.

Suitable reporter genes include, but are not limited to, E. coli β-galactosidase (see SEQ ID NO:10), fire fly luciferase, fluorescent proteins (e.g., GFP) and human placental alkaline phosphatase (PLAP). Suitable resistance genes include, but are not limited to, neomicin phosphotransferase, purimicin (see SEQ ID NO:9) and hygromicin. Suitable further (second) site specific recombinase are all recombinases which do not interfere with the RRSs of the gene trapping construct.

In a further preferred embodiment of the present invention the construct (1) further comprises a selection DNA segment ("selection cassette") suitable for selecting for genes having an incorporated gene trapping construct, said selection DNA segment comprising a reporter or resistance gene and flanking recombinase recognition sites in same orientation. Suitable resistance genes are those mentioned above, provided, however, that they do not interfere with the resistance gene of the gene disruption cassette. Suitable recombinase recognition sites in same orientation include, but are not limited to, loxP and mutants thereof (see SEQ ID NOs:5, 2 and 3), frt and mutants thereof (see SEQ ID NO:7), provided, however, that these RRSs do not interfere with the RRSs of the gene disruption cassette.

The activation mechanism of a conditional gene trap is based on the ability of a site specific recombinase (e.g. Cre or Flp) to invert a double stranded DNA segment flanked by two RRSs (e.g. loxP or frt) inserted in opposite orientations (Abremski et al., Cell, 32, 1301'- 1311 (1983); Hamilton et al., J. Mol. Biol., 178, 481 - 486 (1984); Albert et al., The Plant Journal, 7, 649 - 659 (1995); Vetter et al., Proc. Natl. Acad. Sci. USA, 80, 7284 - 7288 (1983)). If this is used in the context of a gene trap vector, sequence elements that interfere with gene expression (e.g. splice sites, polyadenylation signals) can be first inserted into an intron in an antisense direction relative to the gene. By residing on the non-transcribed DNA strand, these elements will not interfere with gene transcription. Whenever or wherever a gene disruption is desired, inversion is induced by expressing the appropriate site specific recombinase.

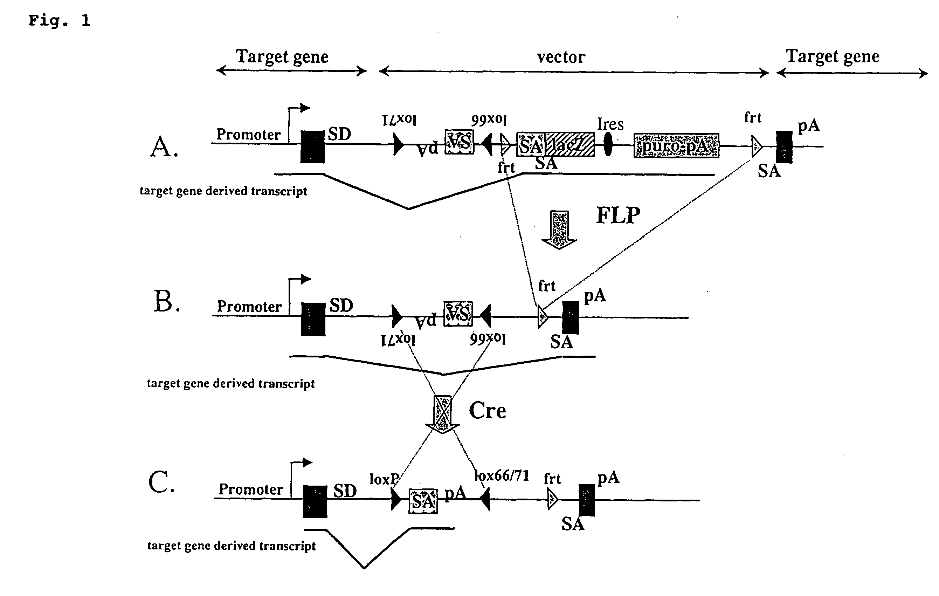

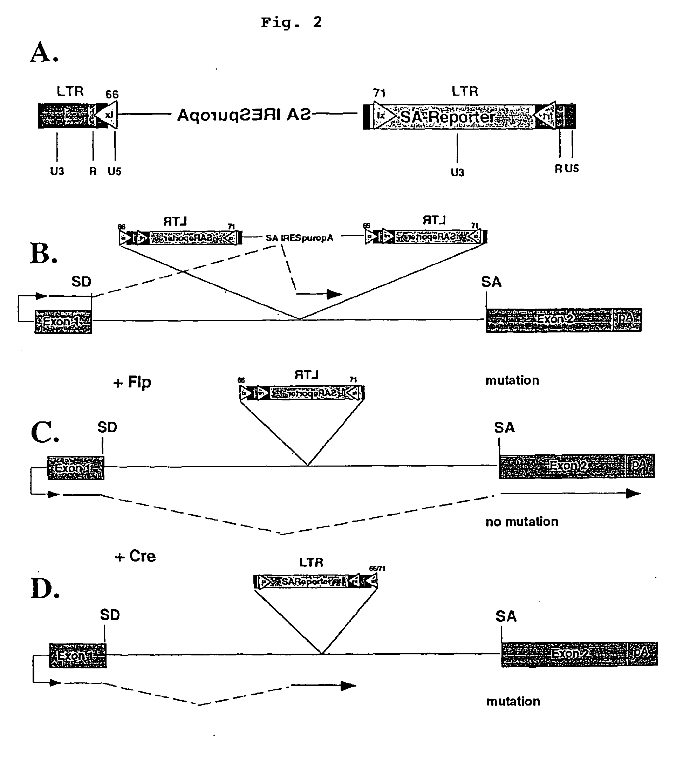

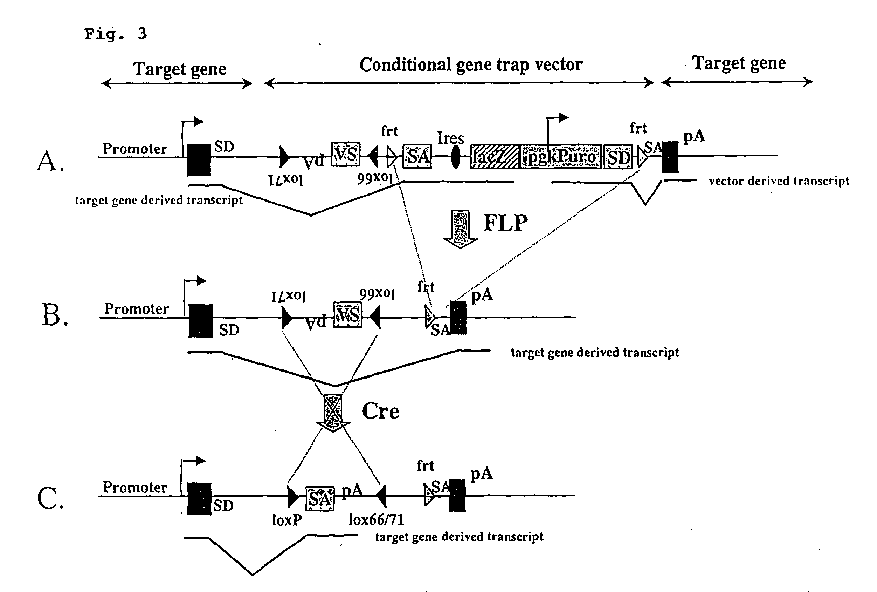

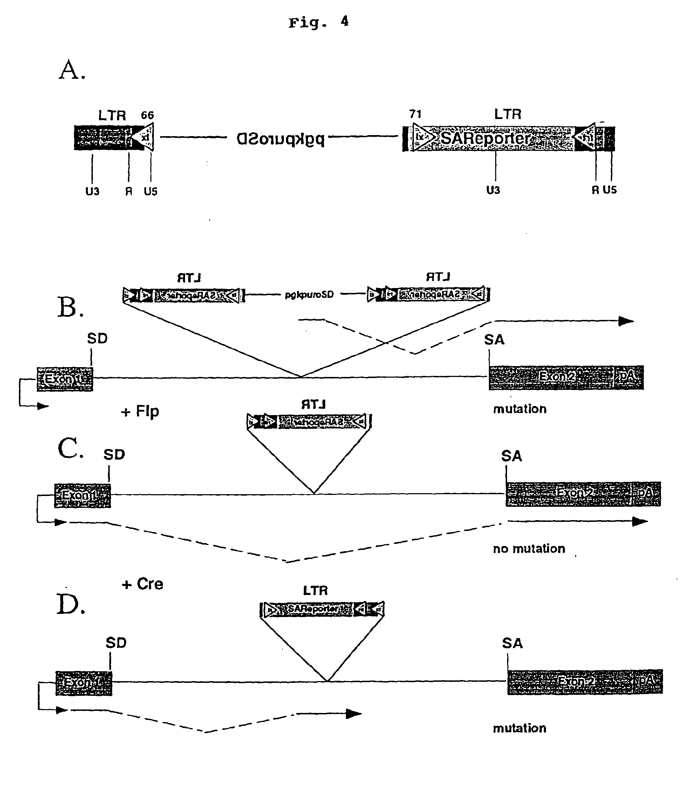

The preferred types of operation with respect to the invention are plasmid (Figures 1 and 3) or retroviral vectors (Figures 2 and 4) which can select for integrations into introns. Selection is based on the expression of a reporter gene that requires either an active cellular promoter (expressed genes) (Figures 1 and 2) or the acquisition of a endogenous transcriptional termination (polyadenylation) signal (Figures 3 and 4).

The principal elements of a conditional gene trap vector of embodiments (1) and (2) of the invention that selects for integrations into expressed genes are (i) a conditional gene disruption cassette, containing a 3' splice site (splice acceptor; SA; see SEQ ID NO:1) and a polyadenylation sequence (polyA; see SEQ ID NO:6) flanked by two RRSs (RRS1) of a site specific recombinase (rec1) in opposite orientation, and (ii) a selection cassette containing a reporter or selectable marker gene flanked by an upstream SA- and a downstream polyA-site. The selection cassette is flanked by two RRSs (RRS2) in same orientation which are recognized by second recombinase (rec2) and is in opposite orientation to the gene disruption cassette (Figures 1 and 3). Selection for gene expression yields recombinants in which the reporter gene is fused to the regulatory elements of an endogenous gene. Transcripts generated by these fusions encode for a truncated cellular protein which has lost its normal function. Since selection for a gene trap event relies on the expression of the selection cassette which is by itself mutagenic, it needs to be removed to recreate gene function. This is achieved by expressing rec2 in recombinants selected for gene trap integrations. In a favoured operational process the conditional gene trap is transduced into ES cells. After selecting for integrations into the introns of expressed genes, rec2 is transiently expressed in individual clones to delete the selection cassette to restore gene function. The resulting clones containing only the gene disruption cassette are used to create transgenic mouse strains. Such mouse strains are crossed to rec1 expressing mouse strains to obtain doubly transgenic offspring where the gene disruption cassette is reversed to its mutagenic (sense) orientation.

As a further preferred embodiment the invention provides a conditional gene trap vector that selects for integrations into all genes. This is achieved by replacing the elements of the original selection cassette by a reporter gene that is fused to an upstream constitutive promoter and to a downstream 5'splice site (splice donor) (Zambrowicz, et al., 1998) (Figures 3 and 4). Expression of this gene trap is dependent on the acquisition of an endogenous polyadenylation sequence which occurs by splicing of the selection cassette to the donwstream exons of the target gene. Since the process is driven by a constitutive promoter, selection for gene trap integrations is independent of target gene expression. As with the other conditional gene trap, a favoured operational process is its transduction into ES cells and the generation of mutant mouse strains.

Thus, as a preferred embodiment the present invention provides for a conditional gene trapping construct selecting for integrations into expressed genes. In said construct the gene disruption cassette preferably comprises a splice acceptor and a polyadenylation sequence flanked by two RRSs of a first site specific recombinase capable of of unidirectional inversion of a double stranded DNA segment as defined above. Said construct further comprises a selection DNA segment (selection cassette) which comprises a reporter or selectable marker gene flanked by an upstream splice acceptor sequence and a downstream polyadenylation sequence, said selection cassette being flanked by two conventional RRSs of a second site specific recombinase in same orientation. As a further preferred embodiment the present invention provides for a conditional gene trapping construct selecting for integrations into all genes. In said construct the gene disruption cassette comprises a splice acceptor and a polyadenylation sequence flanked by two RRSs of a first site specific recombinase capable of unidirectional inversion of a double stranded DNA segment as defined above. Said constuct further comprises a selection DNA segment (selection cassette) which comprises a reporter or selectable marker gene fused to an upstream constitutive promoter and a downstream splice donor site, said selection cassette being flanked by two RRSs of a second site specific recombinase in same orientation.
In said preferred embodiments the RRSs of the second site specific recombinase may be any conventional RRS provided, however, that they do not interfere with the RRSs of the first recombinase.

The cell of embodiment (3) of the invention can be prepared by conventional methods including electroporation or retroviral infection. The cell (3) may be used for the identification and/or isolation of genes.

The construct (2) is useful for a process for the generation of conditional mutations in all genes of an organism comprising
(i) installation of a gene trapping construct as defined in (2) above in a suitable cell,
(ii) selection of cells in which the construct is incorporated in a gene,
(iii) identification and/or isolation of the gene in which the construct is incorporated,
(iv) deletion of the selection cassette from the trapped gene,
(v) induction of a mutation in the trapped gene by inversion of the functional DNA segment.
In a special embodiment of said process the mutation in step (v) is effected by a site specific recombinase capable of unidiretional inversion of the functional DNA of the gene trapping construct. The process is suitable for temporarily and/or spatially restricted inactivation of all genes that constitute a living organism and for preparing transgenic mammals, wherein in step (i) the gene trapping construct is installed in an ES cell.

The above process possesses the following advantages over current technology:
(i) mutations are inducible in prespecified cells and tissues and during prespecified time intervals;
(ii) mutations can be induced in all genes, including those for which cloned sequences are not available;

(i) the functional analysis of the mutant genes in appropriate organisms is relatively fast and cheap.

The transgenic mouse (4) is useful for studying gene function at various developmental stages.

The appended figures further explain the present invention:
Figure 1 shows a favoured operational form of the process relating to a conditional gene trap transduced by electroporation to capture expressed genes.
   A: Target gene after insertion of a conditional gene trap vector into an intron of an expressed gene. In transcripts initiating at the target gene's promoter the LacZ- and the puromycin resistance genes (selection cassette) are fused to the upstream exons by a splice acceptor site. The fusion disrupts and inactivates the target gene.
   B: Modified allele after FLP mediated deletion of the selection cassette. Excision of sequences flanked by the frt recognition sites which include the splice acceptor signal restores wild type transcription and gene function.
   C: Target gene after Cre mediated inversion of the loxP flanked gene trap elements (gene disruption cassette). Gene trap inversion positions the splice acceptor- and the poly A- sites into the sense orientation which is mutagenic. SA: splice acceptor signal sequence; SD: splice donor signal sequence; pA: polyadenylation sequence; lacZ: beta-Galactosidase; puro: puromycin resistance gene; filled rectangles: exons; filled triangles: loxP recognition sites, loxP: wildtype loxP site, lox66: single mutant lox site, lox71: single mutant lox site; lox 66/71: double mutant lox site; shaded triangles: FRT recognition sites (frt); thick continuous lines: mRNA transcripts; thin continuous lines: intron; thin stippled lines: recombinase mediated deletion or inversion between lox or FRT sites. Cre: Cre recombinase; FLP: FLP recombinase. Ires: Internal ribosomal entry site.
Figure 2 shows a favoured operational form of the process relating to a conditional gene trap transduced by retroviruses to capture expressed genes.
   A: Retroviral plasmid vector.
   B. Target gene after insertion of a conditional gene trap provirus into an intron of an expressed gene. Virus replication and LTR mediated duplication places the gene disruption cassette in U3 between mutant loxP sites in both 5' and 3' proviral LTRs. Moreover, it places the selection cassette inserted into the body of the virus between two frt sites. Transcripts initiating in the cellular promoter are spliced to the selection cassette by an upstream splice acceptor site. This fusion disrupts and inactivates the target gene.
   C: Modified allele after FLP mediated excision of all proviral sequences placed between the frt sites. This restores gene function and leaves only one copy of gene disruption cassette behind.
   D: Target gene after Cre mediated inversion of the loxP flanked gene trap elements. LTR inversion positions the gene disruption cassette into a mutagenic sense orientation. LTR = long terminal repeat, U3 = 3' unique region of the retroviral LTR, R = repetitive region of the retroviral LTR, U5 = 5' unique region of the retroviral LTR, SA = splice acceptor signal sequence; SD = splice donor sequence; pA = polyadenylation sequence; Puro = puromycin resistance gene; lx = loxP recognition sites; frt = Flp recognition sites; thin continuous lines = intron; thin stippled lines = sequences excised by mRNA processing; arrows indicate transcriptional orientation.
Figure 3 shows a favoured operational form of the process relating to a conditional gene trap transduced by electroporation to capture all genes.
   A: Target gene after insertion of a conditional gene trap vector into an intron of a gene. Transcripts initiating in the independent pgk promoter express the puromycin resistance gene after splicing into downstream exons to acquire a polyadenylation site. Splicing is mediated by a splice donor sequence inserted downstream of pgkpuro. The fusion of the selection cassette to downstream exons inactivates the target gene.
   B: Modified allele after FLP mediated deletion of the selection cassette which restores gene function.
   C: Target gene after Cre mediated inversion of the gene disruption cassette which causes the mutation.
      SA: splice acceptor signal sequence; SD: splice donor signal sequence; pA: polyadenylation sequence; lacZ: beta-Galactosidase; puro: puromycin resistance gene; filled rectangles: exons; filled triangles: loxP recognition sites, loxP: wildtype loxP site, lox66: single mutant lox site, lox71: single mutant lox site; lox 66/71: double mutant lox site; shaded triangles: FRT recognition sites (frt); thick continuous lines: mRNA transcripts; thin continuous lines: intron; thin stippled lines: recombinase mediated deletion or inversion between lox or FRT sites. Cre: Cre recombinase; FLP: FLP recombinase. Ires: Internal ribosomal entry site; pgk: phosphoglyceratekinase promoter.
Figure 4 shows a favoured operational form of the process relating to a conditional gene trap transduced by retroviruses to capture all genes.
   A: Retroviral plasmid vector
   B: Target gene after insertion of a conditional gene trap provirus into an intron of a gene.
      Virus replication and LTR mediated duplication places the gene disruption cassette in U3 between mutant loxP sites in both 5' and 3' proviral LTRs. Moreover, it places the selection cassette inserted into the body of the virus between two frt sites. Transcripts initiating in the independent pgk promoter express the puromycin resistance gene after splicing into downstream exons to acquire a polyadenylation site. Splicing is mediated by a splice donor sequence inserted downstream of pgkpuro. The fusion of the selection cassette to the downstream exons inactivates the target gene.
   C: Modified allele after FLP mediated excision of all proviral sequences placed between the frt sites. This restores gene function and leaves only one copy of gene disruption cassette behind.
   D: Target gene after Cre mediated inversion of the loxP flanked gene trap elements. LTR inversion positions the gene disruption cassette into a mutagenic sense orientation.
      LTR = long terminal repeat, U3 = 3' unique region of the retroviral LTR, R = repetitive region of the retroviral LTR, U5 = 5' unique region of the retroviral LTR, SA = splice acceptor signal sequence; SD = splice donor sequence; pA = polyadenylation sequence; Puro = puromycin resistance gene; lx = loxP recognition sites; frt = Flp recognition sites; thin continuous lines = intron; thin stippled lines = sequences excised by mRNA processing; pgk = phosphoglyceratekinase promoter; arrows indicate the direction of transcript elongation.

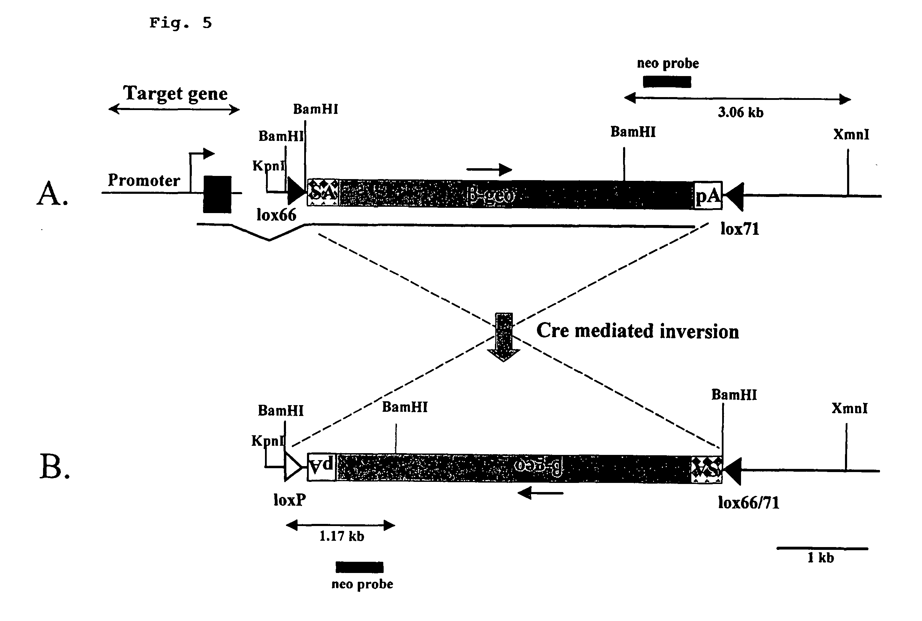

Figure 5 shows the conditional gene trap vector pRK57SA-β
   A: Original configuration of the conditional gene trap vector after insertion into an intron of an expressed gene. In transcripts initiating at the target gene's promoter the β-geo gene is fused to the upstream exons by a splice acceptor site. The fusion disrupts and inactivates the target gene. The splice acceptor - β-geo cassette is flanked by two mutant lox sites in opposite orientation. The arrow indicates the trancriptional direction of the β-geo gene.
   B: Conditional gene trap vector after Cre mediated inversion of the lox flanked gene trap elements (gene disruption cassette). Gene trap inversion positions the splice acceptor- and the poly A- sites into the antisense orientation which is non-mutagenic. The recombination of lox66 and lox71 sites generates one loxP wildtype and one double mutant lox 66/71 site.
      SA: splice acceptor signal sequence; pA: polyadenylation sequence; β-geo: fusion gene of β-galactosidase and neomycinphosphotransferase; filled rectangle: exon; triangles: lox recognition sites, loxP: wildtype loxP site, lox66: single mutant lox site, lox71: single mutant lox site; lox 66/71: double mutant lox site; thick continuous line: mRNA transcript; thin stippled lines: recombinase mediated inversion between lox sites. Cre: Cre recombinase.

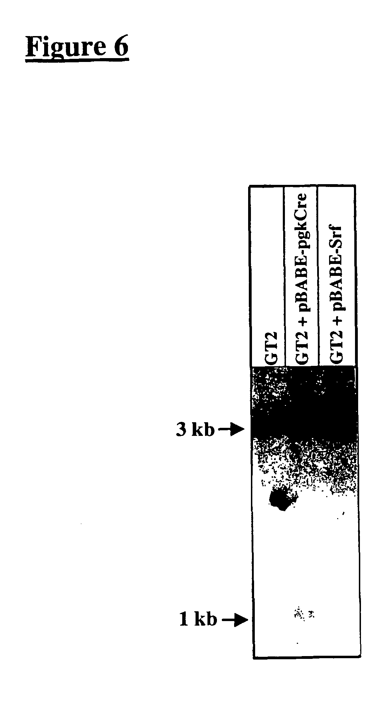

Figure 6 shows a Southern blot analysis of genomic DNA from the gene trap ES clone GT2. Upon digestion with BamHI and XmnI, and a segment of the neomycin phosphotransferase gene as a probe, the integrated pRK57SA-β vector gives rise to a band of 3.06 kb, upon Cre mediated inversion the band is reduced to a size of 1.17 kb. Shown are samples of GT2 cells at 6 days after infection with the Cre expressing retrovirus pBABE-pgkCre, the control virus pBABE-Srf, or which were not treated with virus. The band indicating inversion of the gene trap vector appears only in the sample treated with the Cre expressing retrovirus.
Figure 7 shows the test vector for C31-Int mediated inversion, pRK73, and the product of inversion, pRK73-Inv.
   A: Plasmid pRK73 contains the 1.1 kb cassette of the coding region of the puromycin resistance gene and a polyadenylation signal, which is flanked 5' by the 84 bp attB and 3' by the 84 bp attP recognition site of C31-Int. These attB and attP sites are oriented in opposite to each other (thick black arrows), as compared to the orientation of attB and attP before the integration of the phiC31 phage into the bacterial genome. In addition, the cassette is flanked by two Cre recombinase recognition (loxP) sites in opposite orientation to each other. For better orientation the half sites of the att sequences are labelled by a direction (thin arrow) and numbered 1-4. The 3 bp sequence within the att sites at which recombination occurs is framed by a box. The positions at which the PCR primers P64-1 and P64-3 hybridise to the pRK73 vector are indicated by an arrow, pointing into the 3' direction of both oligonucleotides.
   B: The inversion product pRK73-Inv expected from the C31-Int mediated recombination between the att sites of pRK73. The recombination between a pair of attB/attP sites generates a pair of attR/attL sites flanking the inverted puromycin cassette. In this configuration the primers P64-1 and P64-3 will amplify a PCR product of 608 bp.

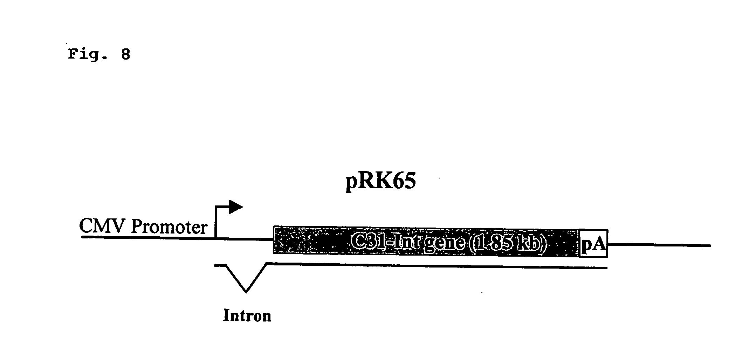

Figure 8 shows the structure of the C31-Int expression vector pRK65.
   The 1.85 kb C31-Int coding region is expressed by the CMV promoter for expression in mammalian cells. A 300 bp hybrid intron between promoter and coding region provides a splice donor and acceptor site; the coding region is followed by a synthetic polyadenylation signal sequence. The expected transcript is depicted as a thin line.

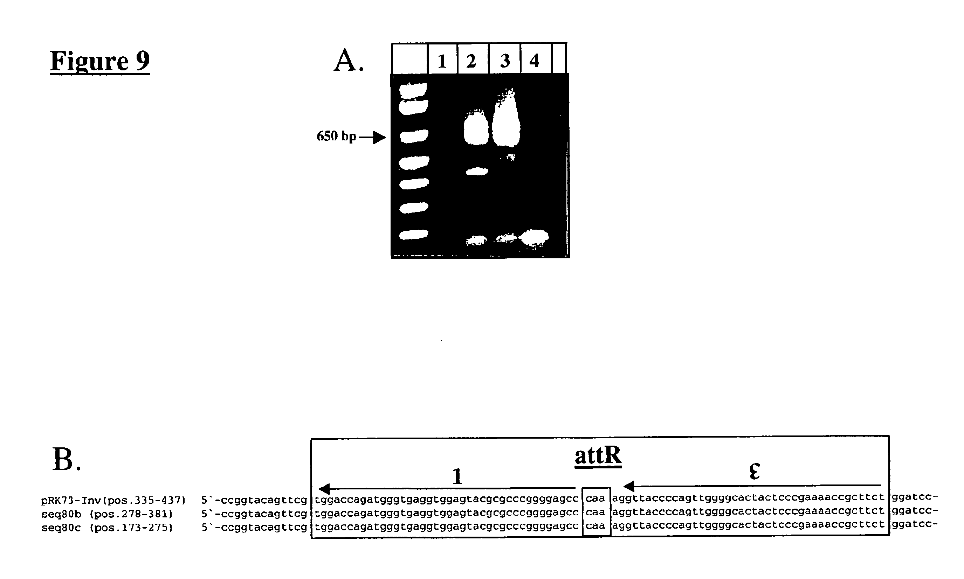

Figure 9 shows the PCR products generated with the primers P64-1 and P64-3 and a sequence comparison of the PCR products.
   A: Analysis of PCR products on an agarose gel from PCR reactions using the Primers P64-1 and P64-3 on DNA extracted from MEF5-5 cells transfected 2 days before with plasmid pRK73 alone (lane 4), with pRK73 + CMV-Cre (lane 3), with pRK73 + pRK65 (lane 2), and from nontransfected cells (lane 1). The products with an apparent size around 650 bp, as compared to the size marker used, from lane 2 were excised from the agarose gel and purified. The PCR products were cloned into a sequencing plasmid vector and gave rise to the plasmids pRK80b and pRK80c. The insert of these plasmids was sequenced using reverse primer (seq80b and seq80c) and compared to the predicted sequence of the pRK73 vector after C31-Int mediated inversion of the att flanked cassette, pRK73-Inv. Both PCR products show a 100% identity with the predicted attR sequence after inversion. The generated attR site is shown in a box, with the same sequence designation used in Figure 7B. The nucleotide positions of the compared sequences pRK73, seq80b and seq80c are indicated.

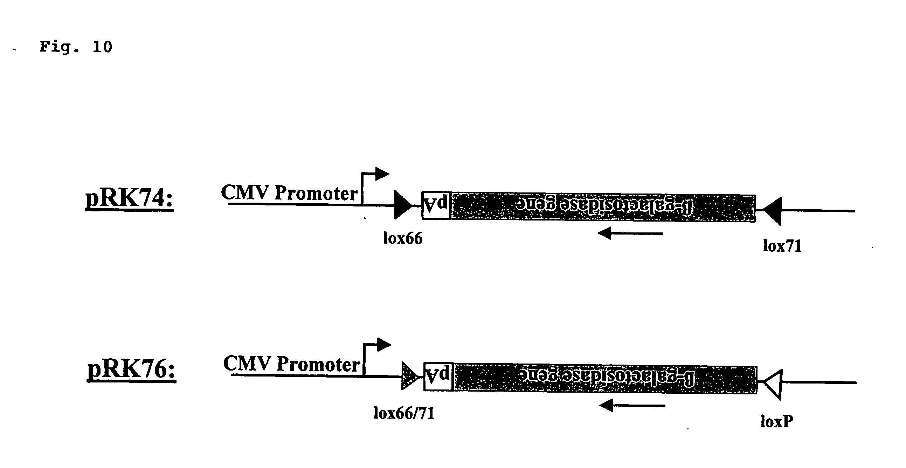

Figure 10 shows the recombination substrate vectors used for Cre mediated inversion.
   Plasmid pRK74 contains a CMV promoter for expression in mammalian cells followed by cassette containing the E. coli β-galactosidase gene and a polyadenylation sequence in inverse orientation to the CMV promoter. This cassette is flanked by the lox66 and lox71 single mutant lox sites (Albert et al., The Plant Journal, 7, 649 - 659 (1995)) in opposite orientation. Plasmid pRK76 is structured like pRK74 except that the inverse β-galactosidase cassette is flanked by a lox66/71 double mutant lox site and a wildtype loxP site. The straight arrow indicates the transcriptional orientation of the β-galactosidase expression cassette.

The present invention is further illustrated by the following Examples which are, however not to be construed as to limit the invention.

### Examples

### Example 1

A plasmid vector containing a splice acceptor sequence followed by a fusion gene of β-galactosidase and neomycin phosphotransferase (SA-β-geo), flanked by two different mutant lox sites in opposite orientation, was constructed and used as a gene trap in ES cells. Cell lines exhibiting β-galactosidase activity were infected with a retroviral vector for expression of Cre to investigate the inactivation of the gene trap vector by recombinase mediated inversion of the lox flanked DNA segment.
A. Construction of the gene trap vector pRK57SA-ß: As the first step of vector construction, a synthetic 74 bp DNA fragment with protruding XbaI ends containing a lox66 and a lox71 Cre recombinase recognition site in opposite orientation (Albert et al., The Plant Journal, 7, 649 - 659 (1995)), separated by a Xho site, was ligated into the XbaI site of the plasmid pNEB193 (New England Biolabs) resulting in the vector pRK57. The synthetic DNA fragment was obtained by annealing of the two synthetic oligonucleotides lox3 (5'-CTAGATAACT TCGTATAGCA TACATTATAC GAACGGTACT CGAGATAACT TCGTATAATG TATGCTATAC GAACGGTA-3' ; SEQ ID NO:11) and lox4 (5' -CTAGATACCG TTCGTATAGC ATACATTATA CGAAGTTATC TCGAGTACCG TTCGTATAAT GTATGCTATA CGAAGTTAT-3' ; SEQ ID NO:12). Next, plasmid pRK57 was cut with the restriction enzyme XhoI, dephosphorylated with calf intestinal phosphatase and the 5' protruding ends were filled in with Desoxynucleotide-triphosphates using the Klenow fragment of E.coli DNA polymerase I. This vector was ligated to a 4.3 kb DNA fragment isolated from the plasmid ROSAbetageo (Friedrich et al., Genes&Development 5, 1513-1523 (1991)), which was obtained by digestion with the restriction enzyme XhoI, and filling in the 5' protruding ends as described. The ligation product, vector pRK57SA-β (SEQ ID NO:13), contains a cassette with the adenovirus type 2 splice acceptor from exon2 of the major late region (SA, pos. 525 to 628; SEQ ID NO:1), a fusion gene of E.coli β-galactosidase and neomycin-phosphotransferase (β-geo, pos. 667 to 4548) and the transcription termination and polyadenylation signal from the bovine growth hormone gene (pA, pos. 4561 to 4843). This SA-β-geo cassette is flanked by one lox66 site 5' to the splice acceptor (pos. 446 to 479) and one lox71 site 3' to the bovine growth hormone gene sequences (pos. 4869 to 4902). The two lox sites are oriented in opposite orientation to each other. The vector pRK57SA-ß contains BamHI sites at the positions 427, 503, 673 and 3723, a single XmnI site at position 6788, and a single KpnI site at position 413.
B. Construction of retroviral vectors: A 1.1 kb NheI fragment carrying the coding sequence for Cre-recombinase (Cre) with an N-terminal nuclear localization signal derived from the simian virus 40 large T-antigen was inserted into the plasmid pBluescript II KS(+) (Stratagene), opened with the restriction enzyme XbaI and dephosphorylated with calf intestinal phosphatase. The resulting plasmid was cut with the restriction enzyme SpeI, dephosphorylated with calf intestinal phosphatase and the 5' protruding ends were filled in with Desoxynucleotide-triphosphates using the Klenow fragment of E.coli DNA polymerase I. This vector was ligated to a 0.6 kb blunt-ended fragment containing the promoter region of the mouse phosphoglycerate kinase (PGK) 1a gene. The ligation product contains a cassette with the PGK promoter and the cre coding sequence in the backbone of pBluescript II KS(+). This cassette was excised as a 1.6 kb NotI fragment, filled in as described and inserted into the filled in SnaBI site of a derivative of the mouse moloney leukemia virus based vector pBABEpuro (Morgenstern and Land, Nucleic Acids Res.18, 3587-3596 (1990)) creating the' retroviral cre expression vector pBABE-pgkCre (SEQ ID NO:14). A control vector pBABE-Srf (SEQ ID NO:15) was produced, which contains a short polylinker region instead of the pgkCre cassette. The plasmid forms of these retroviral vectors contain the following functional elements in a plasmid backbone identical to pBABEpuro:

| pBABE-pgkCre: | position |
|---|---|
| partial MMLV U3 | 8-335 |
| MMLV R | 336-402 |
| MMLV U5 | 403-480 |
| MMLV primer binding site and extended packaging signal (incl. mutated splice donor and 5' end of gag coding sequence with mutated ATG start codon) | 481-1374 |
| mouse PGK promoter | 1417-1921 |
| cre coding sequence | 1972-3024 |
| promoter/enhancer deleted MMLV U3 | 3088-3168 |
| MMLV R | 3187-3253 |
| MMLV U5 | 3254-3332 |

| pBABE-Srf: | |
|---|---|
| partial MMLV U3 | 8-335 |
| MMLV R | 336-402 |
| MMLV U5 | 403-480 |
| MMLV primer binding site and extended packaging signal (incl. mutated splice donor and 5' end of gag coding sequence with mutated ATG start codon) | 481-1374 |
| promoter/enhancer deleted MMLV U3 | 1451-1531 |
| MMLV R | 1549-1616 |
| MMLV U5 | 1617-1695 |
| MMLV U5 cw | 1617-1695 |

Infectious retroviral particles were produced by transfecting 2×10⁷ Phoenix eco packaging cells (http://www.stanford.edu/group/nolan/phoenix_info.html) with a total of 100 µg of the plasmid forms of the retroviral vectors using the calcium-phosphate coprecipitation technique. Two days after transfection the tissue culture supernatants were harvested, filtered through a 0.45 µm filter and mixed with polybrene to a final concentration of 4 µg/ml. This virus supernatant was then used to infect ES cell clones.
To determine the titer of the pBABE-pgkCre virus serial dilutions of the virus containing supernatant were used to infect 10000 NIH-3T3 cells carrying the plasmid pSVpaX1 (Buchholz et al., Nucleic Acids Res. 24, 4256-4262 (1996)) as a stable integrate. Successful infection with the Cre-expression virus leads to excision of the pac/SV40polyA-cassette from the pSVpaX1 construct and concomitantly to expression of the beta galactosidase gene. Expression of this marker was monitored two days after infection by fixing the cells and staining with the chromogenic substrate X-Gal (5-Bromo-4-chloro-3-indolyl-beta-D-galactopyranoside). The number of beta-galactosidase positive cells was used to estimate the titer of the original virus suspensions used for infection. The titer of the pBABE-pgkCre virus supernatant was determined to contain 5000 infectious, particles per milliliter of supernatant.
C. Establishment of ß-galactosidase expressing ES cell clones: 100 µg of vector pRK57SA-ß were linearised with KpnI and electroporated into 3 × 10⁷ cells of the embryonic stem (ES) cell E14, cultured as described in Kühn et al., Science 254, 707-710 (1991). Two days after electroporation the cells were grown for 7 days in medium containing 275 mg/ml of G418 (Gibco BRL) to select for clones which integrated the gene trap vector into an expressed gene. The concomitant expression of the ß-geo fusion gene allows such clones to grow in G418 containing medium due to the expression of the neomycin phosphotransferase. At day 9 after electroporation 60 G418 resistant clones were obtained and further expanded. Aliquots of all clones were tested for expression of ß-galactosidase activity by X-Gal staining, resulting in 23 positive clones showing 1% - 75% positive cells. These clones were analysed further by Southern blot hybridisation for the number of integrated vector copies. 11 of the 23 clones proved to obtain multicopy integrates and were excluded from further analysis. The two single copy integrants, GT2 and GT6, which showed the highest proportion of β-galactosidase positive cells were chosen for further analysis.
D. Infection of ES cell clones with retroviral vectors: To test for the induced inversion of the SA-ß-geo cassette within the integrated pRK57SA-ß vector in the clones GT2 and GT6, cells of both clones were infected either with the Cre recombinase expressing retrovirus pBABE-pgkCre or with the control retrovirus pBABE-Srf lacking the Cre gene. For the later analysis of ß-galactosidase expression 5000 cells/well of clones GT2 and GT6 were plated into wells of 48-well culture plates (Falcon) one day before virus infection. For infection the culture medium was replaced with 300 ml of virus containing supernatants (containing about 1500 infectious particles; i.e. an infection rate of 30%) and the cells were further cultured for 4 hours. After 4 hours the wells were filled up with 300 ml culture medium and incubated overnight. At the following day the supernatants were completely replaced with fresh culture medium and grown for further 2 or 6 days until the analysis of β-galactosidase activity. For the later analysis of the genomic DNA of clone GT2, cells were essentially treated as described above, except that 30000 cells were plated into 24-well culture plates and treated with 600 ml of virus containing supernatants (containing about 3000 infectious particles; i.e. an infection rate of 10%). Genomic DNA was prepared from these samples at day 6 post infection.
E. Histochemical detection of ß-galactosidase activity: To quantitate ß-galactosidase expression the ES cells were plated one day before analysis at low density to be able to easily observe single cells. At the following day the culture medium was removed from the wells, the wells were washed once with phosphate buffered saline (PBS), and the cells were fixed for 5 minutes at room temperature in a solution of 2 % formaldehyde and 1 % glutaraldehyde in PBS. Next, the cells were washed twice with PBS and finally incubated in staining solution for 24 hours at 37 °C (staining solution: 5 mM K₃(Fe(CN)₆), 5 mM K₄(Fe(CN)₆), 2 mM MgCl₂, 1 mg/ml X-Gal (BioMol) in PBS). Blue stained, ß-galactosidase positive cells were detected and distinguished from negative (transparent) cells in a cell culture binocular microscope under 200x magnification. For each determination a minimum of 200 cells was counted.
F. Southern blot hybridization: Genomic DNA was prepared from retrovirus infected ES cell clones following standard methods (Sambrook, Fritsch and Maniatis, Molecular Cloning. A laboratory manual. Cold Spring Harbour Laboratory Press (1989)). Approximately 5 mg of purified DNA were digested with the restriction enzymes BamHI and XmnI to detect the inversion of the pRK57SA-ß genetrap vector. Digested DNA was separated in a 0.8% agarose gel and transferred to nylon membranes (GeneScreen Plus, NEN DuPont) under alkaline conditions for 16 hours. The filter was dried and hybridised for 16 hours at 65°C with a probe representing the 3' part of the neomycin phosphotransferase gene (~600 bp PstI - XbaI fragment of plasmid pgk-neo (Soriano et al., Cell 64,693-702 (1991)). The probe was radiolabeled with P32-marked α-dCTP (Amersham) using the Megaprime Kit (Amersham). Hybridisation was performed in a buffer consisting of 10% Dextranesulfate, 1% SDS, 50mM Tris and 100mM NaCL, pH7.5). After hybridisation the filter was washed with 2x SSC and exposed to BioMax MS1 X-ray films (Kodak) at - 80°C for 3 days.
G. Results: The plasmid vector pRK57SA-β which serves as a conditional gene trap was constructed by placing a cassette, consisting of a splice acceptor sequence followed by the ß-geo gene (SA-ß-geo), between two mutant loxP sites, lox66 and lox71. These recognition sites of Cre recombinase are in opposite orientation to each other and allow to invert the mutagenic splice acceptor-ß-geo cassette within the vector upon expression of Cre (Fig.5). Upon recombination of a lox66 and a lox71 site one wildtype loxP and one double mutant lox66/71 site is generated (Albert et al., The Plant Journal, 7, 649 - 659 (1995)). As the combination of loxP and lox66/71 sites is much less efficiently recombined by Cre than a pair of lox66 and lox71 sites the outcome of the inversion reaction is shifted towards the reaction products, representing 80-90% of all recombined molecules (Albert et al., The Plant Journal, 7, 649 - 659 (1995)). In contrast, the use of two oppositely oriented wildtype loxP sites for Cre mediated inversion would lead to an equilibrium with a 50% proportion for each of the two inversion products (Albert et al., The Plant Journal, 7, 649 - 659 (1995)). The vector pRK57SA-β was linearised 5' to the lox66 site and introduced into murine embryonic stem (ES) cells by electroporation. Stable transfected clones which integrated the conditional gene trap vector into genes which are actively transcribed in ES cells were obtained by selection in G418 containing cell culture medium. Surviving clones need to express the promoterless β-geo fusion gene through a fusion transcript between the splice donor sequence of a mRNA of an endogenous gene and the splice acceptor sequence of the gene trap vector located in an intron.
After the establishment of G418 resistant clones the expression of ß-galactosidase activity was tested by histochemical staining. As expected only a fraction of the G418 resistant clones exhibited some ß-galactosidase activity, varying between 1%-75% of the cells of individual clones. In addition, these clones were analysed for the copy number of the integrated conditional gene trap vector by Southern blot hybridisation. Two ES cell clones, GT2 and GT6, which showed a single copy integration of the vector and a high proportion of β-galactosidase positive cells were choosen for further analysis.
These clones were either infected with the retroviral vector pBABE-pgkCre, designed for expression of Cre recombinase, or with the retroviral control vector pBABE-Srf which lacks the Cre expression cassette. The inversion of the lox-flanked SA-β-geo cassette was tested two and six days after infection by histochemical X-Gal staining for β-galactosidase activity and by Southern blot analysis of genomic DNA. As shown below in Table 1 the proportion of β-galactosidase expressing cells, exhibiting blue staining, among both ES cell clones was significantly reduced by 14 - 27% at both time points in samples infected with the Cre expression retrovirus as compared to samples treated with control virus.

**Table 1:**

| | 2 days post infection | | | 6 days post infection | | |
|---|---|---|---|---|---|---|
| | pBABE-Srf | pBABE-pgkCre | | pBABE-Srf | pBABE-pgkCre | |
| | % blue cells | % blue cells | decrease | % blue cells | % blue cells | % decrease |
| Clone GT2 | 73% | 59% | 14% | 76% | 49% | 27% |
| Clone GT6 | 67% | 48% | 19% | 67% | 47% | 20% |

Since of the clone GT2 only 76% and in clone GT6 only 67% of the cells expressed ß-galactosidase in the control sample, the reduction of blue stained cells by 27% in clone GT2 and by 20% in clone GT6 at day 6 after infection with the Cre expressing virus would correspond to an inversion rate of 36% in clone GT2 and of 30% in clone GT6. These numbers are in close correspondence to the calculated infection rate of 30% of the cells with the pBABE-pgkCre virus supernatant under the conditions used (see above); this correspondence suggests that most of the cells infected with the Cre virus underwent an (almost unidirectional) inversion of the conditional gene trap vector.
The observed increase in non-stained, β-galactosidase negative cells is the expected outcome of Cre mediated inversion of the lox flanked SA-β-geo cassette within its genomic integration site: the SA-β-geo cassette in the clones GT2 and GT6 is initially located in the same transcriptional orientation as the endogenous gene leading to the expression of β-galactosidase from a fusion transcript. Upon inversion of this cassette the transcript of the endogenous gene is not any more interrupted by splicing to the SA-β-geo cassette which, in the inverse orientation, is not any more translated into β-galactosidase protein.
In addition, the inversion reaction of the SA-β-geo cassette upon treatment with Cre expressing retrovirus was directly tested by Southern blot analysis of genomic DNA of the clone GT2. Upon digestion of genomic DNA with BamHI and XmnI the gene trap vector gives rise to a band of 3.06 kb in its original configuration using a segment of the β-geo gene as a probe. Upon inversion of the lox flanked SA-β-geo cassette the fragment detected by the probe is reduced to 1.17 kb (Fig.5). As shown in Figure 6 the expected band demonstrating the recombinase mediated inversion reaction is specifically detected in the sample of the gene trap ES clone GT2 treated with Cre expressing retrovirus but not in the controls. The relatively weak strength of the band indicating inversion corresponds to the inefficient infection of the GT2 cells with Cre virus (10% infection rate) under the conditions used for DNA preparation.
We conclude from this experiment that it is possible by the use of mutant lox sites to invert the DNA segment of a gene trap vector, which is initially mutagenic for the trapped endogenous gene, preferentially (>50%) into a configuration which is not any more mutagenic and allow its wildtype expression. Likewise, if the Sa-ß-geo cassette would be initially integrated into an endogenous gene in its non-mutagenic orientation, Cre mediated inversion would allow to switch its orientation preferentially to the mutagenic form.

### Example 2

A plasmid vector was constructed which allows to test for the inversion of a DNA segment flanked by the attB and attP recognition sequences of the phiC31 phage derived integrase (C31-Int). This vector was transfected together with an expression vector for phiC31 integrase into a murine cell line and recombinase mediated inversion was detected by a specific PCR reaction. The amplified PCR product was cloned and its sequence determined. The obtained sequence confirms that integrase mediated inversion of the test vector occurs in a mammalian cell line and that the recombination occurs at the known breakpoints within the attB and attP sites.
A. Plasmid constructions:
   Construction of the recombination test vector pRK73 (SEQ ID NO:16): First, an attB site (Thorpe and Smith, Proc. Natl. Acad. Sci. USA 95, 5505 - 5510 (1998)), generated by the annealing of the two synthetic oligonucleotides C31-4 (5'-CGTGA CGG TCT CGA AGC CGC GGT GCG GGT GCC AGG GCG TGC CCT TGG GCT CCC CGG GCG CGT ACT CCA CCT CAC CCA TCT GGT CCA; SEQ ID NO:17) and C31-5 (5' -CG TGG ACC AGA TGG GTG AGG TGG AGT ACG CGC CCG GGG AGC CCA AGG GCA CGC CCT GGC CCA CGC ACC GCG GCT TCG AGA CCG TCA; SEQ ID NO:18) was ligated into the BstBI restriction site of the vector PSV-Pax1 (Buchholz et al., Nucleic Acids Res., 24, 4256-4262 (1996)), 5' of its puromycin resistance gene and loxP site, giving rise to plasmid pRK52. The sequence and orientation of the cloned attB site was confirmed by DNA sequence analysis. Next, an attP site, generated by the annealing of the two synthetic oligonucleotides C31-6-2 (GATCCAGAAG CGGTTTTCGG GAGTAGTGCC CCAACTGGGG TAACCTTTGAG TTCTCTCAGTT GGGGGCGTAG GGTCGCCGAC ATGACACG; SEQ ID NO:19) and C31-7-2 (GATCCGTGTC ATGTCGGCGA CCCTACGCCC CCAACTGAGA GAACTCAAAG GTTACCCCAG TTGGGGCACT ACTCCCGAAA ACCGCTTCTG; SEQ ID NO:20), was ligated into the BamHI restriction site of the plasmid pRK52, downstream of the puromycin resistance gene and loxP site, giving rise to plasmid pRK64-dir. In this plasmid the newly cloned attB and attP sites are in the same orientation flanking the puromycin resistance gene. Next, a 260 bp HindII + NruI fragment, containing the attP and a loxP site, was isolated from pRK64-dir, and ligated into plasmid pRK52, which was opened before with BamHI + NruI (the protruding BamHI end was filled with Klenow enzyme and nucleotides) such that the fragment containing the downstream loxP site was removed. The plasmid generated, pRK73, bears a puromycin resistance gene flanked by an attB and attP site in opposite orientation, as well as a pairs of loxP sites in opposite orientation (Fig7A). The sequence and orientation of the newly cloned attP site in pRK73 was confirmed by DNA sequence analysis.
   Construction of the C31-Int expression vector pRK65 (SEQ ID NO:21): First, the C31-Int gene of phage phiC31 was amplified by PCR from phage DNA (# DSM 49156; German collection of microorganisms and cell cultures, Mascheroder Weg 16, 38124 Braunschweig, Germany) using the primers PC31-1 (5' -ATAAGAAT GCGGCCGC CCGAT ATG ACA CAA GGG GTT GTG ACC GGG-3' ; SEQ ID NO:22) and PC31-3 (5' -ATAAGAAT GCGGCCGC ATC CGC CGC TAC GTC TTC CGT GCC-3'; SEQ ID NO:23). The ends of the PCR product were digested with NotI and the product was ligated into plasmid pBluescript II KS, opened with NotI, giving rise to plasmid pRK40. The DNA sequence of the 1.85 kb insert was determined and found to be identical to the published C31-Int gene (Kuhstoss and Rao, J. Mol. Biol. 222, 897-908 (1991)), except for an error in the stop codon. This error was repaired by PCR amplification of 300 bp fragment from plasmid pRK40 using the primers PC31-8 (5' -CCCGTTGGCA GGAAGCACTT CCGG-3' ; SEQ ID NO:24) and PC31-9 (5'- GGATCCTCGA GCCGCGGGCG GCCGCCTACG CCGCTACGTC TTCCGTGCCG TCCTG-3' ; SEQ ID NO:25), which provide a corrected Stop codon. The ends of this PCR fragment were digested with Eco47III and XhoI and the fragment ligated into plasmid pRK40, opened with Eco47III and XhoI to remove the defetive stop codon. The resulting plasmid, pRK55, contains the correct C31-Int gene as confirmed by DNA sequence analysis. The gene was isolated from pRK55 as 1.85 kb fragment by digestion with NotI and XhoI and ligated into the plasmid pRK50, opened with NotI and XhoI, giving rise to the C31-Int expression vector pRK65. PRK65 contains a 700 bp Cytomegalovirus immediated early gene promoter (position 1 - 700) and a 270 bp hybrid intron (position 700 - 970) upstream of the NotI site and a 189 bp synthetic polyadenylation sequence (position 2831 - 3020) downstream of the XhoI site (all elements derived from pRK50). The C31-Int gene occupies position 978 - 2819 of pRK65. To generate the Cre expression plasmid CMV-Cre, the coding sequence of Cre recombinase was cloned into the NotI and XhoI sites of plasmid pRK50.
B.Transfection of Cells and PCR amplification: MEF5-5 mouse fibroblasts (Schwenk et al., Nucleic Acids Research, 26, 1427-1432 (1998)) (20000 cells per well of a 12 well plate (Falcon)) were transfected with 0.5 µg pRK73 alone or together with 125 ng pRK65 or CMV-Cre using the FuGene transfection reagent (Roche Diagnostics). After 2 days DNA was extracted from these cells and used for PCR amplification with Primers P64-1 (5' -TCA GCA ACC AGG CTC CCC AGC AGG C-3' ; SEQ ID NO:26) and P64-3 (5'-TAG AGG ATC ATA AAT CAG CCA TAC CAC-3' ; SEQ ID NO:27) using the Expand High Fidelity PCR kit (Roche Diagnostics).
   PCR products were separated on a 0.8% agarose gel, extracted with QuiaEx^{®} (Quiagen) and cloned into the pCR2.1 vector using the TA cloning kit (Invitrogen) resulting in plasmids pRK80b and pRK80c. The sequence of the inserts - determined using the reverse sequencing primer and standard methods (MWG Biotech) - are shown in SEQ ID NOs:28 and 29, respectively.
C. Results: As a test vector for C31-Int mediated DNA inversion the plasmid pRK73 was constructed as described in A. above. PRK73 contains a DNA segment of 1.1 kb size (the coding region of the puromycin resistance gene) which is flanked 5' by the 84 bp attB and 3' by the 84 bp attP recognition site of C31-Int (Fig. 7A). These attB and attP sites are oriented in opposite to each other, allowing inversion of the flanked DNA segment (Fig.7B), as compared to the natural orientation of attB and attP in the phiC31 phage and the bacterial genome, which allows integration or deletion of the phage genome. As a control, vector pRK73 contains in addition two Cre recombinase recognition (loxP) sites in opposite orientation next to the att sites.

The predicted product of C31-Int mediated inversion of pRK73 is shown in pRK73-inv (SEQ ID NO:30).

As the second component of the desired test system we constructed a mammalian expression vector for C31-Int, plasmid pRK65, which contains the CMV-IE-Promoter upstream of the C31-Int coding region followed by a synthetic polyadenylation signal (Fig.8).
The recombination substrate vector pRK73 was transfected into the murine fibroblast cell line MEF5-5 either alone, or together with the C31-Int expression vector pRK65, or together with an expression vector for Cre recombinase, CMV-Cre. Two days after transfection DNA was prepared from the three samples and tested for the occurrence of the expected Cre or C31-Int generated inversion product by a specific polymerase chain reaction (PCR). Using primers P64-1 and P64-3 for amplification a 608 bp product can be only obtained after recombinase mediated inversion between the lox or att sites (Fig.7B). This product represents the junction 5' of the puromycin resistance gene which is occupied by an attB site in the vector pRK73; after C31-Int mediated inversion this site would be recombined into an attR site.
As shown in Figure 9A such an amplification product was found only in the samples cotransfected with the Cre or C31-Int recombinase expression vectors. To prove that the PCR product found after cotransfection of plasmids pRK73 and pRK65 represents indeed the inversion product of C31-Int mediated recombination, this DNA fragment was cloned into a plasmid vector and its DNA sequence determined. Two independent clones, pRK80b and pRK80c, were analysed and showed exactly the sequence of an attR site as expected from C31-Int mediated recombination (Thorpe and Smith, Proc. Natl. Acad. Sci. USA 95, 5505 - 5510 (1998)), which leads to the inversion of the attB/attP flanked puromycin resistance gene (Fig.9B). Although not formally proven here, it follows from the knowledge on C31-Int mediated recombination (Thorpe and Smith, Proc. Natl. Acad. Sci. USA 95, 5505 - 5510 (1998)) that the junction located 3' of the inverted puromycin resistance gene represents an attL site.
In conclusion, this experiment demonstrates that it possible to invert in a mammalian cell line a DNA segment flanked by a pair of C31-Int attB/attP recognition sites, generating a pair of attR/attL sites. As the combination of attR/attL is not recombined by C31-Int alone (Thorpe and Smith, Proc. Natl. Acad. Sci. USA 95, 5505 - 5510 (1998)) the reaction described above represents a system for the unidirectional inversion of DNA segments.

### Example 3

Plasmid vectors were constructed which allow to test for the efficiency of the Cre recombinase mediated inversion of a DNA segment flanked by different single mutant lox sites compared to inversion of the same DNA segment flanked by a wildtype loxP site and a double mutant lox site. These vectors were transfected together with a Cre expression vector into a murine cell line and the relative inversion efficiency was determined through the expression of β-galactosidase as a reporter gene.
A. Vector construction:
   Construction of pRK74 (SEQ ID NO:31): starting from plasmid pRK62, which is identical to plasmid pRK57 (see example 1), except that a pair of (irrelevant) FRT sites has been inserted into the PmeI site, a 4.4 kb XhoI - NotI (protruding ends filled up) cassette was ligated into the NotI site of pRK62 (ends filled up). The 4.4 kb cassette contains a 120 bp splice acceptor sequence from from exon2 of the major late region of adenovirus type 2, followed by the 550 bp internal ribosomal entry site of ECMV virus and the coding region of β-galactosidase (AgeI-BamHI fragment of plasmid pCH110; Pharmacia) and a polyadenylation sequence from plasmid PSV-Pax1 (Buchholz et al., Nucleic Acids Res., 24, 4256-4262 (1996)). The resulting plasmid pRK72 contains the β-galactosidase cassette between a pair of lox66 and lox71 sites in opposite orientation. Next, plasmid pRK72 was opened with PacI and ligated with a 850 bp PmeI-HindIII fragment (ends blunted) from plasmid pRK50 providing a CMV promoter and a splice donor site (Choi et al., Mol. Cell. Biol. 11, 3070-3074 (1991)), resulting in plasmid pRK74. To derive pRK76, plasmid pRK72 was opened with SalI (ends filled) and ligated with the 850 bp PmeI-HindIII fragment (ends blunted) from plasmid pRK50, resulting in plasmid pRK75. PRK75 was co-transformed into E.coli DH5 cells with the Cre expression vector p705-Cre (Buchholz et al., Nucleic Acids Research, 24, 3118-3119 (1996)) to invert the ß-galactosidase cassette in bacteria. One of the inverted subclones was designated pRK76 (SEQ ID NO:32). To generate the Cre expression plasmid CMV-Cre, the coding sequence of Cre recombinase was cloned into the NotI and XhoI sites of plasmid pRK50 (SEQ ID NO:33).
B. Transfection and measurement of ß-galactosidase activity: MEF5-5 mouse fibroblasts (Schwenk et al., Nucleic Acids Research, 26, 1427-1432 (1998)) (10000 cells per well of a 24 well plate (Falcon)) were transfected with 1 µg pRK74 or pRK76 alone or together with 500 ng CMV-Cre plasmid using the FuGene transfection reagent (Roche Diagnostics). After 2 days the cells were lysed and the ß-galactosidase activities were determined with the ß-galactosidase reporter gene assay (Roche Diagnostics) according to the manufacturers guidelines using a Lumat LB 9507 luminometer (Berthold).
C. Results: The efficiency of Cre mediated inversion of a DNA segment flanked by the single mutant lox66 and lox71 sites was compared to the inversion of the same DNA segment flanked by a wildtype loxP site and a double mutant lox site using the recombination substrate vectors pRK74 and pRK76 (Fig. 10). Plasmid pRK74 contains a CMV promoter for expression in mammalian cells followed by cassette containing the E. coli ß-galactosidase gene and a polyadenylation sequence in inverse orientation to the CMV promoter. This cassette is flanked by the lox66 and lox71 single mutant lox sites (Albert et al., The Plant Journal, 7, 649 - 659 (1995)) in opposite orientation. Upon expression of Cre recombinase this cassette can be inverted and allows the production of β-galactosidase as a measure of the recombination efficiency. Plasmid pRK76 is structured like pRK74 except that the inverse β-galactosidase cassette is flanked by a lox66/71 double mutant lox site and a wildtype loxP site. Since the lox66 and lox71 sites are recombined into a pair of lox66/71 (named lox72 in Albert et al., The Plant Journal, 7, 649 - 659 (1995)) and loxP sites (Albert et al., The Plant Journal, 7, 649 - 659 (1995)), the comparison of the β-galactosidase activities generated from plasmids pRK74 or pRK76 after co-expression of Cre recombinase allows to compare the relative efficiency at which a pair of lox66//lox71 or lox66/71//loxP sites are recombined.
   PRK74 and pRK76 were transiently transfected into a murine fibroblast cell line either alone or with the Cre expression vector CMV-Cre. After two days the cells were lysed and the levels of β-galactosidase activity in the lysates was determined by chemoluminescence. As shown in Table 2 below, the co-expression of Cre with plasmid pRK74 resulted in an increase of 20024 RLU while co-expression of Cre with plasmid pRK76 showed an reduced activity increase of 7554 RLU. If the latter value is defined as an inversion efficiency of 1, plasmid pRK74 is recombined with a 2.64-fold higher efficiency (Table2).

**Table 2**

| Plasmids | ß-galactosidase activity (relative light units(RLU) | activity increase (RLU) | relative increase |
|---|---|---|---|
| pRK74 | 1923 | | |
| pRK74 + CMV-Cre | 21947 | 20024 | 2.64 |
| pRK76 | 5252 | | |
| pRK76 + CMV-Cre | 12826 | 7574 | 1 |
| No DNA | 1660 | | |

From these results we conclude that a pair of lox66/71//loxP sites (in pRK76) can be recombined (by inversion) by Cre in mammalian cells, but that this type of recognition sites is about 3-fold less efficiently recognised than a pair of lox66/lox71 sites (in pRK74). Thus, under equilibrium conditions, providing a saturating level of Cre recombinase, a plasmid containing one of the two lox site combinations, should be recombined such that about 75% of the molecules contain the lox66/71//loxP conformation and about 25% of the molecules contain the lox66/lox71 conformation. This result confirms the similar observation obtained by Albert et al., The Plant Journal, 7, 649 - 659 (1995) in plant cells and confirms that unequal recombination between mutant lox sites also occurs in a murine cell line.

In conclusion, this reaction can be used to shift the equilibrium of a Cre mediated inversion reaction to the side of the product containing the pair of 1ox66/71//IoxP sites.

## Claims

1. A gene trapping construct capable of causing conditional mutations in genes, which comprises a gene disruption cassette comprising a polyadenylation site, said gene disruption cassette being flanked by two recombinase recognition sequences (RRSs) selected from mutant loxP sites, mutant frt sites and AttP/AttB sites, which are present in opposite orientation and which are specific to the site specific recombinases Cre, Flp, Φ C31-Int and λ-Int, said site-specific recombinases
(i) being capable of unidirectional inversion of a double stranded gene disruption cassette being flanked by said two RRSs present in opposite orientation, and
(ii) producing the inverted double stranded gene disruption cassette in an amount of greater 75%, relative to the starting double stranded disruption cassette.

2. The construct according to claim 1, wherein the RRSs are specific to a site specific recombinase producing the inverted double stranded gene disruption cassette in an amount of greater 90%, relative to the starting double stranded gene disruption cassette.

3. The construct according to claim 2, wherein the two RRSs are single mutant loxP sites, preferably a lox66 and a lox71 site (SEQ ID NOs:2 and 3) or an AttP and an AttB site of Φ C31, and the site specific recombinase is Cre or Φ C31 respectively.

4. The construct according to claim 2 or 3, wherein the gene disruption cassette is flanked by two pairs of RRSs in opposite orientation.

5. The construct according to any one of claims 1 to 4, wherein the gene disruption cassette further comprises one or more of the following: splice acceptor, splice donor, internal ribosomal entry site, a gene coding for a reporter protein, a resistance gene and a gene coding for a further site specific recombinase.

6. The construct according to any one of claims 1 to 5, which further comprises a selection cassette suitable for selecting for genes having an incorporated gene trapping construct, said selection cassette comprising a reporter or resistance gene and flanking recombinase recognition sites in same orientation.

7. The construct according to any one of claims 1 to 3 which comprises
(a) a gene disruption cassette being flanked by two RRSs which are specific to a first site specific recombinase capable of unidirectional inversion of a double stranded gene disruption cassette, and
(b) a selection cassette, being positioned in opposite orientation relative to the gene disruption cassette and being flanked by two RRSs of a second site specific recombinase in the same orientation.

8. The construct according to claim 7, wherein the gene disruption cassette comprises a splice acceptor and a polyadenylation sequence flanked by said two RRSs of the first site specific recombinase, and the selection cassette comprises a reporter or selectable marker gene flanked by an upstream splice acceptor sequence and a downstream polyadenylation sequence, said construct being a conditional gene trapping construct selecting for integrations into expressed genes.

9. The construct according to claim 7, wherein the gene disruption cassette comprises a splice acceptor and a polyadenylation sequence flanked by said two RRSs of the first site specific recombinase, and the selection cassette comprises a reporter or selectable marker gene fused to an upstream constitutive promoter and a downstream splice donor site, said construct being a conditional gene trapping construct selecting for integrations into all genes.

10. A cell comprising the gene trapping construct as defined in claims 1 to 9.

11. A transgenic mouse containing in an intron of a gene a gene trapping construct as defined in claims 1 to 9 in an antisense direction relative to the gene.

## Patentansprüche

1. Genfallen-Konstrukt, das dazu fähig ist, in Genen konditionale Mutationen zu verursachen, umfassend eine Polyadenylierungsstelle umfassende Genzerstörungskassette, wobei die Genzerstörungskassette von zwei Rekombinase-Erkennungssequenzen (RRS) flankiert wird, die aus mutanten loxP-Stellen, Mutanten-frt-Stellen und AttP/AttB-Stellen ausgewählt sind, die in entgegengesetzter Orientierung vorhanden sind und für die ortsspezifischen Rekombinasen Cre, Flp, Φ C31-Int und λ-Int spezifisch sind, wobei die ortsspezifischen Rekombinasen
(i) zu einer unidirektionalen Inversion einer doppelsträngigen Genzerstörungskassette, die von den beiden vorhandenen RRS, die in entgegengesetzter Orientierung vorhanden sind, flankiert ist, fähig sind und
(ii) die invertierte, doppelsträngige Genzerstörungskassette in einer Menge von mehr als 75 %, bezogen auf die doppelsträngige Ausgangs-Zerstörungskassette, erzeugen.

2. Konstrukt nach Anspruch 1, wobei die RRS für eine ortsspezifische Rekombinase spezifisch sind, durch welche die invertierte, doppelsträngige Genzerstörungskassette in einer Menge von mehr als 90 %, bezogen auf die doppelsträngige Ausgangs-Zerstörungskassette, erzeugt wird.

3. Konstrukt nach Anspruch 2, wobei die beiden RRS einzelne mutante loxP-Stellen vorzugsweise eine lox66- und eine lox71-Stelle (SEQ-ID NR.: 2 und 3) oder eine AttP- und eine AttB-Stelle von Φ C31 sind und die ortsspezifische Rekombinase Cre bzw. Φ C31 ist.

4. Konstrukt nach Anspruch 2 oder 3, wobei die Genzerstörungskassette von zwei RRS-Paaren in entgegengesetzter Orientierung flankiert ist.

5. Konstrukt nach einem der Ansprüche 1 bis 4, wobei die Genzerstörungskassette weiterhin ein oder mehr des Folgenden umfasst: einen Spleißakzeptor, einen Spleißdonor, eine interne Ribosomen-Eintrittsstelle, ein Gen codierend für ein Reporterprotein, ein Resistenzgen und ein Gen, das für eine weitere ortsspezifische Rekombinase codiert.

6. Konstrukt nach einem der Ansprüche 1 bis 5, das weiterhin eine Selektionskassette umfasst, die zur Selektion von Genen mit einem eingearbeiteten Genfallen-Konstrukt geeignet ist, wobei die Selektionskassette weiterhin ein Reporter- oder ein Resistenzgen und flankierende Rekombinase-Erkennungsstellen in derselben Orientierung umfasst.

7. Konstrukt nach einem der Ansprüche 1 bis 3, umfassend
(a) eine Genzerstörungskassette, die von zwei RRS flankiert wird, die für eine erste ortsspezifische Rekombinase spezifisch sind, die zur unidirektionalen Inversion einer doppelsträngigen Genzerstörungskassette fähig ist, und
(b) eine Selektionskassette, die in entgegengesetzter Orientierung in Bezug auf die Genzerstörungskassette angeordnet ist und von zwei RRS einer zweiten ortsspezifischen Rekombinase in derselben Orientierung flankiert ist.

8. Konstrukt nach Anspruch 7, wobei die Genzerstörungskassette einen Spleißakzeptor und eine Polyadenylierungssequenz umfasst, die von den beiden RRS der ersten ortsspezifischen Rekombinase flankiert sind, und die Selektionskassette ein Reporter- oder ein selektierbares Markergen umfasst, das stromaufwärts von einer Spleißakzeptor-Sequenz und stromabwärts von einer Polyadenylierungssequenz flankiert ist, wobei das Konstrukt ein konditionales Genfallen-Konstrukt ist, das Integrationen in exprimierte Gene selektiert.

9. Konstrukt nach Anspruch 7, wobei die Genzerstörungskassette einen Spleißakzeptor und eine Polyadenylierungssequenz umfasst, die von den beiden RRS der ersten ortsspezifischen Rekombinase flankiert sind und die Selektionskassette ein Reporter- oder ein selektierbares Markergen umfasst, die stromaufwärts mit einer konstitutiven Promotor- und stromabwärts mit einer Spleiß-Donorstelle verknüpft sind, wobei das Konstrukt ein konditionales Genfallen-Konstrukt ist, das Integrationen in alle Gene selektiert.

10. Zelle, umfassend das in den Ansprüchen 1 bis 9 definierte Genfallen-Konstrukt.

11. Transgene Maus, enthaltend in einem Intron eines Gens ein Genfallen-Konstrukt gemäß der Definition in den Ansprüchen 1 bis 9 in einer Antisense-Richtung in Bezug auf das Gen.

## Revendications

1. Construction pour piégeage de gène capable de provoquer des mutations conditionnelles dans des gènes, qui comprend une cassette de disruption de gène comprenant un site de polyadénylation, ladite cassette de disruption de gène étant flanquée par deux séquence de reconnaissance de recombinase (RRS) sélectionnées depuis des sites loxP mutants, des sites frt mutants et des sites AttP/AttB, qui sont présents en orientation opposée et qui sont spécifiques aux recombinases spécifiques à un site Cre, Flp, Φ C31-Int et λ-Int, lesdites recombinases spécifiques à un site
(i) étant capable d'une inversion unidirectionnelle d'une cassette de disruption de gène à double brin étant flanquée par deux RRS présentes en orientation opposée, et
(ii) produisant la cassette de disruption de gène à double brin inversée en une quantité de plus de 75%, par rapport à la cassette de disruption de gène à double brin de départ.

2. Construction selon la revendication 1, dans laquelle les RRS sont spécifiques à une recombinase spécifique à un site produisant la cassette de disruption de gène à double brin inversée en une quantité de plus de 90 %, par rapport à la cassette de disruption de gène à double brin de départ.

3. Construction selon la revendication 2, dans laquelle les deux RRS sont des sites loxP mutants seuls, préférablement un site lox66 et un site lox71 (SEQ ID NO : 2 et 3) ou un site AttP et un site AttB de Φ C31, et la recombinase spécifique à un site est Cre ou Φ C31 respectivement.

4. Construction selon la revendication 2 ou 3, dans laquelle la cassette de disruption de gène est flanquée par deux paires de RRS en orientation opposée.

5. Construction selon l'une quelconque des revendications 1 à 4, dans laquelle la cassette de disruption de gène comprend en outre un ou plus de ce qui suit : un site accepteur, un site donneur, un site d'entrée ribosomal interne, un gène de codage pour une protéine rapporteuse, un gène de résistance et un gène de codage pour une recombinase spécifique à un autre site.

6. Construction selon l'une quelconque des revendications 1 à 5, qui comprend en outre une cassette de sélection appropriée pour sélectionner des gènes ayant une construction de piégeage de gène incorporée, ladite cassette de sélection comprenant un gène de résistance ou rapporteur et flanquant des sites de reconnaissance de recombinase dans la même orientation.

7. Construction selon l'une quelconque des revendications 1 à 3, qui comprend
(a) une cassette de disruption de gène étant flanquée par deux RRS qui sont spécifiques à une recombinase spécifique à un premier site capable d'une inversion unidirectionnelle d'une cassette de disruption de gène à double brin, et
(b) une cassette de sélection, étant positionnée en orientation opposée par rapport à la cassette de disruption de gène et étant flanquée par deux RRS d'une recombinase spécifique à un second site dans la même orientation.

8. Construction selon la revendication 7, dans laquelle la cassette de disruption de gène comprend un site accepteur et une séquence de polyadénylation flanquée pas deux RRS de la recombinase spécifique à un premier site, et la cassette de sélection comprend un gène marqueur sélectionnable ou rapporteur flanqué par une séquence amont de site accepteur et une séquence avale de polyadénylation, ladite construction étant une construction de piégeage de gène conditionnelle choisissant des intégrations en des gènes exprimés.

9. Construction selon la revendication 7, dans laquelle la cassette de disruption de gène comprend un site accepteur et une séquence de polyadénylation flanquée pas deux RRS de la recombinase spécifique à un premier site, et la cassette de sélection comprend un gène marqueur sélectionnable ou rapporteur développé en un promoteur constitutif amont et un site donneur aval, ladite construction étant une construction de piégeage de gène conditionnelle choisissant des intégrations en tous gènes.

10. Cellule comprenant la construction de piégeage de gène comme définie dans les revendications 1 à 9.

11. Souris transgénique contant dans un intron d'un gène, une construction de piégeage de gène comme définie dans les revendications 1 à 9 dans une direction antisens par rapport au gène.
